# EUROPEAN PATENT APPLICATION

(11) **EP 4 342 488 A1**
(43) Date of publication of application: **27.03.2024**
(21) Application number: 22197771.3
(22) Date of filing: 26.09.2022
(51) Int. Cl.: A61K 39/00, A61P 35/00, C07K 16/28

(54) **CHIMERIC ANTIGEN RECEPTOR SPECIFIC FOR FOLATE RECEPTOR 1**

(71) Applicant: Miltenyi Biotec B.V. & Co. KG, 51429 Bergisch Gladbach (DE)
(72) Inventor: HERBEL, Christoph, 51429 Bergisch Gladbach (DE); DAIGRE, Julie, 51429 Bergisch Gladbach (DE)

(57) **Abstract**

The present invention provides a chimeric antigen receptor (CAR) comprising: an antigen binding domain specific for folate receptor 1 (FolRl), a spacer, a transmembrane domain and an intracellular signaling domain. Moreover it was found that an antigen binding domain that comprises from the N- to the C-terminus the heavy chain variable region of an antibody (VH) comprising the amino acid sequence Seq ID No:2 a light chain variable region of an antibody (VL) comprising the amino acid sequence Seq ID No:4 shows superior killing properties compared to other variants.

## Description

### Field of the invention

The present invention relates to the field of adoptive immunotherapy for the treatment of cancer, in particular by using a chimeric antigen receptor specific for the tumor antigen folate receptor 1 (FolR1).

### Background of the invention

Cancer is a broad group of diseases involving unregulated growth of malignant cells. In cancer, cells divide and grow uncontrollably, forming malignant tumors, and invading nearby parts of the body. The cancer may also spread to more distant parts of the body through the lymphatic system or bloodstream. There are over 200 different cancers known that affect humans. Good treatment options are available for many cancer types. However, others still represent unmet medical needs. In particular, ovarian cancer, lung cancer, gastric cancer, breast cancer, renal cancer, and pancreatic cancer are malignancies with limited therapeutic options⁸. Ovarian cancer is defined as a group of tumors that originate in the ovaries. Ovarian cancer is the fourth most common cancer among women and is the leading cause of gynecological cancer-related death in women. Most clinical cases are epithelial ovarian cancers, which represent approximately 90% of diagnosed patients. Epithelial ovarian cancer has four main histologic subtypes: serous, endometrioid, mucinous, and clear cell. Approximately 70% of patients present with serous histology, and clinical trials have demonstrated that a patient's subtype has prognostic importance. Other less common types of ovarian tumors include primary peritoneal, fallopian tube, and malignant germ cell tumors⁹.

Ovarian cancer patients have an approximate overall five-year survival rate of 49.1%. As with most cancers, the five-year survival rate can differ depending on the stage at which the cancer is diagnosed. If the cancer is caught early at a localized stage (Stage I-II), the five-year survival rate is 92.6%. Ovarian cancer that has spread to a different part of the body, also known as regional ovarian cancer (Stage III), has a five-year survival rate of 74.8%. The five-year survival rate decreases further to 30.3% in metastatic (Stage IV) ovarian cancer. Most patients present with advanced disease at diagnosis, and despite high responses to initial treatment, the majority will eventually relapse with incurable disease⁹.

FolR1 is a membrane protein, which binds folic acid with high affinity and mediates the cellular uptake of this vitamin via receptor-mediated endocytosis¹⁰. Folate is a basic component of cell metabolism and DNA synthesis and repair, and rapidly dividing cancer cells have an increased requirement for folate to maintain DNA synthesis. FolR1 levels are high in specific malignant tumors of epithelial origin compared to normal cells, and are positively associated with tumor stage and grade, raising questions of its role in tumor etiology and progression. It has been suggested that FolR1 might confer a growth advantage to the tumor by modulating folate uptake from serum or by generating regulatory signals¹¹.

The chimeric antigen receptor (CAR) provides a promising approach for adoptive cell immunotherapy for cancer. Commonly, CARs comprise a single chain fragment variable (scFv) of an antibody specific for a tumor associated antigen (TAA) coupled via spacer and transmembrane regions to cytoplasmic domains involved in T-cell signaling. The most common lymphocyte activation moieties include a T-cell costimulatory (e.g. CD28, CD137, OX40, ICOS, and CD27) domain in combination with a T-cell triggering (e.g. CD3ζ) moiety. The CAR-mediated adoptive immunotherapy allows CAR-expressing cells to directly recognize the TAAs on target tumor cells in an HLA independent manner.

Surgery in combination with chemotherapy is still standard of care. However, the majority of patients relapse and, finally, will develop resistance to chemotherapy. Thus, ovarian cancer has a strong unmet medical need and, alternative therapeutic approaches such as immunotherapy using CAR expressing cells are urgently needed. Especially for triple-negative breast cancer, gastric cancer, lung cancer, renal cancer and pancreatic cancers a high expression of the tumor associated FolR1 was identified and it therefore represents a promising target for CAR T cell therapy. There is a need in the art for an improved or alternative CAR specific for FolR1 for use in treatment of cancer expressing FolR1.

### Brief description of the invention

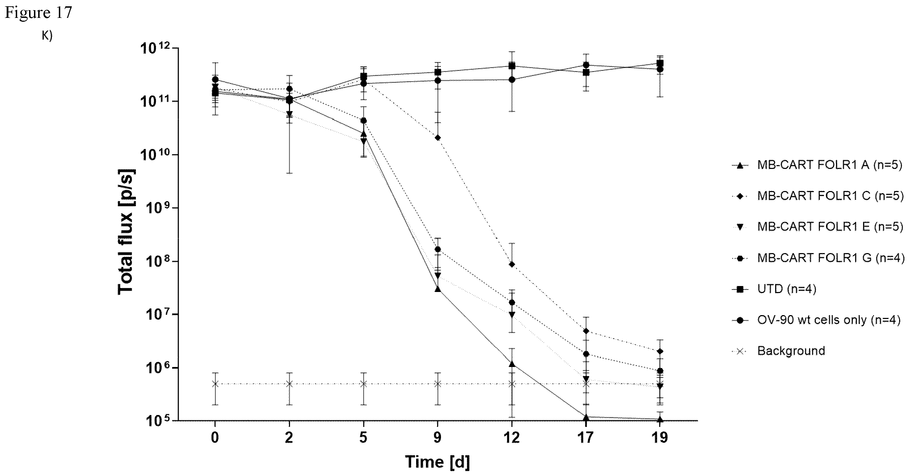

The cell surface antigen FolR1 is expressed on cancer cells such as triple-negative breast cancer, gastric cancer, lung cancer, renal cancer, pancreatic cancer or ovarian cancer and can be used for targeted immunotherapy. The inventors found that immune cells engineered to express a chimeric antigen receptor specific for FolR1 are able to kill cells expressing FolR1 *in vivo* and *in vitro.* Therefore, the invention is related to adoptive cell transfer of cells engineered to express a chimeric antigen receptor wherein said CAR is specific for FolR1 ("FolR1-CAR"), resulting in the recognition of and binding to the cancerous cells expressing FolR1. Then the genetically modified cell, i.e. the CAR expressing cell, performs its specific function, for example killing the target cell, secreting cytokines and/or proliferating. Moreover the inventors surprisingly found that an antigen binding domain that comprises from the N- to the C-terminus the heavy chain variable region of an antibody (VH) comprising the amino acid sequence Seq ID No:2 a light chain variable region of an antibody (VL) comprising the amino acid sequence Seq ID No:4 shows superior killing properties compared to other variants of anti-FolR1-CARs (see Example 2 and Figure 17k).

### Brief description of the drawings

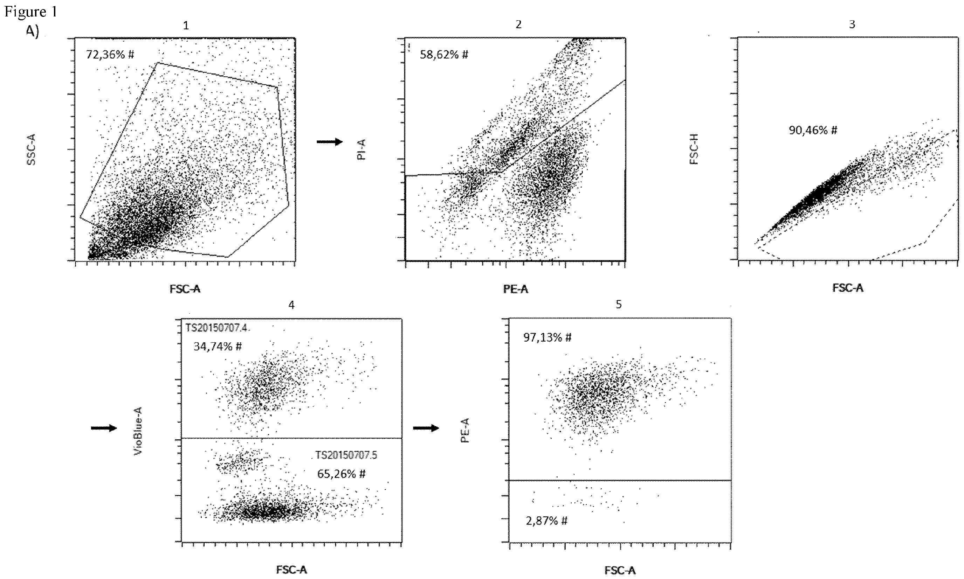

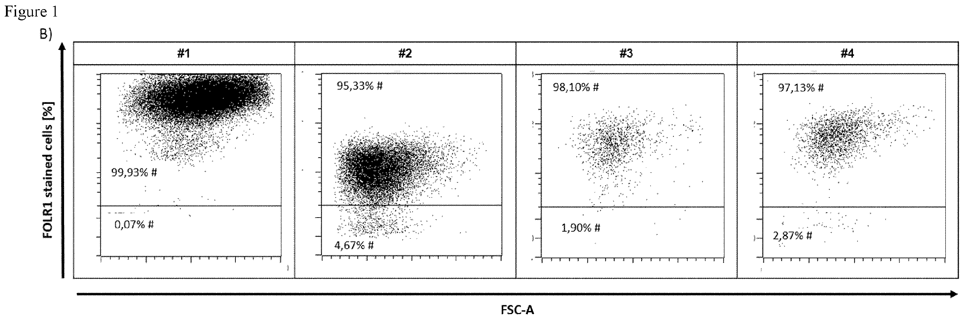

Figure 1: Flow cytometric screening assay reveals expression of FOLR1 on high-grade serous ovarian carcinoma: A) Gating strategy for the analysis of target expression on primary high grade serous ovarian carcinoma samples. In a first step debris are excluded from further analysis. Secondly, living cells were selected for further analysis. Third, doublets were excluded and, fourth, non-lineage cells were selected for final analysis of FOLR1 expression. B) Several human high-grade serous ovarian carcinoma (#1-#4) were dissociated and subsequently analyzed by flow cytometry for FOLR1 expression according to the gating strategy described in A).

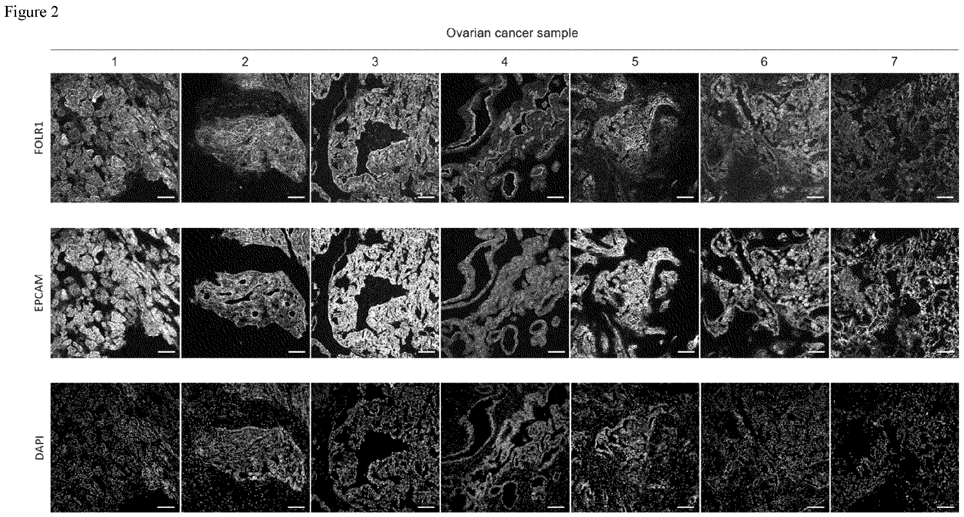

Figure 2 Ultrahigh-content imaging enables the discovery of FOLR1 as target for high-grade serous ovarian cancer. Fresh-frozen human high-grade serous ovarian cancer samples (each number indicates an individual patient sample) were sliced and analyzed by MICS for the expression of FOLR1 and EPCAM. DAPI and the indicated markers of interest are shown. Scale bar represents 100 µm.

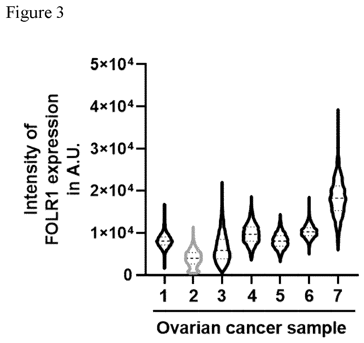

Figure 3 Ovarian cancer samples express elevated FOLR1 levels. Tumor marker FOLR1 expression was quantified on a single-cell level on primary ovarian cancer tissue (samples are numbered as in Figure 2). Image data sets were segmented using MACSiQ View Software based on nuclei, i.e. DAPI signal, and epithelial cell membrane marker EPCAM identifying individual cells. Single cells were analyzed for EPCAM and FOLR1 expression. Each data point represents a region of interest.

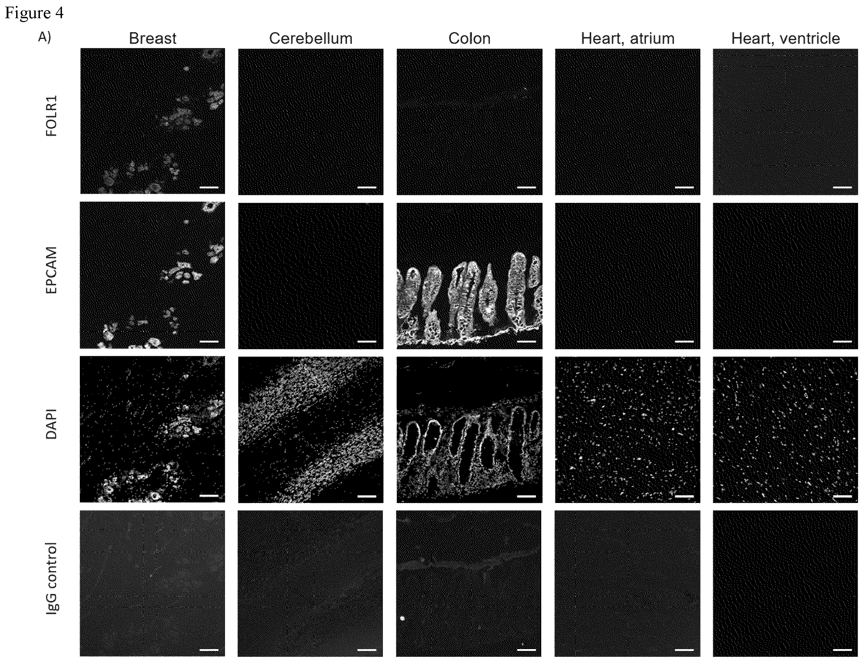

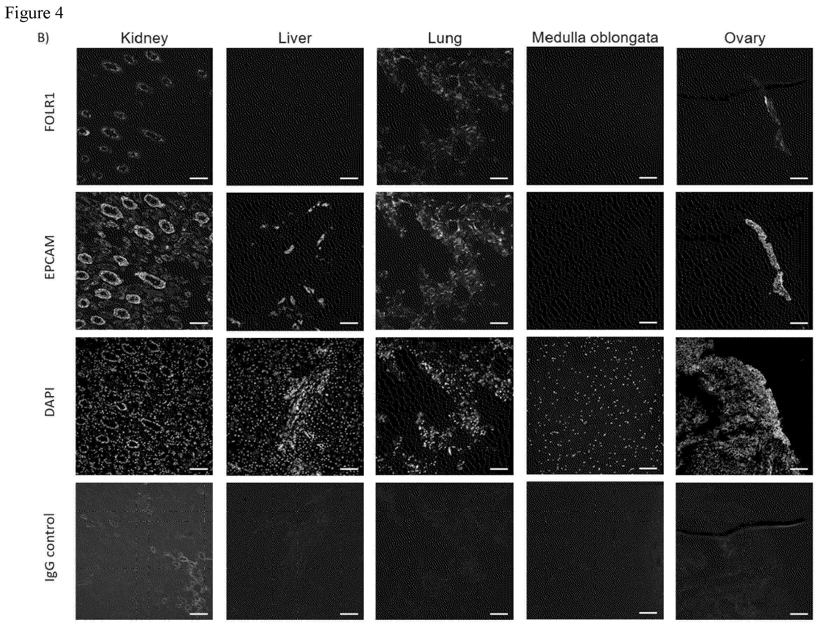

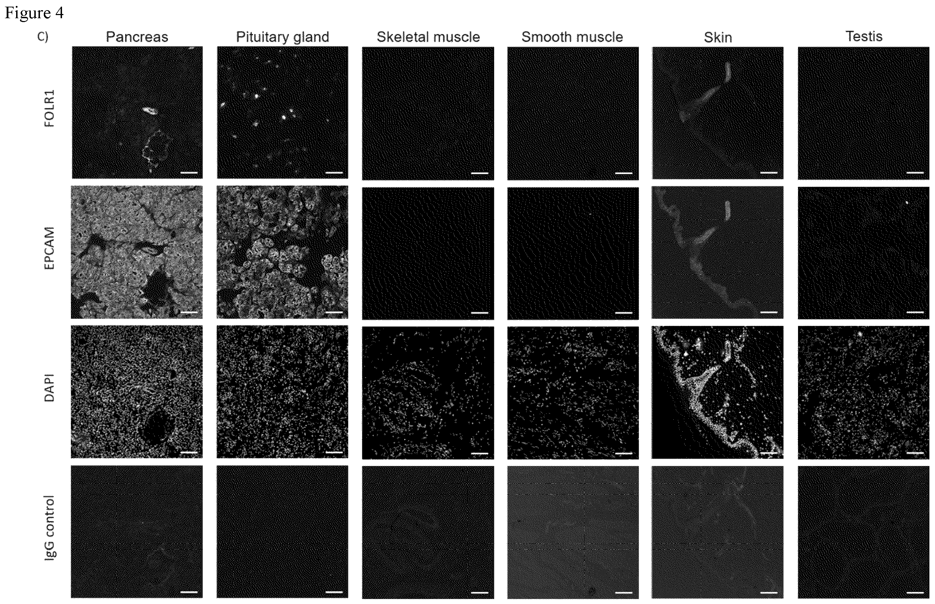

Figure 4 Ultrahigh-content imaging confirms expression of FOLR1 on healthy human tissues. Fresh-frozen human tissues were sliced and fixed with acetone. The subsequent screening was performed on the MACSima Imaging Platform by employing a sequential staining of antibodies. Healthy human tissues,
   A) breast, cerebellum, colon, heart atrium, heart ventricle,
   B) kidney, liver, lung, medulla oblongata, ovary,
   C) pancreas, pituitary gland, skeletal muscle, smooth muscle, skin and testis were analyzed for the expression of ovarian cancer target FOLR1 (top row) and EPCAM (second row from the top). DAPI (second row from bottom) visualizes nuclei and IgG control (bottom row) are depicted. Scale bar represents 100 µm.

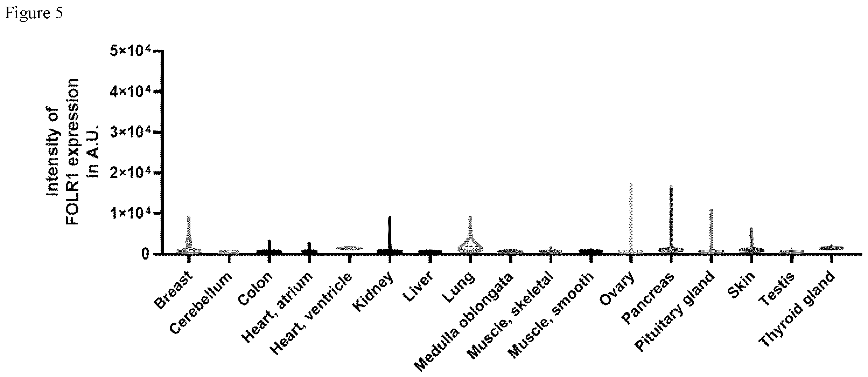

Figure 5 Various healthy tissues express no or low levels of FOLR1: Tumor marker FOLR1 expression was quantified on a single-cell level on healthy human tissue. Image data sets were segmented using MACSiQ View Software based on nuclei, i.e. DAPI signal, and epithelial cell membrane marker EPCAM identifying individual cells. Single cells were analyzed for EPCAM and FOLR1 expression. Each data point represents a region of interest.

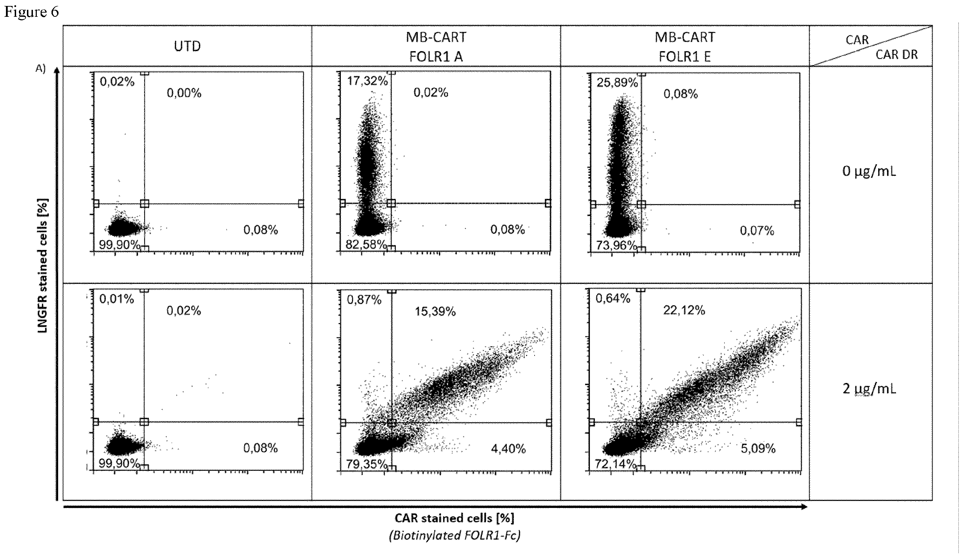

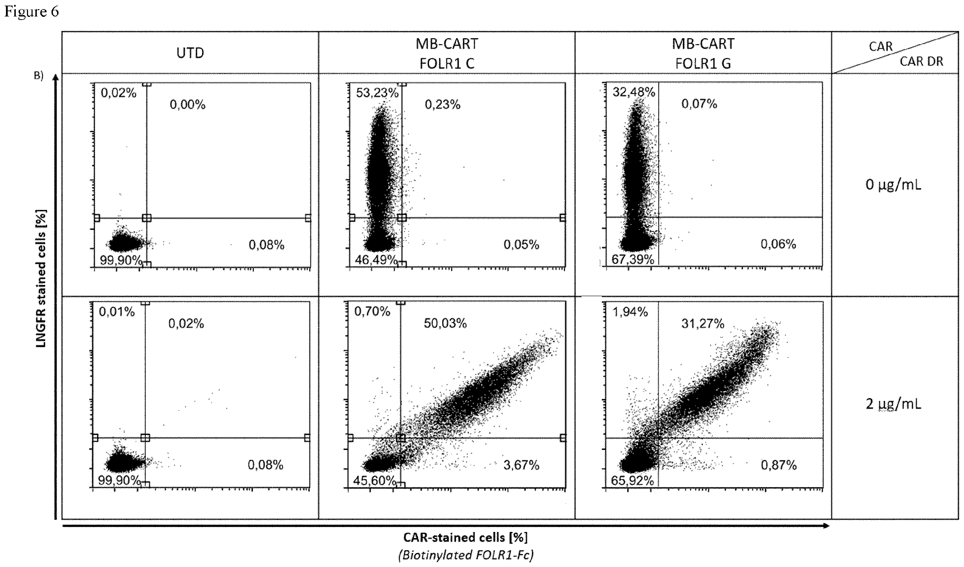

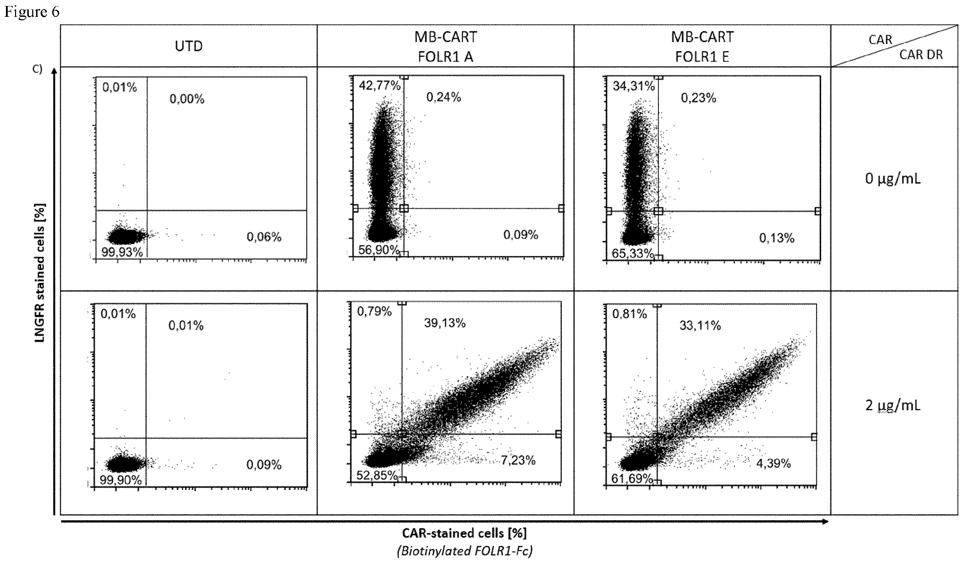

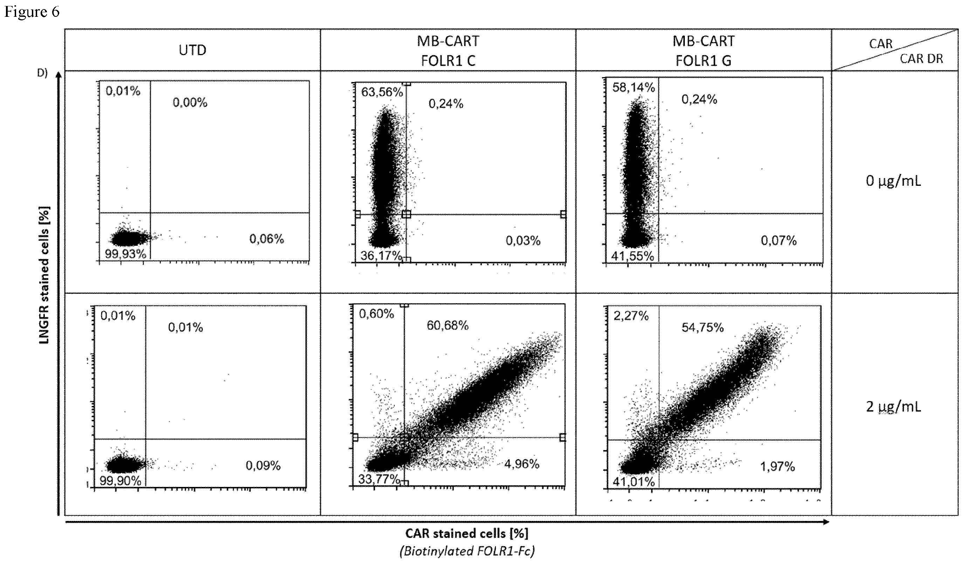

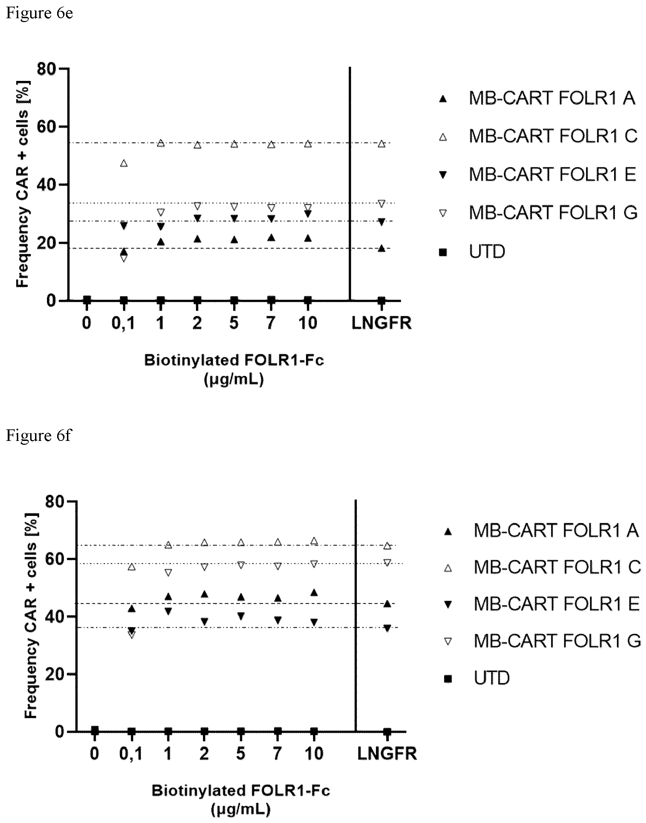

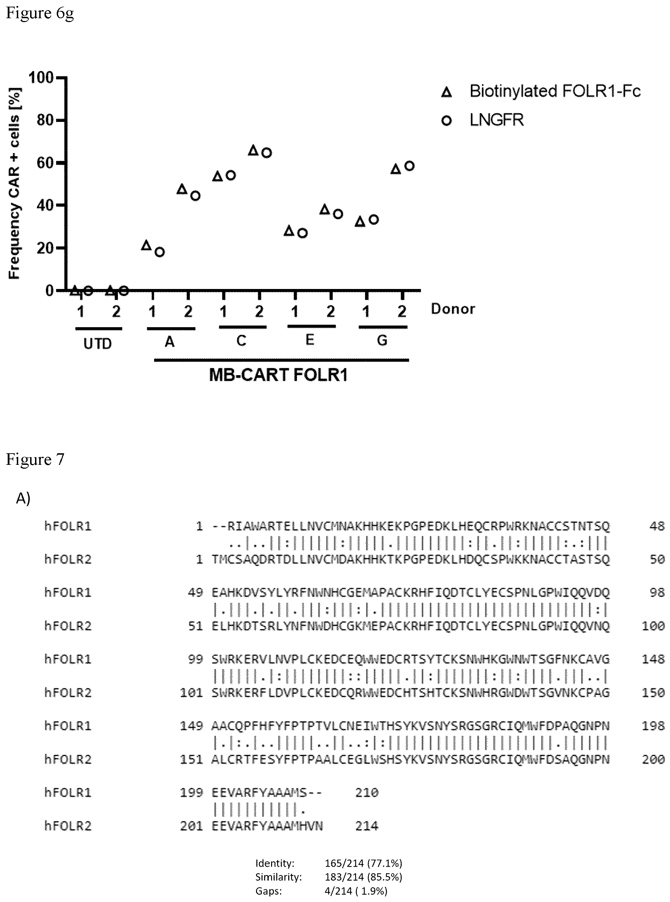

Figure 6 Anti-FOLR1 CAR T candidates are specifically and dose-dependently detected with biotinylated FOLR1-Fc fusion protein. Titration and flow cytometric analysis of biotinylated FOLR1-Fc fusion protein (CAR DR) on anti-FOLR1 CAR T cells from two independent donors using anti-biotin-PE secondary staining to determine anti-FOLR1 CAR expression. All flow cytometric data analyzed were selected among the viable CD3 positive population.
   A) Density plots from flow cytometric analysis of anti-FOLR1 CAR (candidate A and E) expression via biotinylated FOLR1-Fc fusion protein (in combination with anti-biotin-PE secondary staining) in transduced or untransduced T cells (UTD) from donor 1. CAR expression was detected via anti-LNGFR staining and co-staining without (upper row) or with biotinylated FOLR1-Fc protein (lower row) at 2 µg/mL.
   B) Density plots from flow cytometric analysis of anti-FOLR1 CAR (candidate C and G) expression via biotinylated FOLR1-Fc fusion protein (in combination with anti-biotin-PE secondary staining) in transduced or untransduced T cells (UTD) from donor 1. CAR expression was detected via anti-LNGFR staining and co-staining without (upper row) or with biotinylated FOLR1-Fc protein (lower row) at 2 µg/mL.
   C) Density plots from flow cytometric analysis of anti-FOLR1 CAR (candidate A and E) expression via biotinylated FOLR1-Fc fusion protein (in combination with anti-biotin-PE secondary staining) in transduced or untransduced T cells (UTD) from donor 2. CAR expression was detected via anti-LNGFR staining and co-staining without (upper row) or with biotinylated FOLR1-Fc protein (lower row) at 2 µg/mL.
   D) Density plots from flow cytometric analysis of anti-FOLR1 CAR (candidate C and G) expression via biotinylated FOLR1-Fc fusion protein (in combination with anti-biotin-PE secondary staining) in transduced or untransduced T cells (UTD) from donor 2. CAR expression was detected via anti-LNGFR staining and co-staining without (upper row) or with biotinylated FOLR1-Fc protein (lower row) at 2 µg/mL.
   E) Quantification of flow cytometric analysis of anti-FOLR1 CAR expression via biotinylated FOLR1-Fc fusion protein (in combination with anti-biotin-PE secondary staining) in transduced or untransduced T cells (UTD) from donor 1. Dotted lines represent the frequency of CAR-expressing T cells determined via CD3 and LNGFR double staining.
   F) Quantification of flow cytometric analysis of anti-FOLR1 CAR expression via biotinylated FOLR1-Fc fusion protein (in combination with anti-biotin-PE secondary staining) in transduced or untransduced T cells (UTD) from donor 2. Dotted lines represent the frequency of CAR-expressing T cells determined via CD3 and LNGFR double staining.
   G) Comparison of anti-FOLR1 CAR expression in T cells derived from two independent donors (donor 1 and donor 2) via flow cytometry measuring anti-LNGFR and biotinylated FOLR1-Fc fusion protein staining.

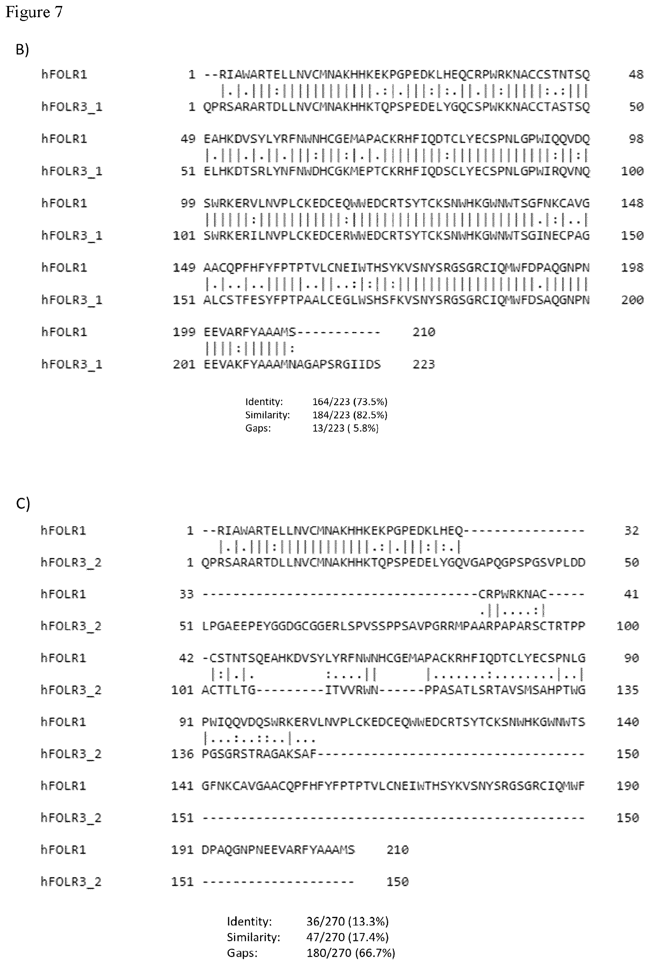

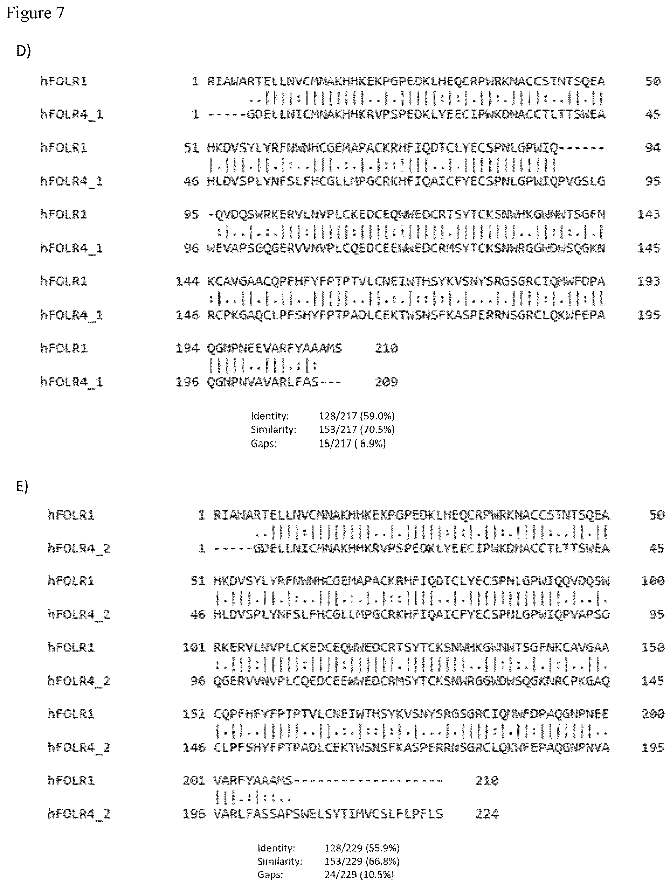

Figure 7 Pairwise protein sequence alignments of human FOLR variants reveal high degree of identity and similarity. Human FOLR1 (Gene ID: 2348) protein sequence (P15328, aa25-235) was aligned with human FOLR variants using EMBOSS needle algorithm (matrix: EBLOSUM62, gap_penalty: 10.0, extend_penalty: 0.5).
   A) Human FOLR1 (Gene ID: 2348) protein sequence (P15328, aa25-235, Seq ID No:69) was aligned with human FOLR2 (Gene ID: 2350) protein sequence (P14207, aa17-230, Seq ID No:70).
   B) Human FOLR1 (Gene ID: 2348) protein sequence (P15328, aa25-235, Seq ID No:69) was aligned with human FOLR3 (Gene ID: 2352) isoform1 protein sequence (P41439, aa23-245, Seq ID No:71).
   C) Human FOLR1 (Gene ID: 2348) protein sequence (P15328, aa25-235, Seq ID No:69) was aligned with human FOLR3 (Gene ID: 2352) isoform2 protein sequence (P41439-4, aa23-172, Seq ID No:72).
   D) Human FOLR1 (Gene ID: 2348) protein sequence (P15328, aa25-235, Seq ID No:69) was aligned with human FOLR4 (Gene ID: 390243) isoform1 protein sequence (A6ND01, aa20-228, Seq ID No:73).
   E) Human FOLR1 (Gene ID: 2348) protein sequence (P15328, aa25-235, Seq ID No:69) was aligned with human FOLR4 (Gene ID: 390243) isoform2 protein sequence (A6ND01-2, aa20-243, Seq ID No:74).
Figure 8 Recombinant Jurkat cell lines express human and mouse HA-tagged FOLR variants. Density plots from flow cytometric analysis of Jurkat cells wt or Jurkat cells expressing different human (h) or mouse (m) FOLR variants. HA-tagged FOLR variants expression was detected via an anti-3xHA-tag staining.
Figure 9 Anti-FOLR1 CAR T candidates express activation markers antigen-dependently. Anti-FOLR1 CAR T cell candidates
   A) MB-CART FOLR1 A ,
   B) MB-CART FOLR1 B,
   C) MB-CART FOLR1 C ,
   D) MB-CART FOLR1 D,
   E) MB-CART FOLR1 E,
   F) MB-CART FOLR1 F,
   G) MB-CART FOLR1 G,
   H) MB-CART FOLR1 H, or
   I) untransduced T cells (UTD)
   were co-cultured with Jurkat wt cells or Jurkat cells expressing different human (h) or mouse (m) FOLR variants at an effector cell : target cell ratio of 2:1. After 48 hours of co-culture, CAR T cells were assessed for activation marker expression by flow cytometry. Data points represent mean of technical triplicates and are presented as fold change relative to the respective T cell co-culture with FOLR-deficient Jurkat wt cells. Each bar represents the mean +/- SEM.

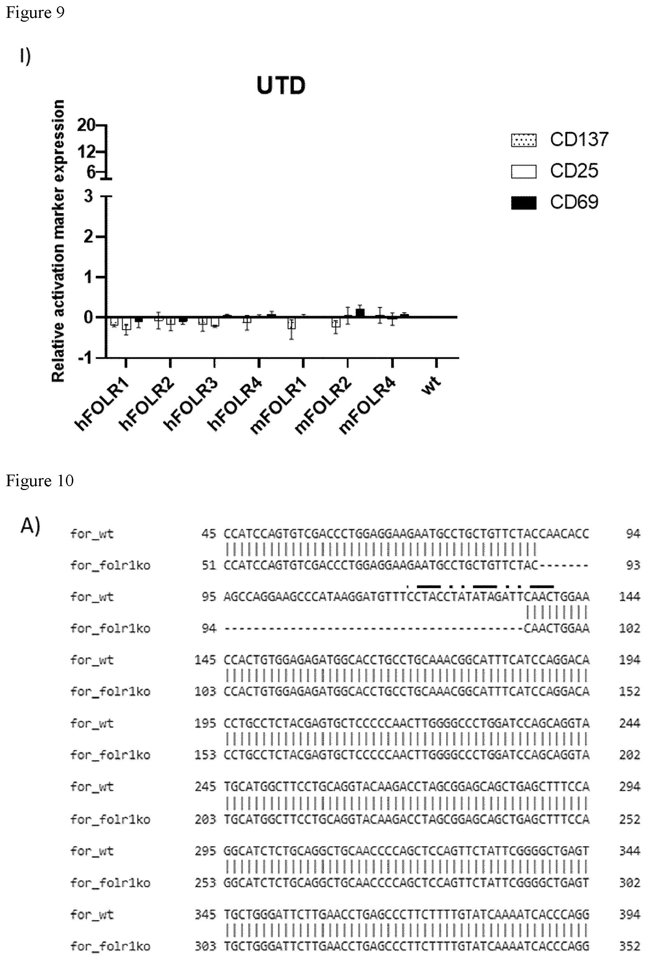

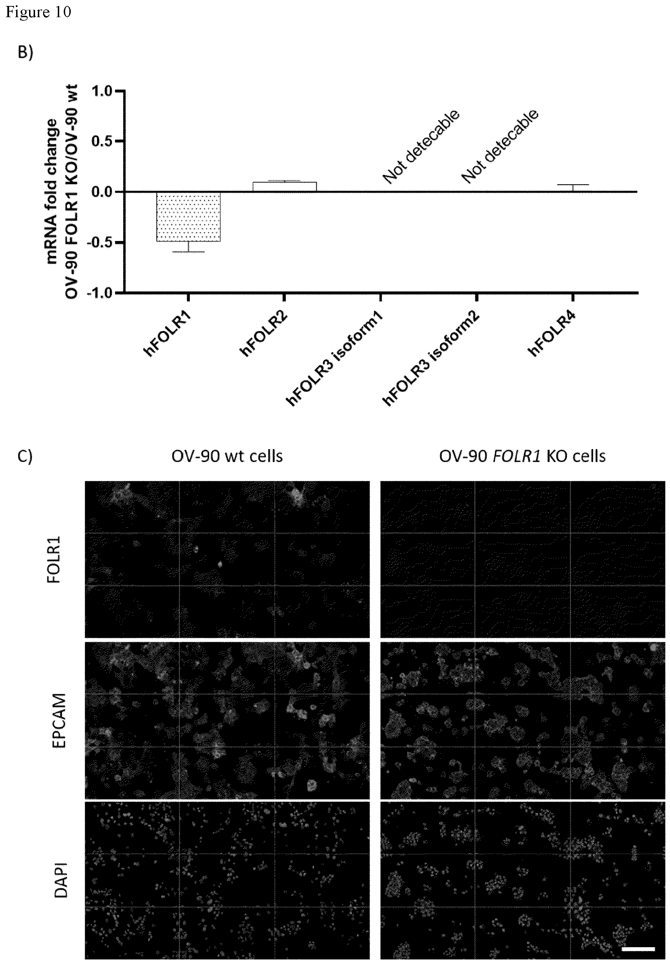

Figure 10 CRISPR/CAS9-mediated FOLR1 knock-out in OV-90 cells is validated at genome, transcript, and protein level. Deletion in FOLR1 led to homozygous knock-out in OV-90 cells.
   A) Alignment of OV-90 wt (for_wt, Seq ID No:112) and OV-90 FOLR1 KO (for folrko; Seq ID No: 113) genomic DNA sequences using EMBOSS needle algorithm (matrix: EBLOSUM62, gap_penalty: 10.0, extend_penalty: 0.5). Sequencing was performed with specific FOLR1 gDNA primers (Seq ID No:90 and Seq ID No:91). The sequence highlighted with dashed-dotted line corresponds to the guide RNA sequence (Seq ID No:89) used for the CRISPR/CAS9-mediated knock-out of FOLR1.
   B) Relative mRNA expression of folate receptor variants in OV-90 FOLR1 KO compared to OV-90 wt cells. RT-qPCR analysis of mRNA isolated from OV-90 FOLR1 KO and wt cells. FOLR transcripts were amplified with primer pairs for different folate receptors variants corresponding to the SEQ ID No:92-101. Individual gene expression is analyzed by ΔΔCt method, normalized to GAPDH mRNA expression and fold change is relative to OV-90 wt mRNA levels. Data is shown as mean ± SEM of duplicates.
   C) Immune fluorescence staining of OV-90 wt as well as OV-90 FOLR1 KO cells. Cells were analyzed for the expression of ovarian cancer target FOLR1 and EPCAM. DAPI visualizes nuclei. Scale bar represents 100 µm.

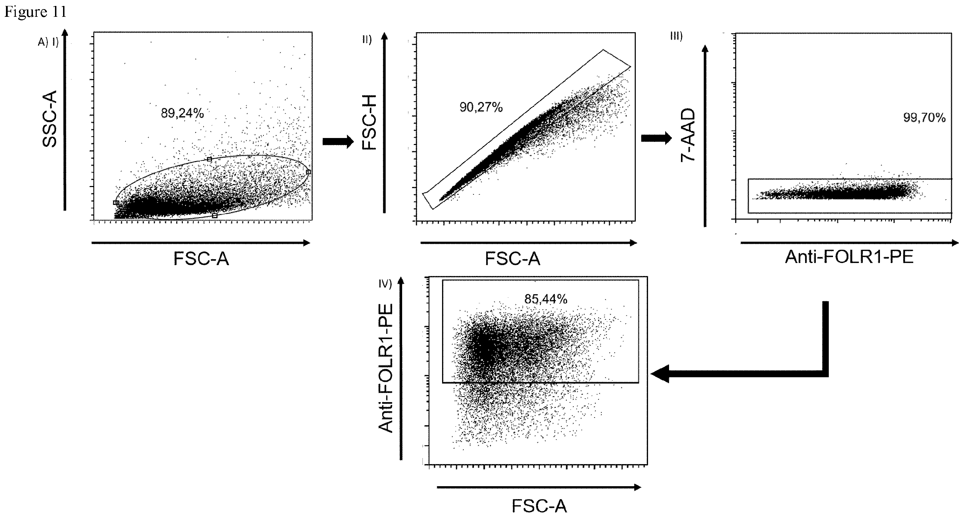

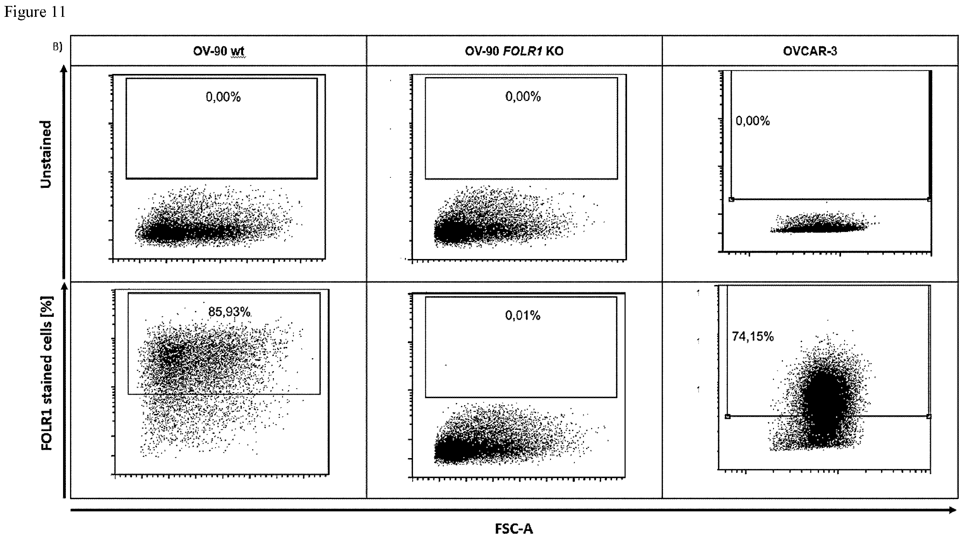

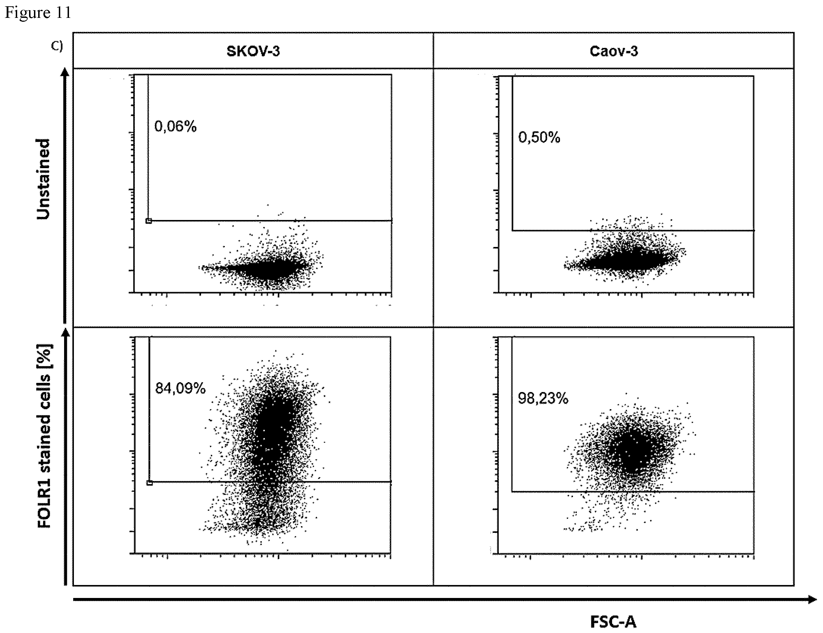

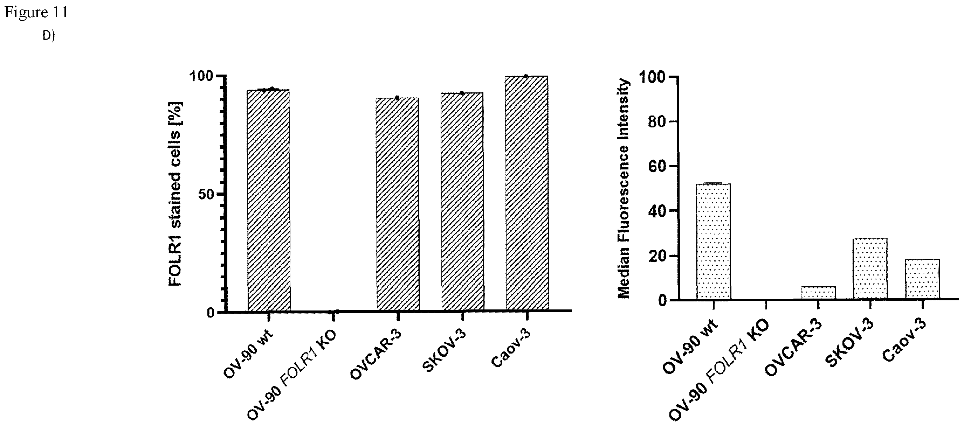

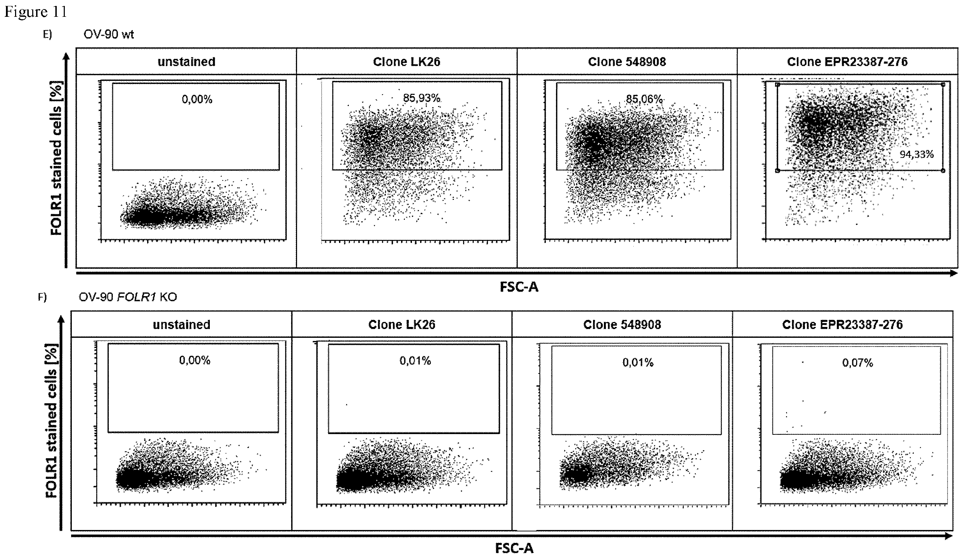

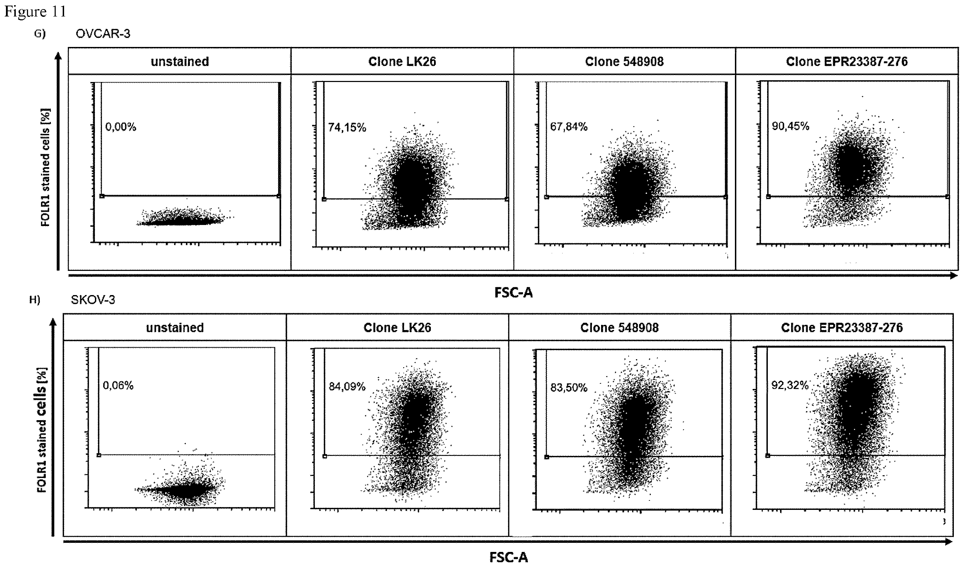

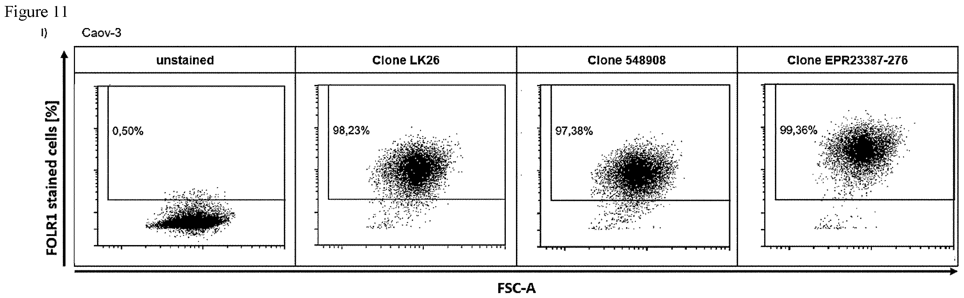

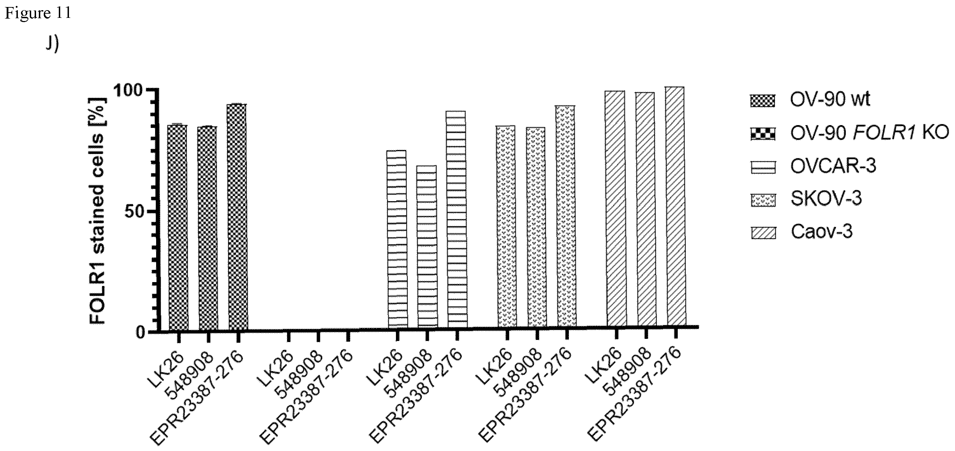

Figure 11 Ovarian cancer cell lines differentially express FOLR1. Ovarian cancer cell lines were analyzed by flow cytometry to characterize FOLR1 expression.
   A) Gating strategy for the detection of FOLR1 expression on ovarian cancer cell lines. Ovarian cancer cell lines were stained by flow cytometry with an anti-FOLR1-PE antibody and density plots analyzed with the depicted gates. First debris were excluded (I). Subsequently, the selected cell population is gated for singlets (II). Viable cells from singlets population are determined by negative 7-AAD signal (III). Finally, the viable cells are analyzed for FOLR1 expression (IV). Arrows indicate subgating steps.
   B) Density plots of viable ovarian cancer cells (OV-90 wt, OV-90 FOLR1 KO, OVCAR-3) unstained (top row) or stained with anti-FOLR1-PE antibody (bottom row) are shown. Flow cytometric analysis of indicated ovarian cancer cell lines gated as described in (A).
   C) Density plots of viable ovarian cancer cells (SKOV-3, Caov-3) unstained (top row) or stained with anti-FOLR1-PE antibody (bottom row) are shown. Flow cytometric analysis of indicated ovarian cancer cell lines gated as described in (A).
   D) Quantification of FOLR1 expression from B) via cell frequency (left) as well as median fluorescence intensity (right) is shown. Data points represent mean ± SEM.
   E) Density plots of viable ovarian cancer cells (OV-90 wt) unstained or stained with different anti-FOLR1 antibodies are shown. Flow cytometric analysis of indicated ovarian cancer cell lines gated as described in (A). Various ovarian cancer cell lines were analyzed by flow cytometry using different anti-FOLR1 antibody clones to exclude clone-bias.
   F) Density plots of viable ovarian cancer cells (OV-90 FOLR1 KO) unstained or stained with different anti-FOLR1 antibodies are shown. Flow cytometric analysis of indicated ovarian cancer cell lines gated as described in (A). Various ovarian cancer cell lines were analyzed by flow cytometry using different anti-FOLR1 antibody clones to exclude clone-bias.
   G) Density plots of viable ovarian cancer cells (OVCAR-3) unstained or stained with different anti-FOLR1 antibodies are shown. Flow cytometric analysis of indicated ovarian cancer cell lines gated as described in (A). Various ovarian cancer cell lines were analyzed by flow cytometry using different anti-FOLR1 antibody clones to exclude clone-bias.
   H) Density plots of viable ovarian cancer cells (SKOV-3) unstained or stained with different anti-FOLR1 antibodies are shown. Flow cytometric analysis of indicated ovarian cancer cell lines gated as described in (A). Various ovarian cancer cell lines were analyzed by flow cytometry using different anti-FOLR1 antibody clones to exclude clone-bias.
   I) Density plots of viable ovarian cancer cells (Caov-3) unstained or stained with different anti-FOLR1 antibodies are shown. Flow cytometric analysis of indicated ovarian cancer cell lines gated as described in (A). Various ovarian cancer cell lines were analyzed by flow cytometry using different anti-FOLR1 antibody clones to exclude clone-bias.
   J) Quantification of FOLR1 expression from D) via cell frequency is shown. Data points represent mean ± SEM.

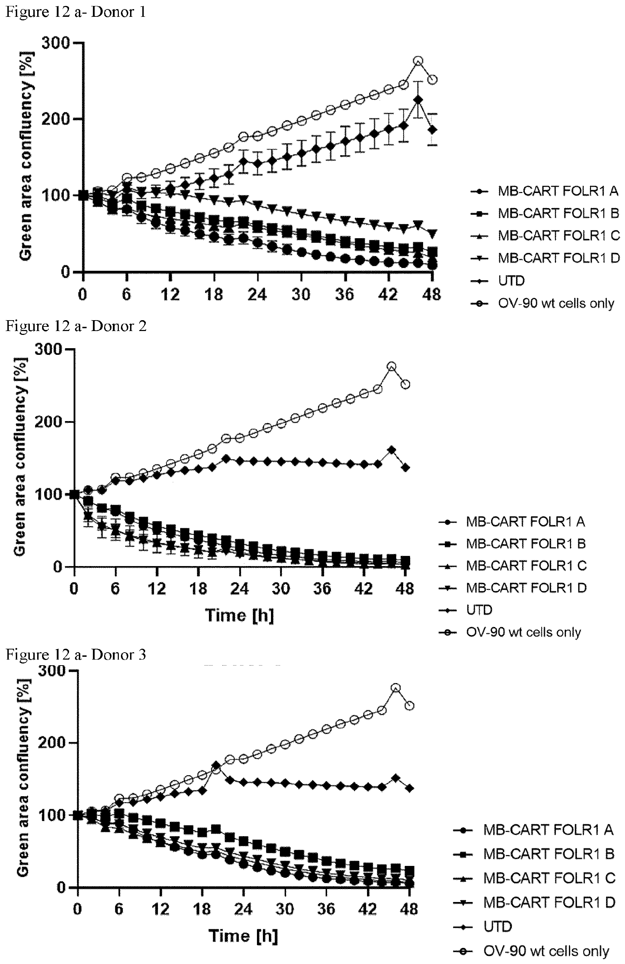

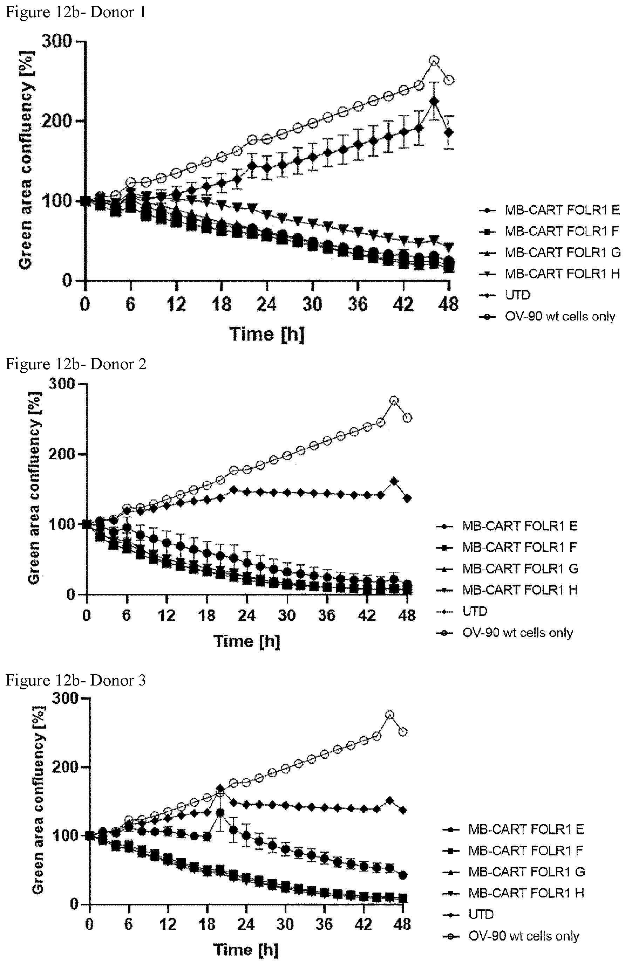

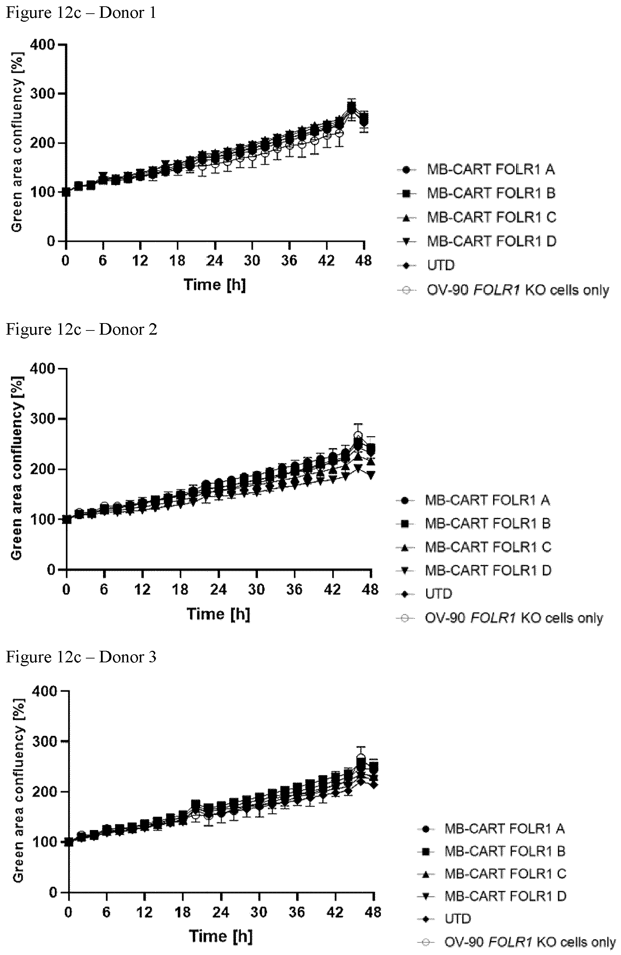

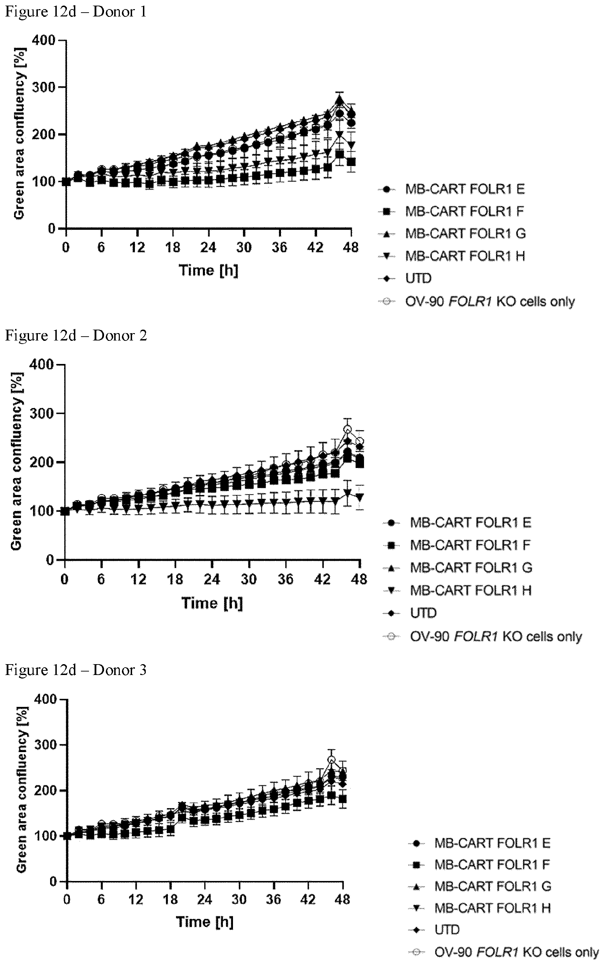

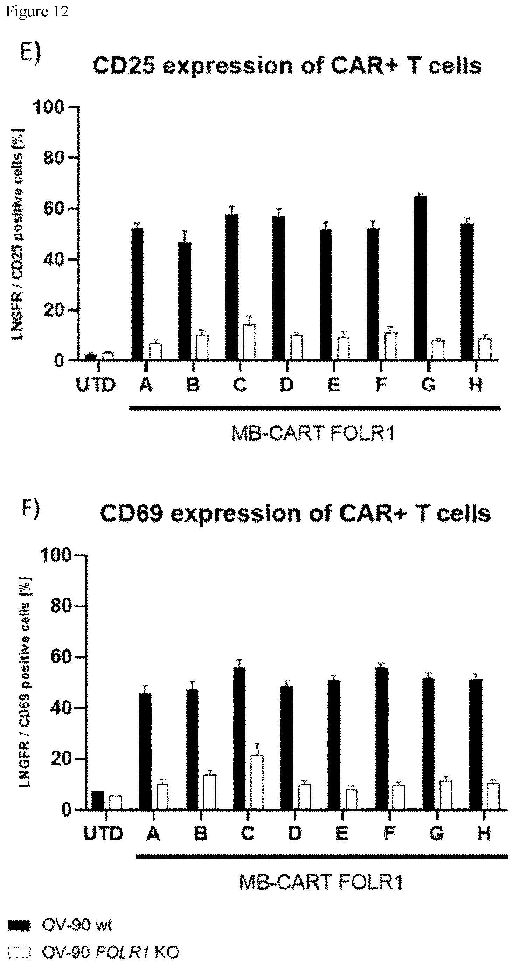

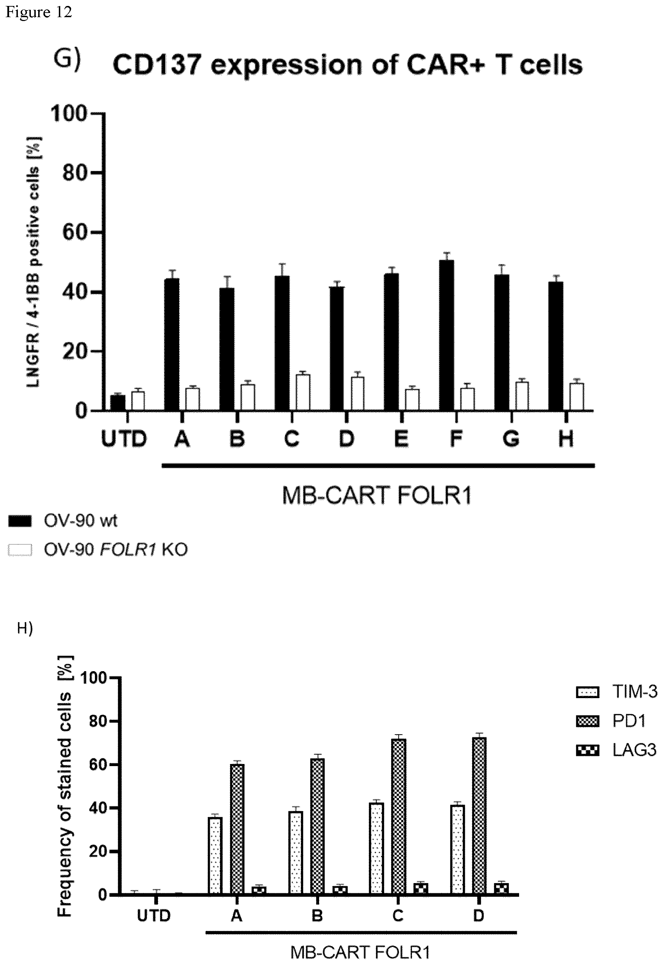

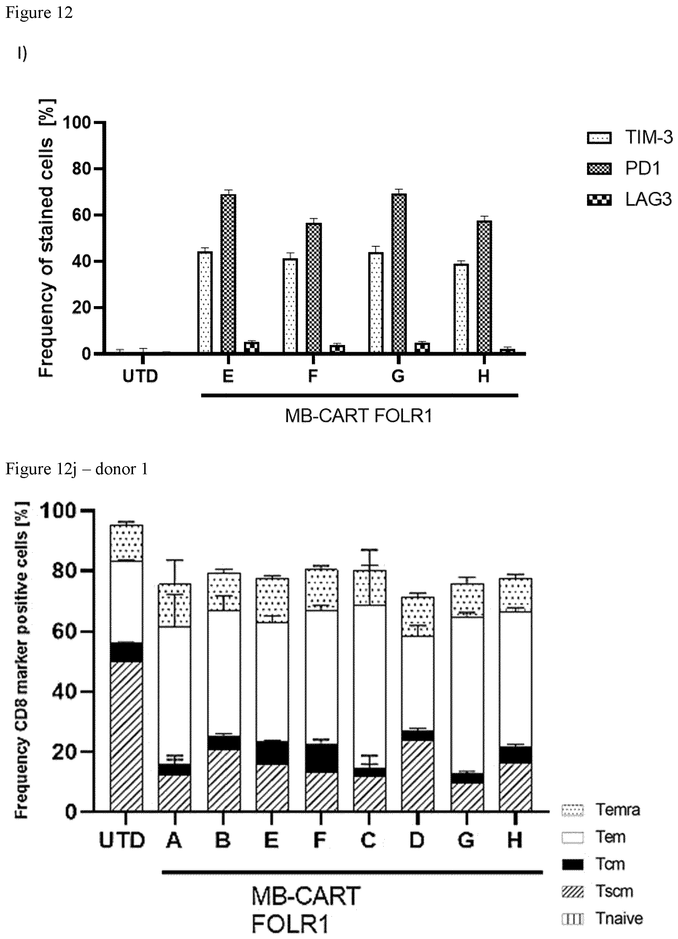

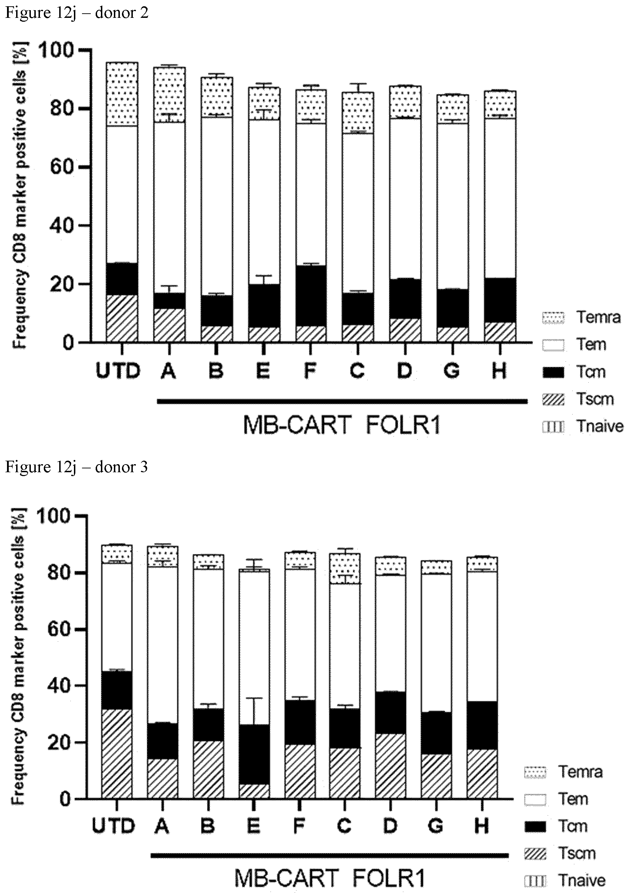

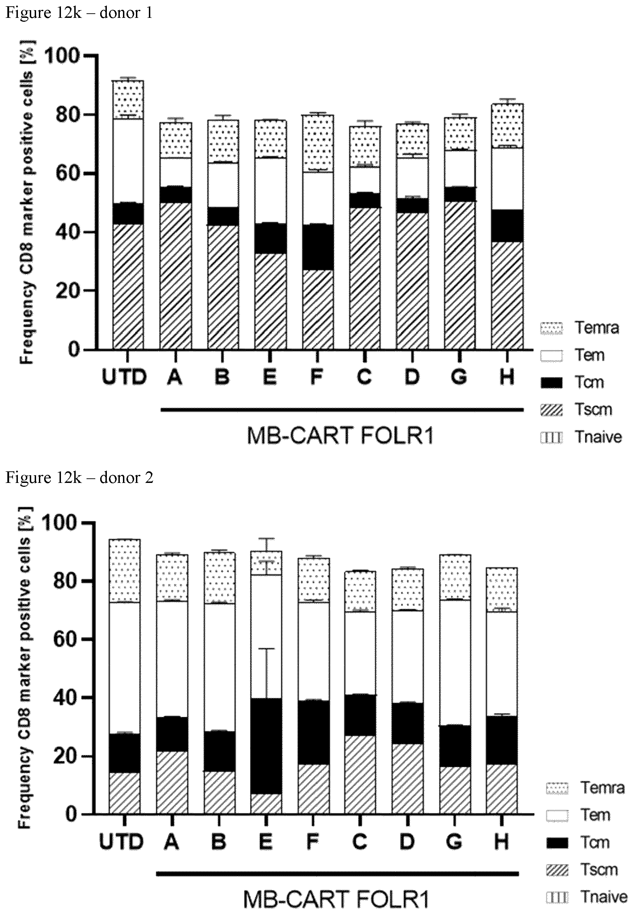

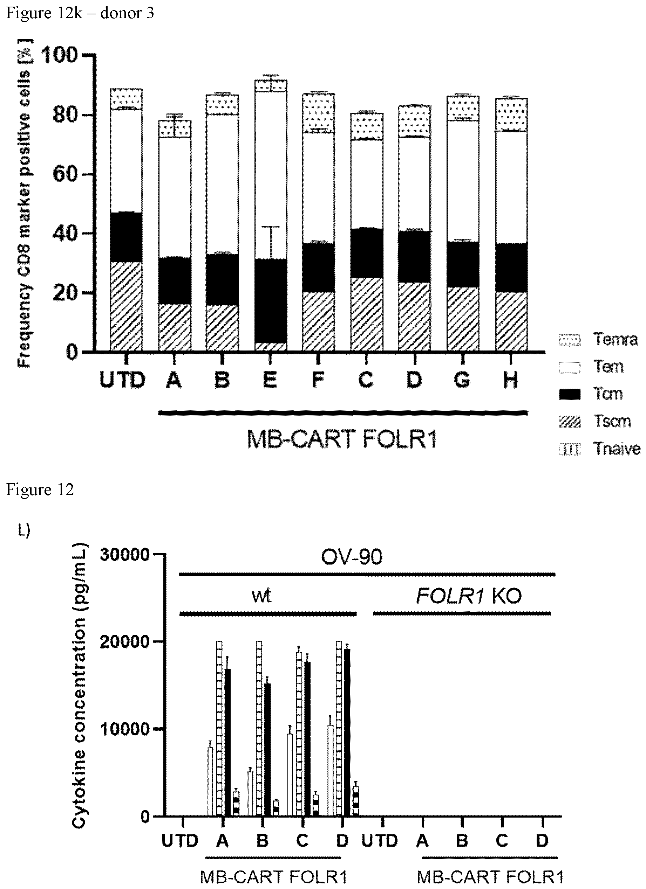

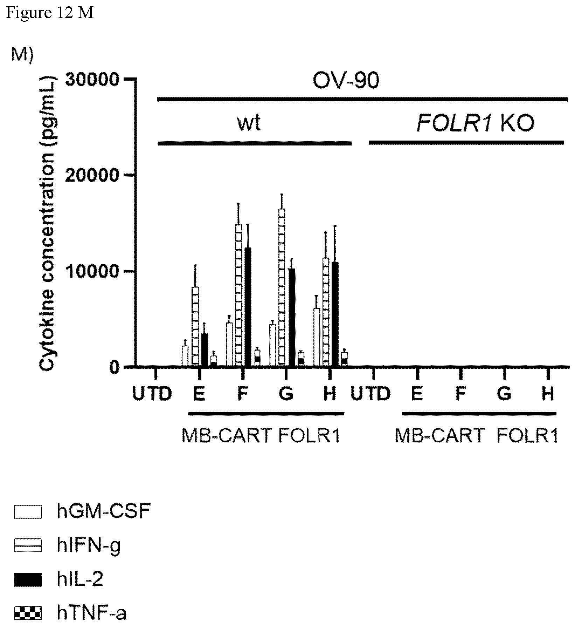

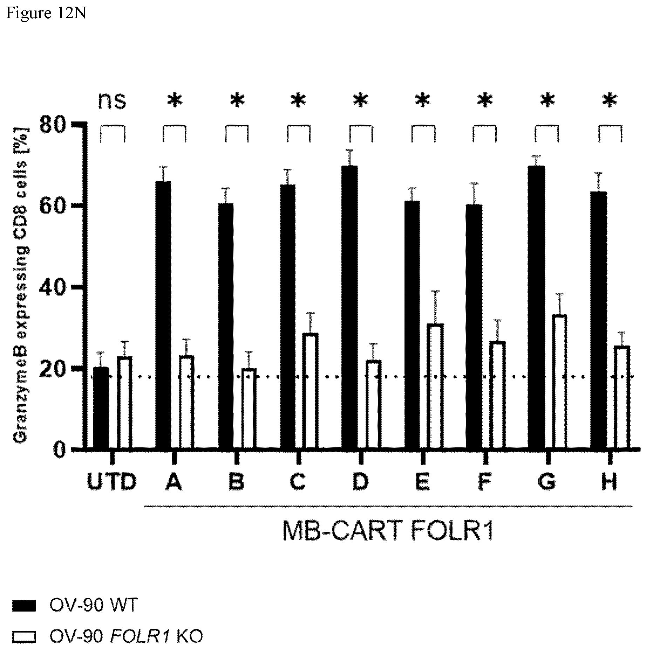

Figure 12 Anti-FOLR1 CAR T cells are functional *in vitro* in an antigen-dependent manner. Anti-FOLR1 CAR T cells or untransduced T cells (UTD) from three independent donors were co-cultured for 48 hours at an effector cell : target cell ratio of 2:1
   with OV-90 wt cells,
   A) MB-CART FOLR1 candidates A-D,
   B) MB-CART FOLR1 candidates E-H, or
      with OV-90 FOLR1 KO cells
   C) MB-CART FOLR1 candidates A-D,
   D) MB-CART FOLR1 candidates E-H.
   Data points of each group are normalized to baseline (defined as 100% confluency at the first timepoint (T=0 hours)) and each datapoint represents mean +/- SEM (n=3).
   Anti-FOLR1 CAR T cells antigen-dependently express activation markers . After 48 hours of co-culture with OV-90 or with OV-90 FOLR1 KO cells, respectively, CAR T cells were assessed for activation marker expression
   E) CD25,
   F) CD69, and
   G) CD137
   by flow cytometry. Each bar represents the mean +/- SEM of three replicates from three donors (n=9).
   Anti-FOLR1 CAR T cells antigen-dependently express exhaustion markers. Anti-FOLR1 CAR T cells
   H) candidates A-D,
   I) candidates E-H (I)) or untransduced T cells (UTD)
   were co-cultured with OV-90 wt cells at an Effector cell : target cell ratio of 2:1. After 48 hours of co-culture, T cells were assessed for exhaustion marker TIM-3, PD1, and LAG3 expression by flow cytometry, respectively. Each bar represents the mean +/- SEM of three replicates from three donors (n=9).
   Anti-FOLR1 CAR T cells (CD8 T cells) were phenotyped after co-cultivation. Anti-FOLR1 CAR T cells or untransduced T cells (UTD) from three independent donors were co-cultured with
   J) OV-90 wt cells or
   K) OV-90 FOLR1 KO cells at an effector cell : target cell ratio of 2:1. After 48 hours of co-culture, T cells were assessed for phenotype markers expression by flow cytometry. T cell subtypes were defined as following: Tscm - CD45RO- CD197+ CD62L+ CD95+, Tnaive - CD45RO- CD197+ CD62L+ CD95-, Temra - CD45RO- CD197- CD62L- CD95+, Tern - CD45RO+ CD62L- CD95+, and Tcm - CD45RO+ CD62L+ CD95+. Each bar represents mean +/- SEM (n=3).
   Anti-FOLR1 CAR T cells antigen-dependently secrete cytokines in co-culture experiments with OV-90 wt and not OV-90 FOLR1 KO cells. Anti-FOLR1 CAR T cell candidates
   L) A-D and
   M) E-H or untransduced T cells (UTD)
   from three independent donors were co-cultured with OV-90 wt or OV-90 FOLR1 KO cells at an effector cell : target cell ratio of 2:1. Supernatants were collected after 24 hours of co-culture and cytokine concentration was subsequently determined with the human MACSplex Cytokine 12 Kit. Each bar represents the mean +/- SEM of three replicates from three donors (n=9). Anti-FOLR1 CAR T cells antigen-dependently express Granzyme B in co-culture experiments with OV-90 wt and not OV-90 FOLR1 KO cells.
   N) Anti-FOLR1 CAR T cell candidates A-H or untransduced T cells (UTD) from three independent donors were co-cultured with OV-90 wt or OV-90 FOLR1 KO cells at an effector cell : target cell ratio of 2:1. T cells were collected after 24h of co-culture, and protein secretion was blocked by incubation with Brefeldin A (1 µg/mL) overnight. Cells were subsequently fixed, stained for CD8 surface marker, permeabilized and finally intracellular Granzyme B was detected via flow cytometry. Each bar represents the mean +/- SEM of three replicates from three donors (n=9).

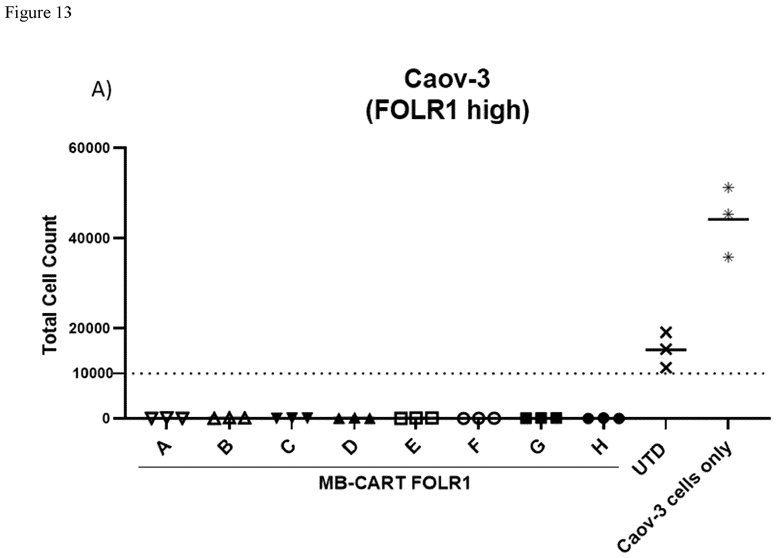

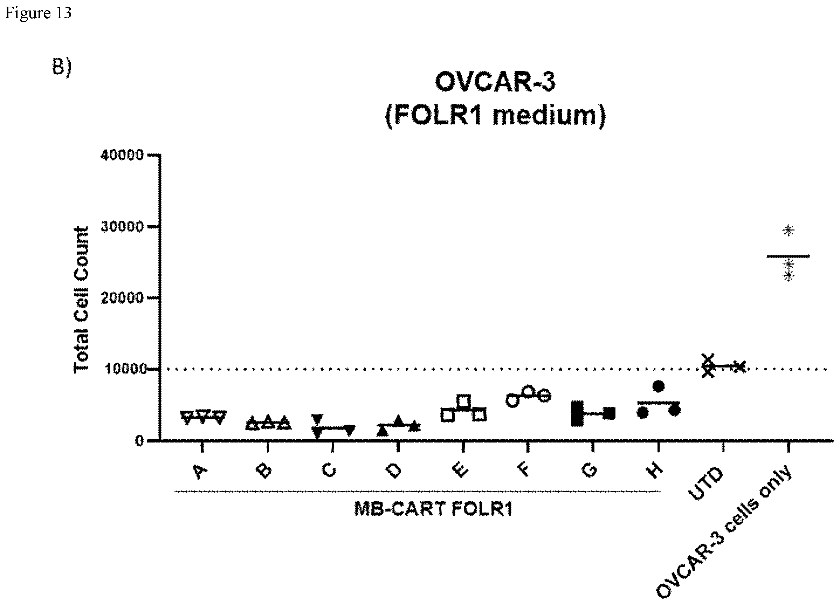

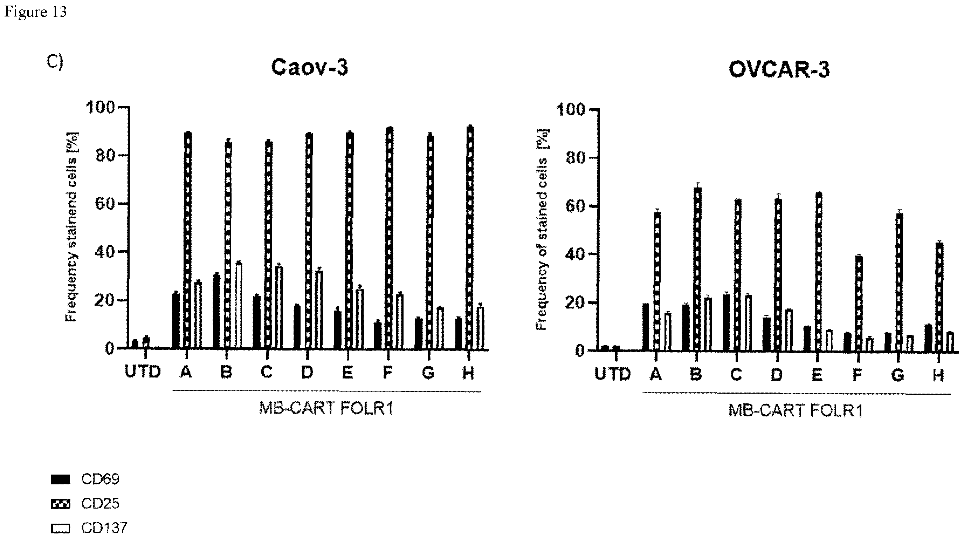

Figure 13 Anti-FOLR1 CAR T cells mediate antigen-dependent lysis of ovarian cancer cell lines differentially expressing FOLR1.
   Anti-FOLR1 CAR T cells or untransduced T cells (UTD) were co-cultured with
   A) Caov-3 (high FOLR1 expression ) or
   B) OVCAR-3 (medium FOLR1 expression)
   ovarian cancer cell lines co-expressing GFP and luciferase for 48 hours at an effector cell : target cell ratio of 2:1. After 48 hours of co-culture, the viable target cell numbers were analyzed by flow cytometry determined by 7-AAD staining. Dashed lines indicate starting target cell number and continuous lines represent mean of the triplicates.
   C) Anti-FOLR1 CAR T cells antigen-dependently express activation markers after co-cultivation with Caov-3 or OVCAR-3, respectively. Anti-FOLR1 CAR T cells or untransduced T cells (UTD) were co-cultured with target cells at an effector cell : target cell ratio of 2:1. After 48 hours of co-culture, T cells were assessed for activation marker CD25, CD69, and CD137 expression by flow cytometry. Data points represent the mean +/- SEM (n=3).

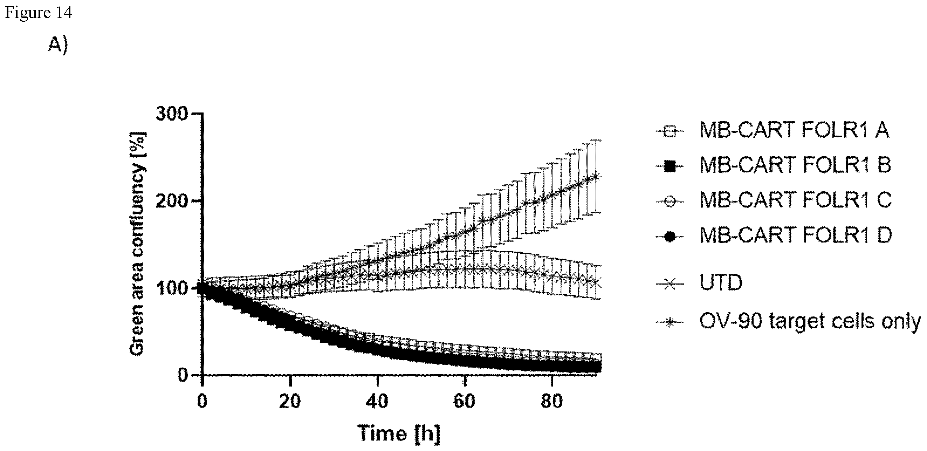

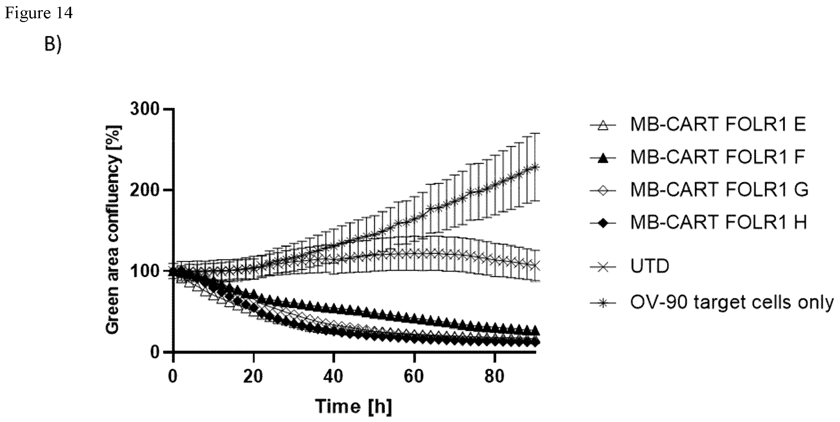

Figure 14 Anti-FOLR1 CAR T cells are cytolytically active against xenograft-derived OV-90 wt target cells *in vitro.*
   OV-90 wt cell-derived xenografts from NSG mice were dissociated in single cell suspension. Isolated tumor cells were subsequently cultured *in vitro.* Anti-FOLR1 CAR T cells
   A) candidates A-D,
   B) candidates E-H, or untransduced T cells (UTD)
   were co-cultured with xenograft-derived OV-90 wt cells for 90 hours at an effector cell : target cell ratio of 2:1. Data points of each group are normalized against the baseline (defined as 100% confluency at the first timepoint (T=0 hours)) and each datapoint represents the respective mean +/- SEM (n=3).

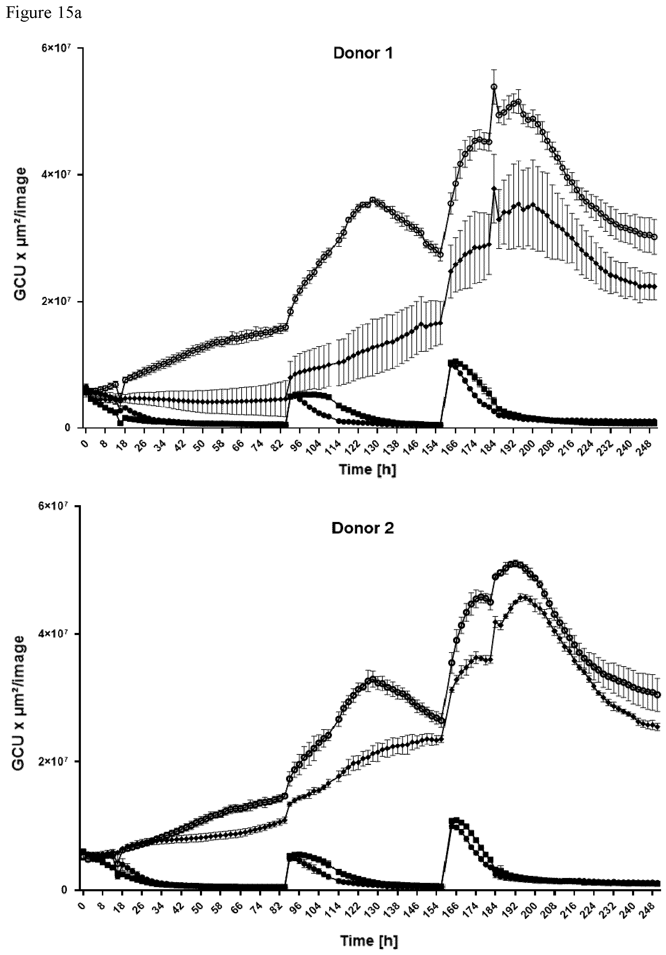

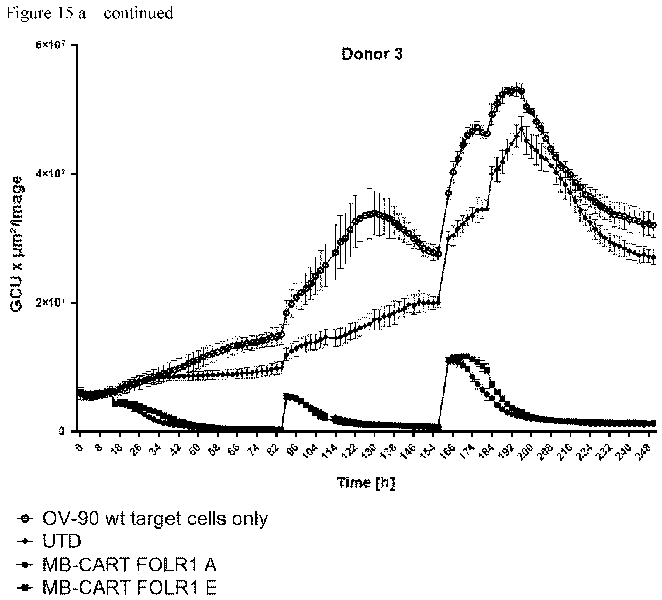

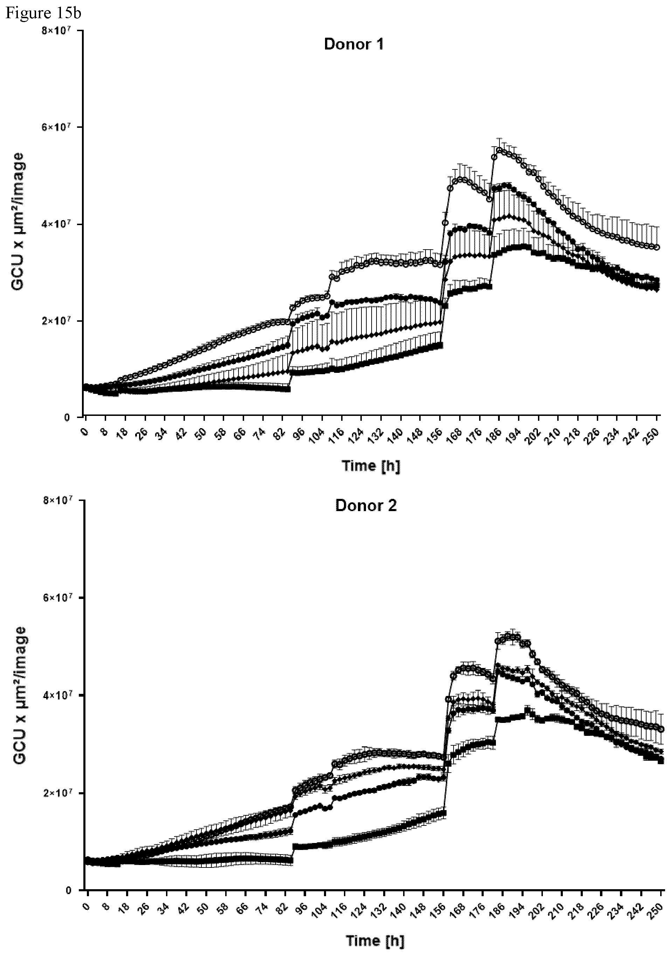

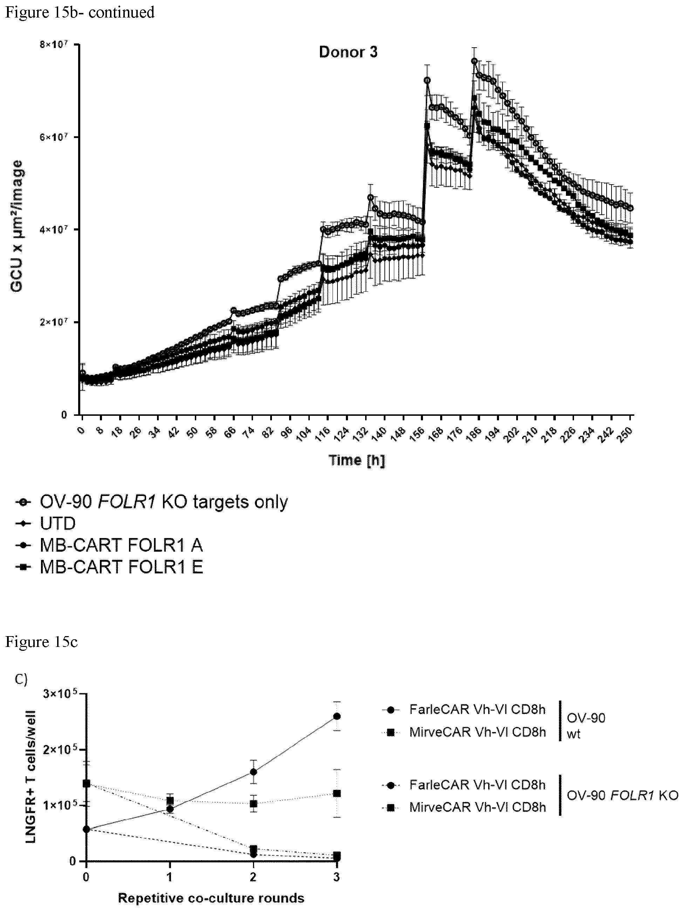

Figure 15 Anti-FOLR1 CAR T cells are repeatedly challenged with OV-90 cells and retain cytolytic activity *in vitro.* Serial killing assays performed in co-cultures of anti-FOLR1 CAR T cells with OV-90 cells with three individual donors. Anti-FOLR1 CAR T cell candidates (MB-CART-FOLR1 A and E) or untransduced T cells (UTD) were co-cultured with
   A) OV-90 wt cells or
   B) OV-90 FOLR1 KO cells
      for 250 hours at an initial effector cell : target cell ratio of 2.5:1 (50,000 CAR T cells : 20,000 OV-90 cells). After 92 hours 20,000 OV-90 target cells/ well were added into the co-culture. Subsequently, after 164 hours total T cells co-culture, 50,000 OV-90 target cells/ well were added into the co-culture. Data points are normalized and each represent mean +/- SD of triplicates well from a single donor (n=3).
   C) Expansion of CAR T cells was analyzed over several rounds of co-culture with OV-90 wt and OV-90 FOLR1 KO cells, respectively, by flow cytometry and LNGFR staining for CAR-expressing T cells. Data points represent mean +/- SEM of duplicate wells from three donor (n=6).

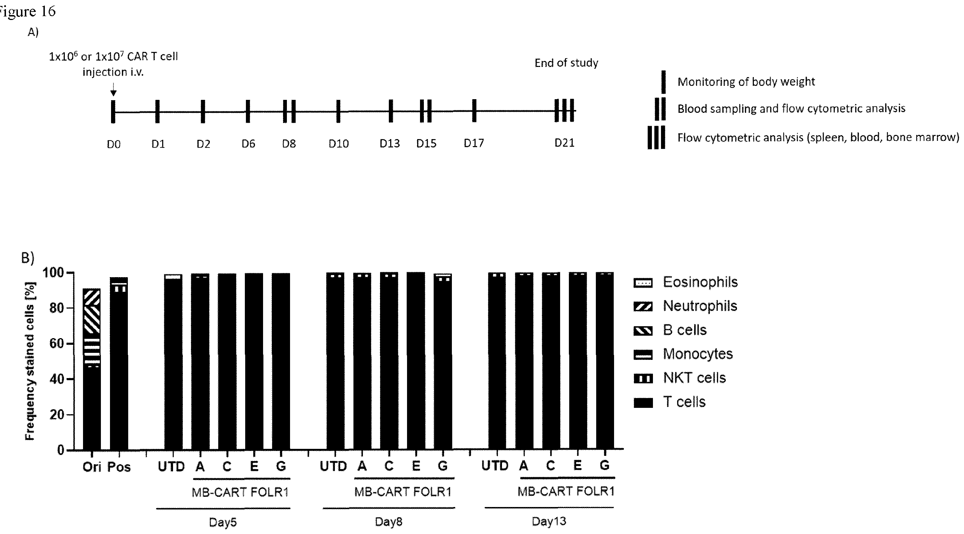

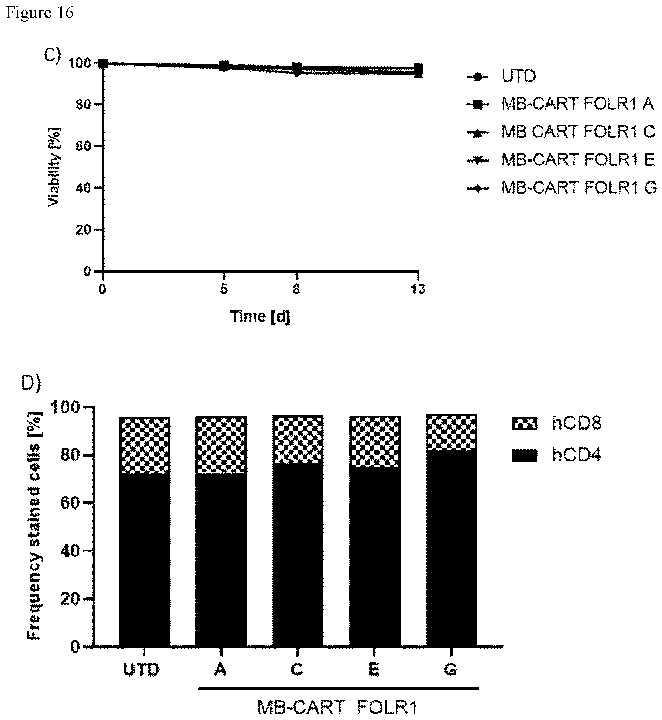

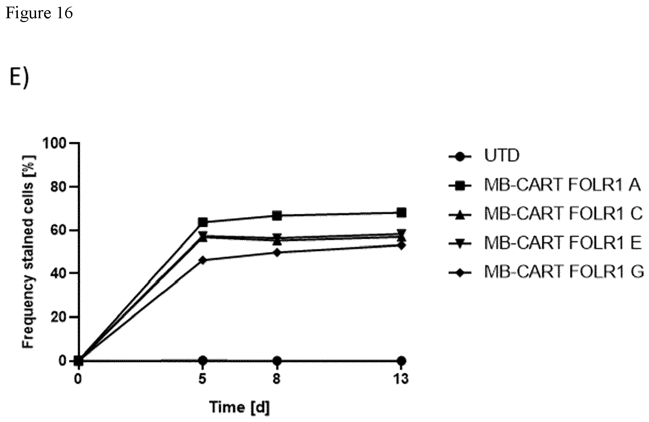

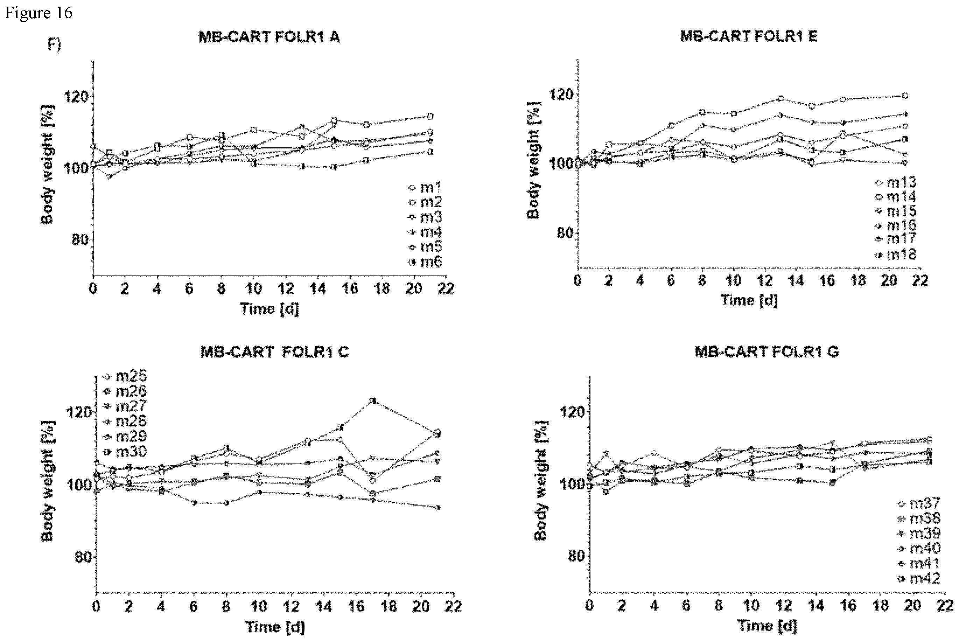

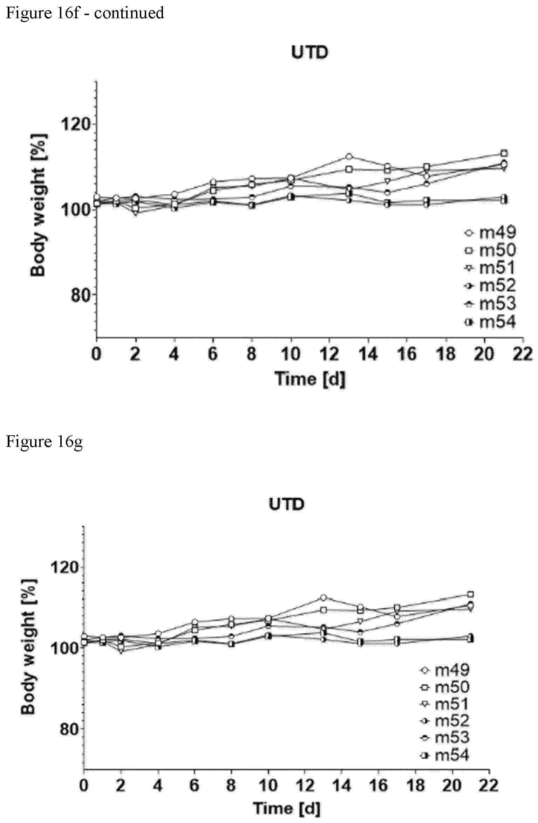

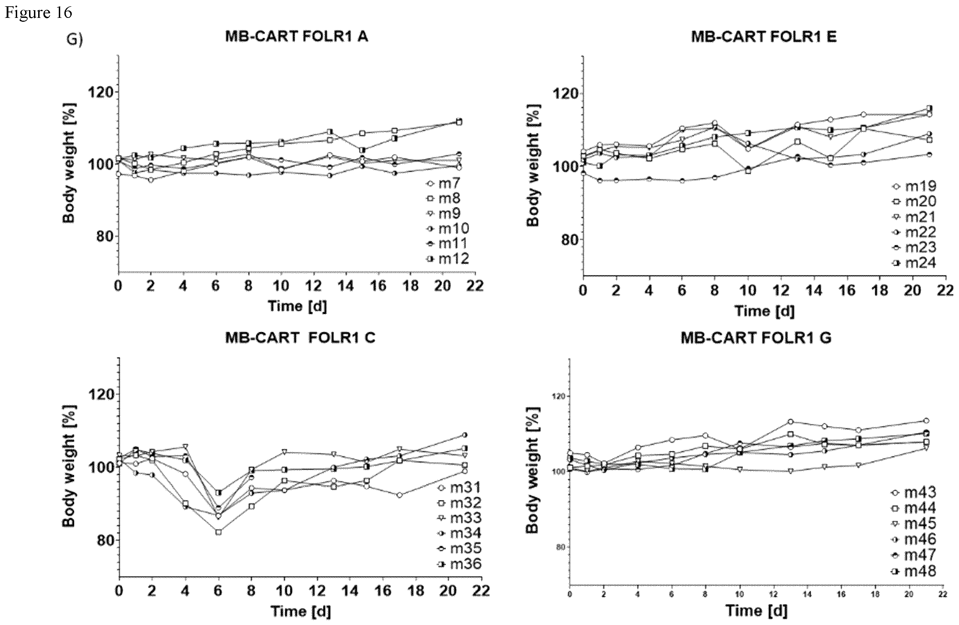

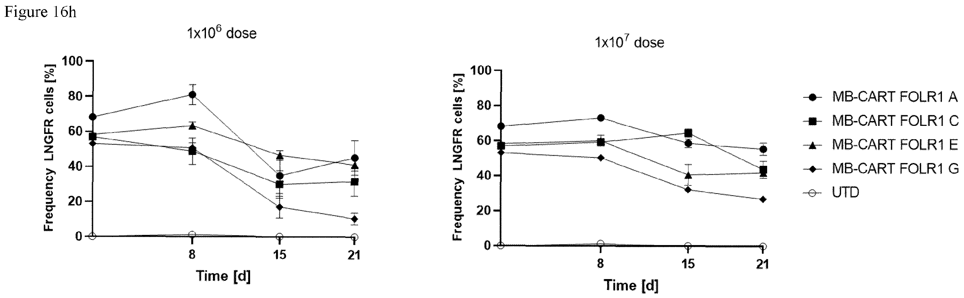

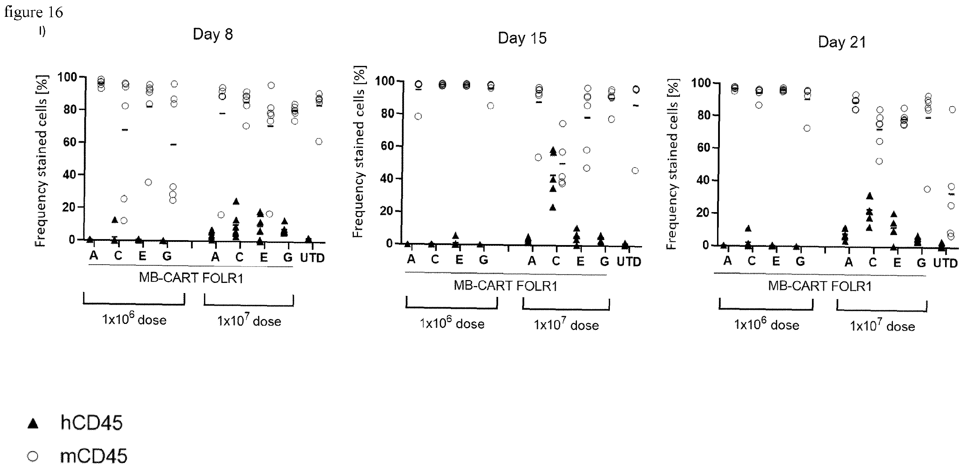

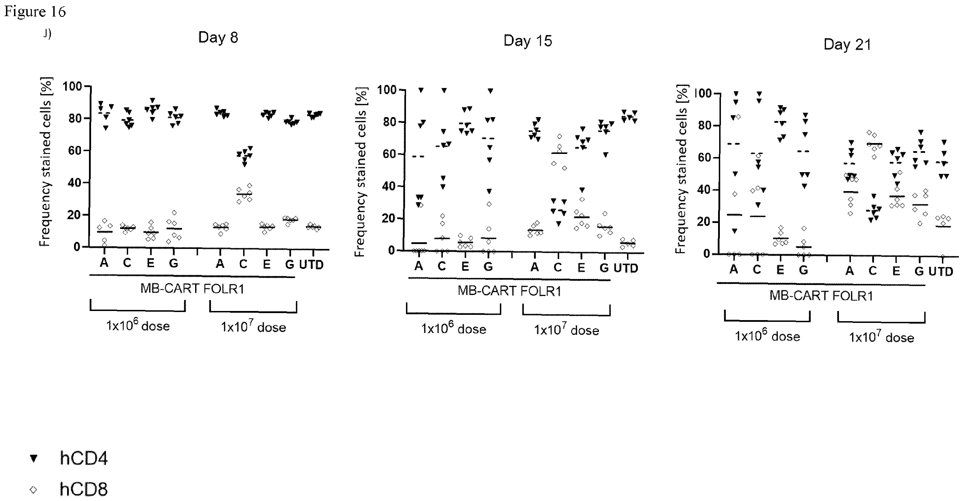

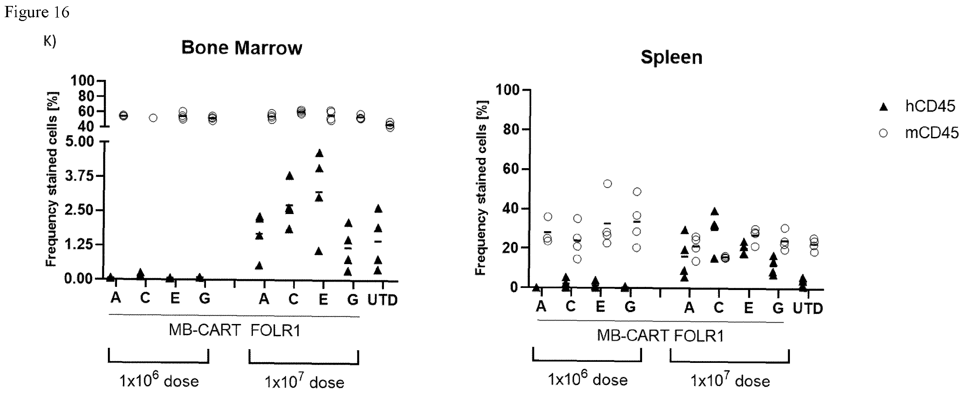

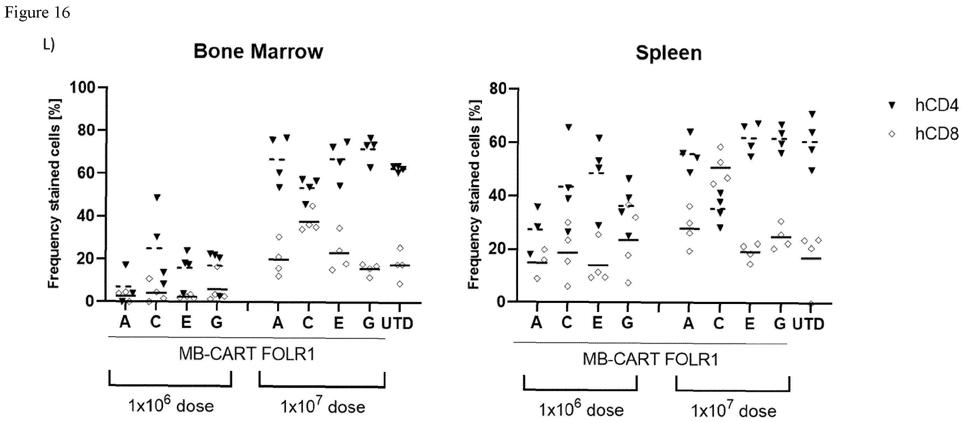

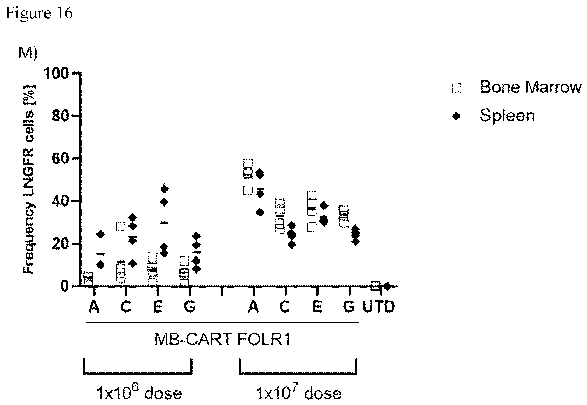

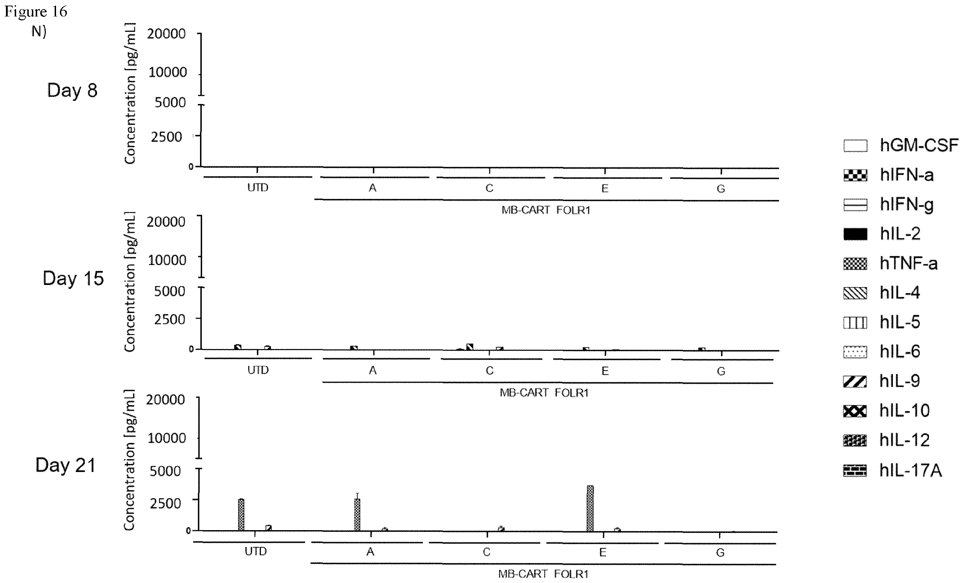

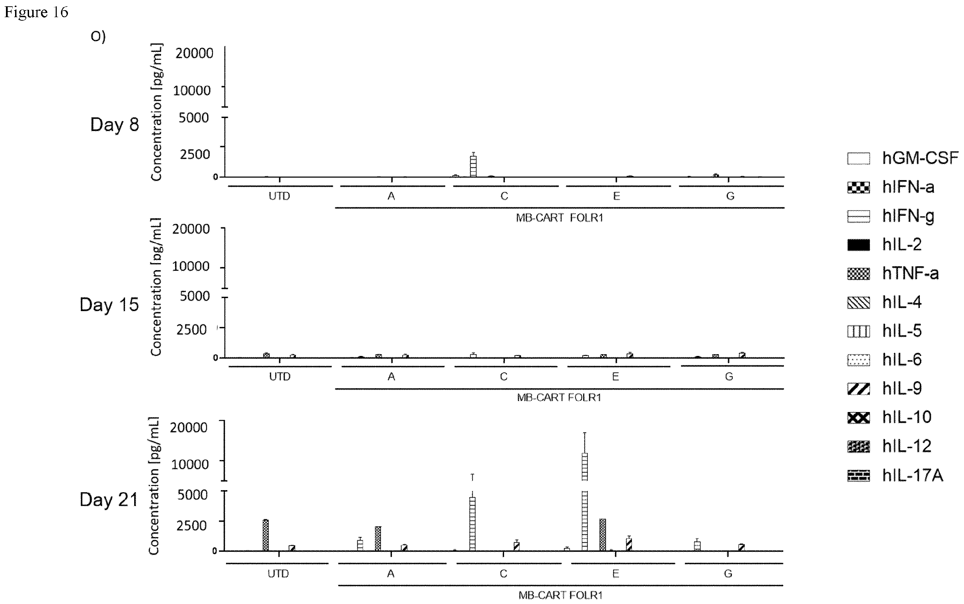

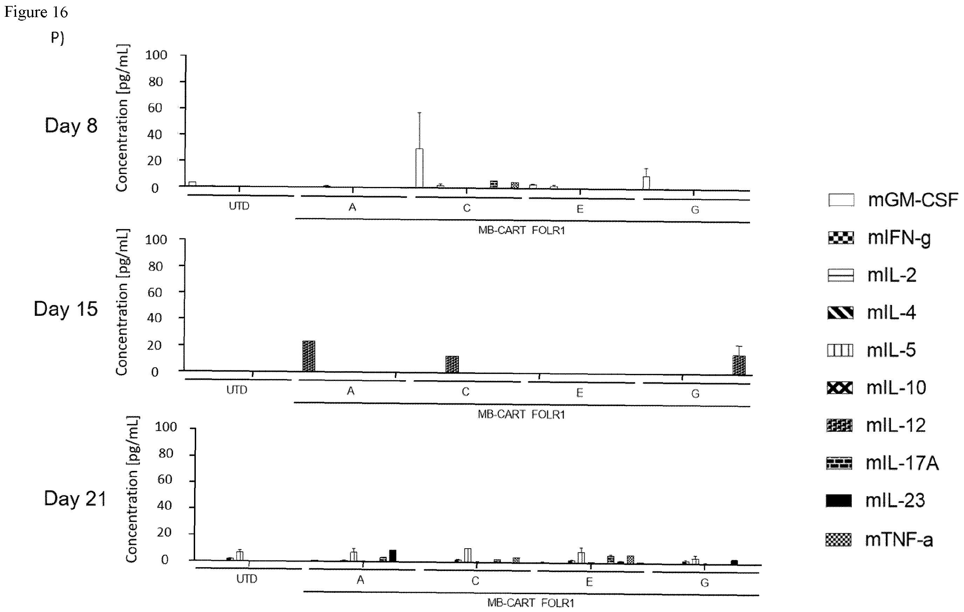

Figure 16 Most anti-FOLR1 CAR T cells are well tolerated in tumor-free NSG mice. Tolerability of MB-CART FOLR1 candidates was assessed in NSG mice intravenously applying different doses of CAR T cells produced in the CliniMACS Prodigy^{®}.
   A) Scheme of study design including time points of analysis.
   B) White blood cell composition of leukapheresis (Ori), isolated pan T cells (Pos) and different MB-CART FOLR1 candidates was analyzed by flow cytometry over the course of CAR T cell generation. Cell populations were defined as: T cells: CD3+, CD56-, CD16-; NKT cells: CD3+, CD56+, CD16+; Monocytes: CD3-, CD14+; B cells: CD3-, CD19+; Neutrophils: CD3-, CD14-, CD19-, CD56+, CD16+; Eosinophils: CD3-, CD14-, CD19-, CD56-, CD16- .
   C) Viability of CAR T cells was monitored over time by flow cytometry during the CliniMACS Prodigy ^{®} TCT process and analyzed by flow cytometry (7-AAD staining).
   D) Human CD4 and CD8 T cell composition of final CAR T cells at the end of the CliniMACS Prodigy^{®} TCT process was analyzed by flow cytometry.
   E) Transduction efficiency of CAR T cells was monitored over time by flow cytometry during the CliniMACS Prodigy ^{®} TCT process and analyzed by flow cytometry (anti-LNGFR staining).
   F) NSG mice were intravenously injected with 1×10⁶ anti-FOLR1 CAR T cells (MB-CART-FOLR1 A, C, E, G) or 1×10⁷untransduced T cells (UTD) produced in the CliniMACS Prodigy^{®} to assess tolerability. Body weight was monitored over the 21 days post injection. Each line corresponds to a single mouse.
   G) NSG mice were intravenously injected with 1×10⁷anti-FOLR1 CAR T cells ((MB-CART-FOLR1 A, C, E, G) or 1×10⁷untransduced T cells (UTD) produced in CliniMACS Prodigy^{®} to assess tolerability. Weight was monitored over the 21 days post injection . Each line corresponds to a single mouse.
   H) CAR expression was analyzed over time in human T cells from peripheral blood samples of mice treated with the indicated CAR candidates at doses of 1×10⁶CAR T cells (left) or 1×10⁷CAR T cells (right), respectively. CAR T cells were analyzed by flow cytometry for LNGFR (co-expressed with the CAR) expression, before injection (d0) and in blood samples collected at day 8, 15, and 21 post T cell injection. Data is shown as mean ± SEM of values from 6 mice per group (n=6).
   I) Mouse and human leucocytes composition in peripheral blood of CAR T cell treated mice was analyzed. Anti-FOLR1 CAR T cells or untransduced T cells (UTD) produced in CliniMACS Prodigy^{®} were injected at a 1×10⁶or 1×10⁷CAR T cells dose. Murine blood samples were analyzed by flow cytometry for murine or human CD45 expression, respectively, at day 8, 15, and 21 post T cell injection. Each data point represents an individual mouse and horizontal lines represent the mean of the respective group .
   J) Human T cell composition (CD4, CD8) in peripheral blood of CAR T cell treated mice was assessed. Anti-FOLR1 CAR T cells or untransduced T cells (UTD) produced in CliniMACS Prodigy^{®} were injected at a 1×10⁶or 1×10⁷CAR T cells dose. Human T cells were analyzed by flow cytometry for human CD4 and CD8 expression, respectively, in blood samples collected at day 8, 15 and 21 post T cell injection. Each data point represents an individual mouse and horizontal lines represent the mean of the respective group.
   K) Mouse and human leucocytes composition in mouse bone marrow (left) and spleen (right) was analyzed. On day 21 post T cell injection mice were sacrificed and the respective organs collected and dissociated. Single cell suspensions of the organs were analyzed ex vivo by flow cytometry for murine or human CD45 expression, respectively. Each data point represents an individual mouse and horizontal lines represent the mean of the respective group.
   L) Human T cell composition (CD4, CD8) in mouse bone marrow (left) and spleen (right) was assessed.. On day 21 post T cell injection mice were sacrificed and the respective organs collected and dissociated. Single cell suspensions of the organs were analyzed ex vivo by flow cytometry for human CD4 and CD8 expression, respectively. Each data point represents an individual mouse and horizontal lines represent the mean of the respective group
   M) CAR expression was analyzed in human T cells from mouse bone marrows and spleens. CAR T cells were analyzed by flow cytometry for LNGFR (co-expressed with the CAR) expression. On day 21 post T cell injection mice were sacrificed and the respective organs collected and dissociated. Single cell suspensions of the organs were analyzed ex vivo by flow cytometry for LNGFR expression. Each data point represents a single mouse and horizontal lines represent the mean of the respective group.
   N) Secretion of human cytokines was analyzed in peripheral blood samples of CAR T cell treated mice over time. Anti-FOLR1 CAR T cells or untransduced T cells (UTD) produced in CliniMACS Prodigy^{®} were injected at a 1×10⁶ CAR T cells dose. Plasma was isolated from blood samples collected at day 8, 15, and 21 post T cell injection and cytokine levels were subsequently determined with the human MACSplex Cytokine 12 Kit. Data is shown as mean ± SEM of values from six mice per group (n=6).
   O) Secretion of human cytokines was analyzed in peripheral blood samples of CAR T cell treated mice over time. Anti-FOLR1 CAR T cells or untransduced T cells (UTD) produced in CliniMACS Prodigy^{®} were injected at a 1×10⁷CAR T cells dose. Plasma was isolated from blood samples collected at day 8, 15, and 21 post T cell injection and cytokine levels were subsequently determined with the human MACSplex Cytokine 12 Kit. Data is shown as mean ± SEM of values from six mice per group (n=6).
   P) Secretion of murine cytokines was analyzed in peripheral blood samples over time. Anti-FOLR1 CAR T cells or untransduced T cells (UTD) were injected at a 1×10⁷ CAR T cells dose. Plasma was isolated from blood samples collected at day 8, 15, and 21 post T cell injection and cytokine levels were subsequently determined with the human MACSplex Cytokine 12 Kit. Data is shown as mean ± SEM of values from six mice per group (n=6).

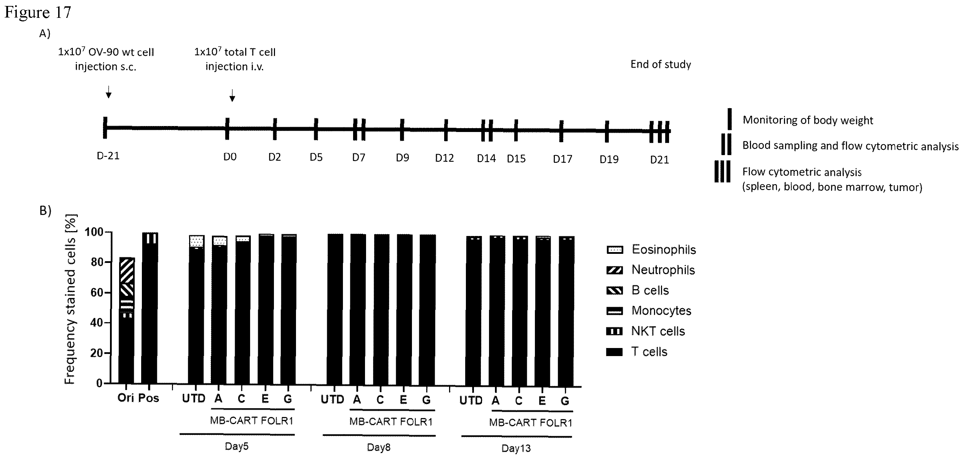

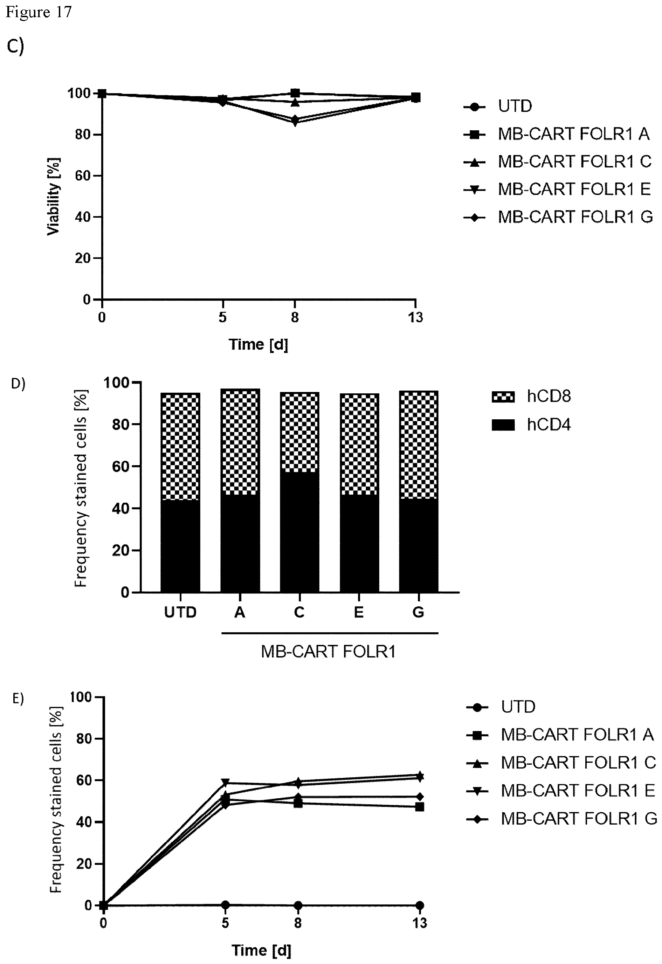

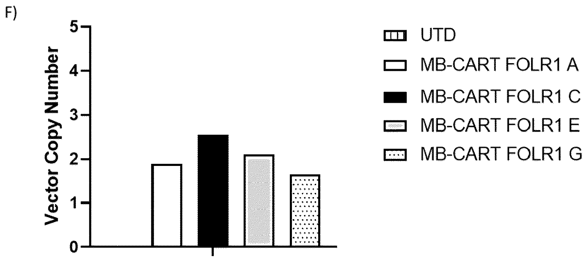

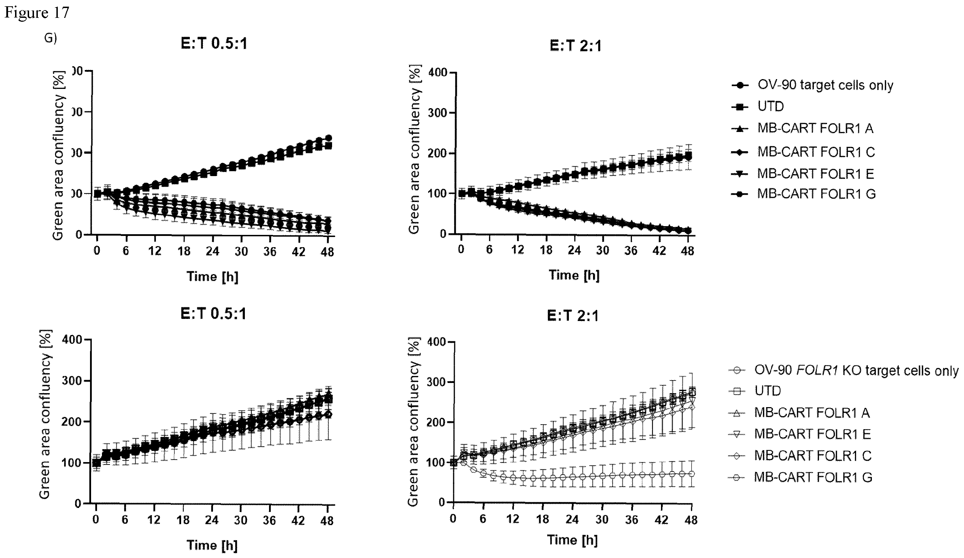

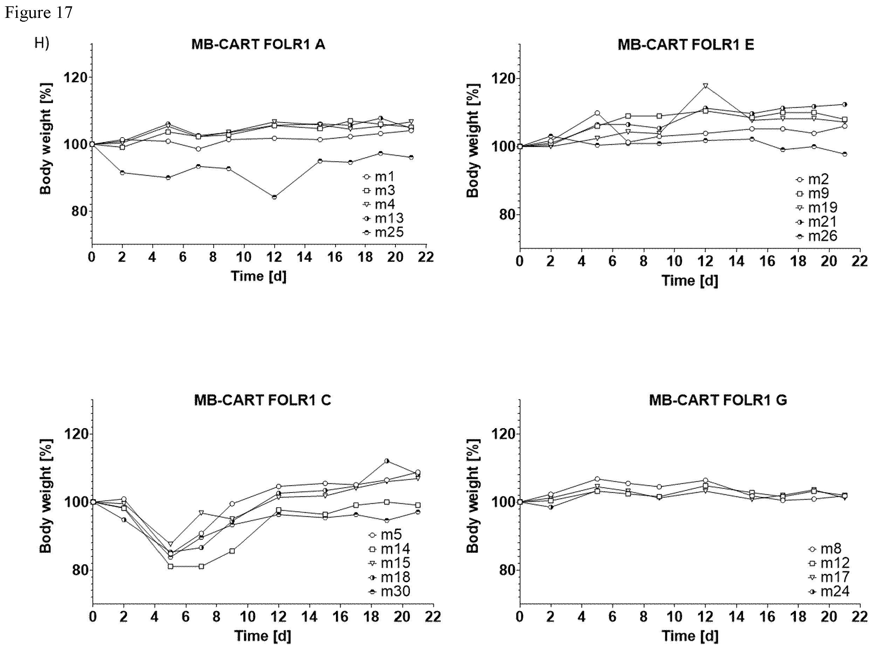

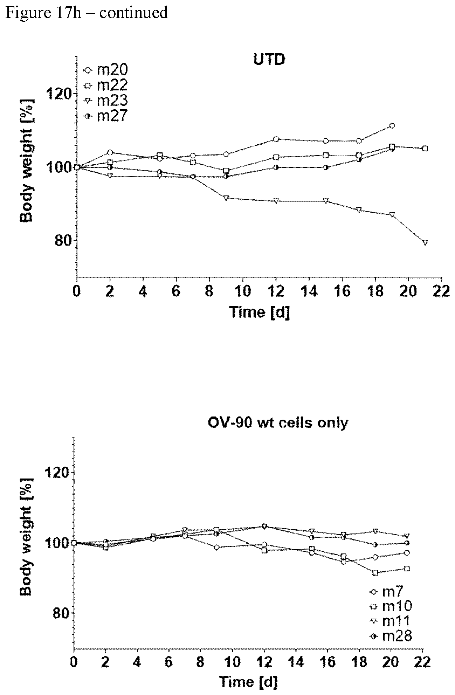

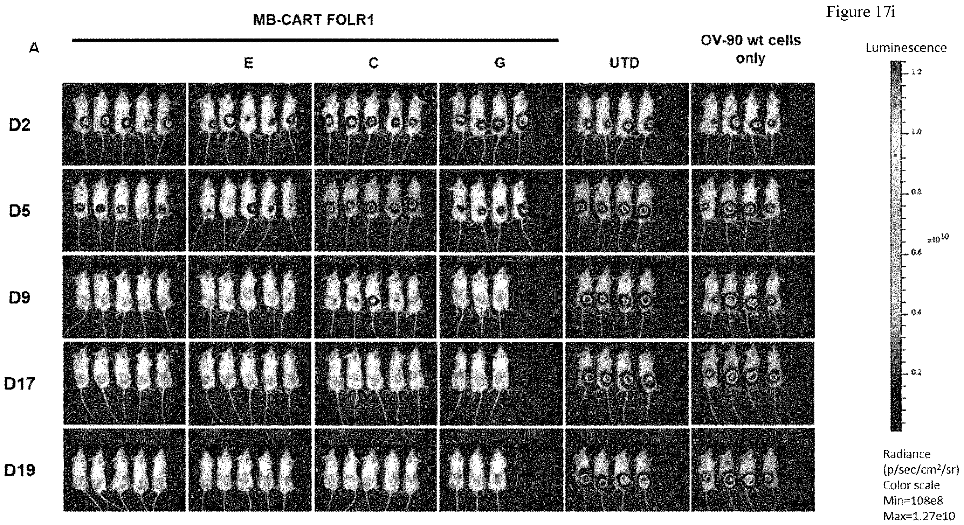

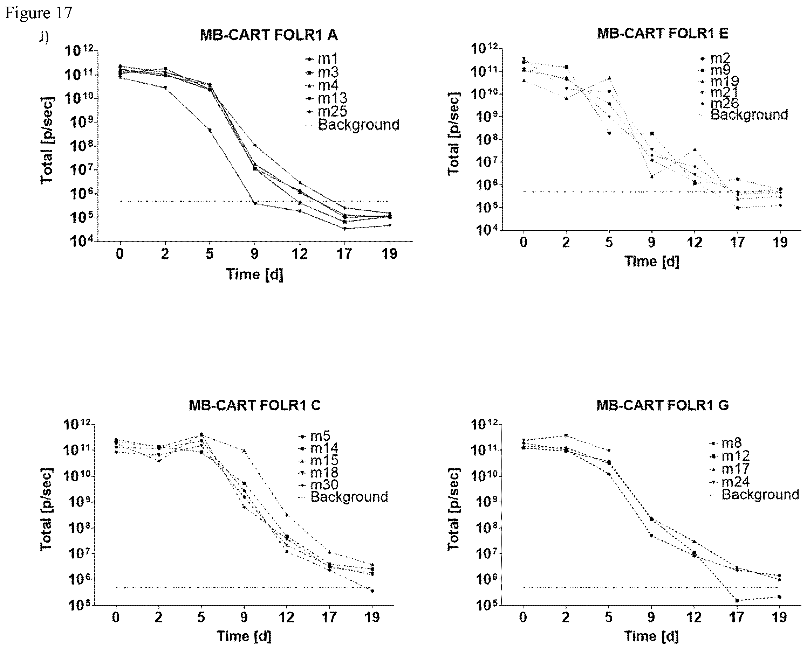

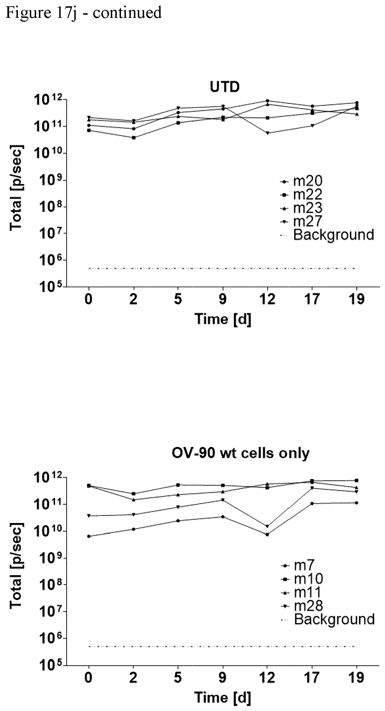

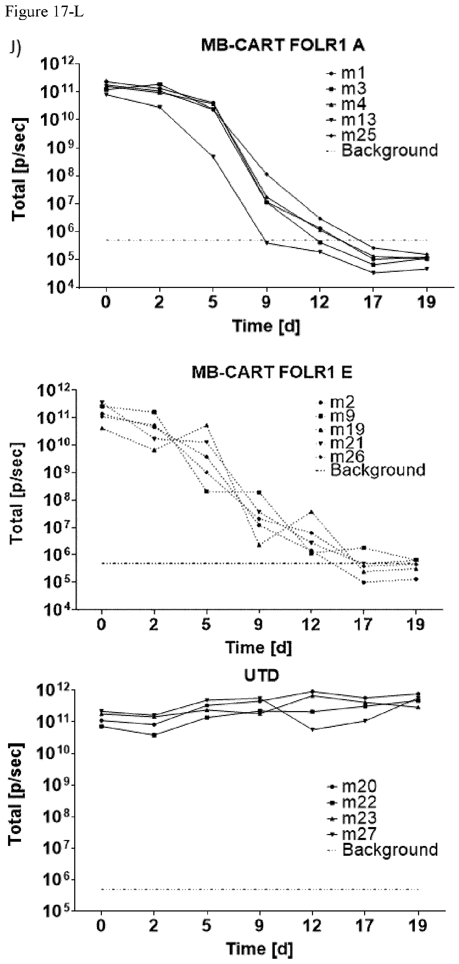

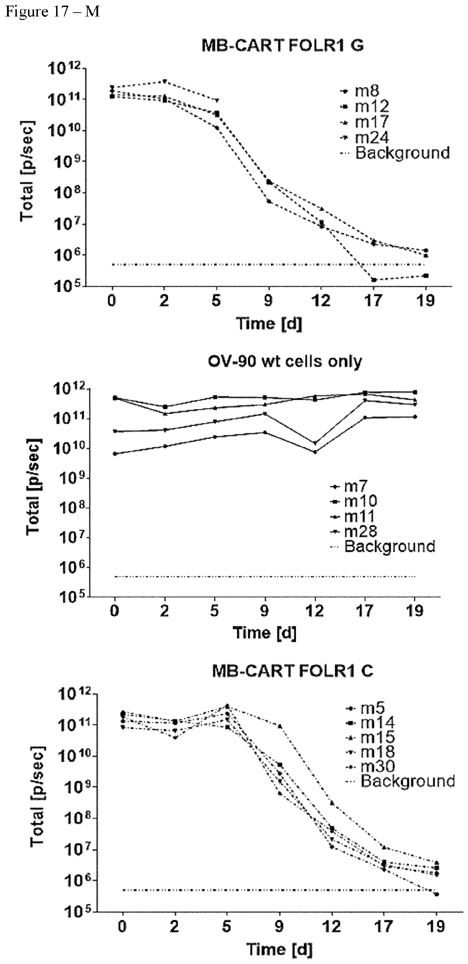

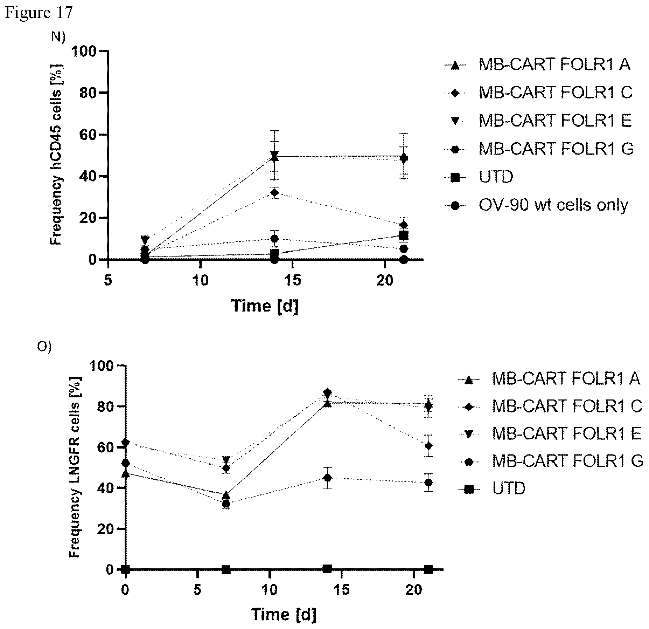

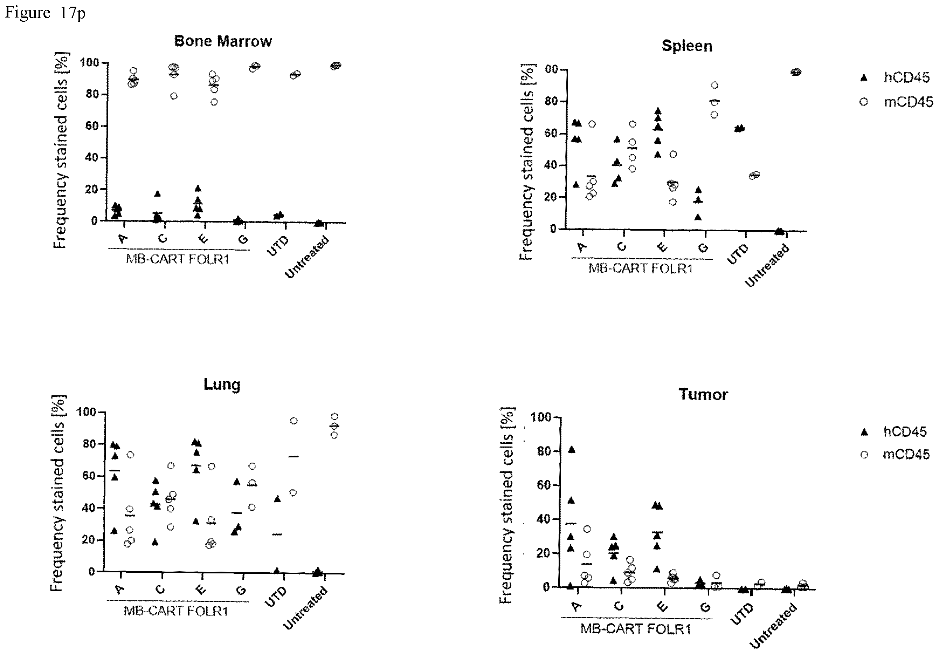

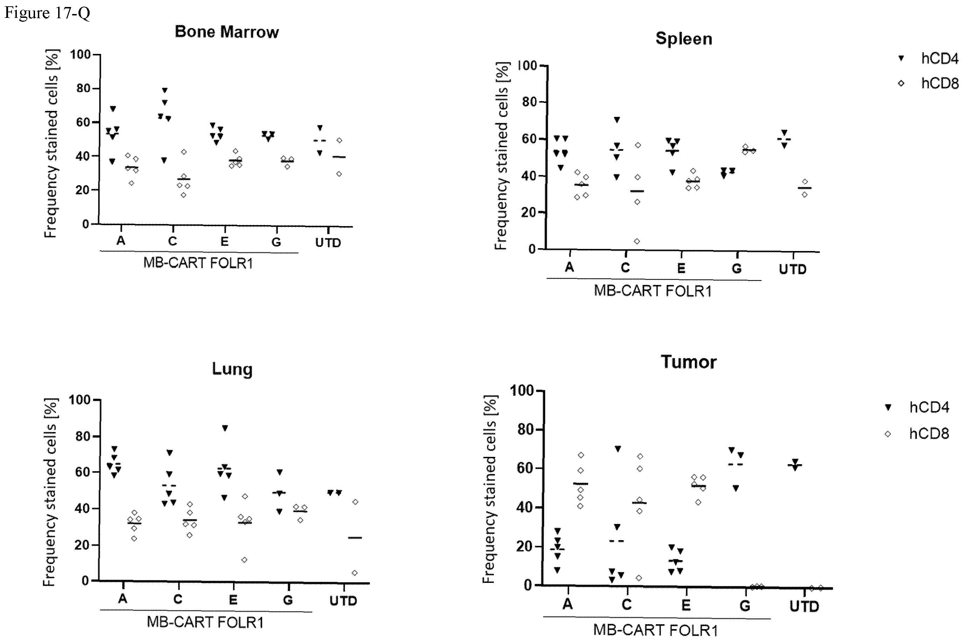

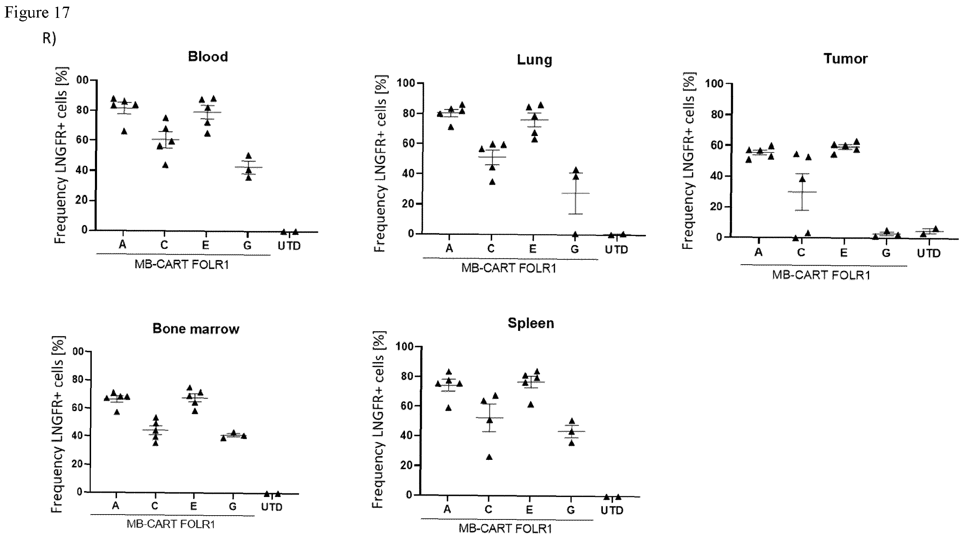

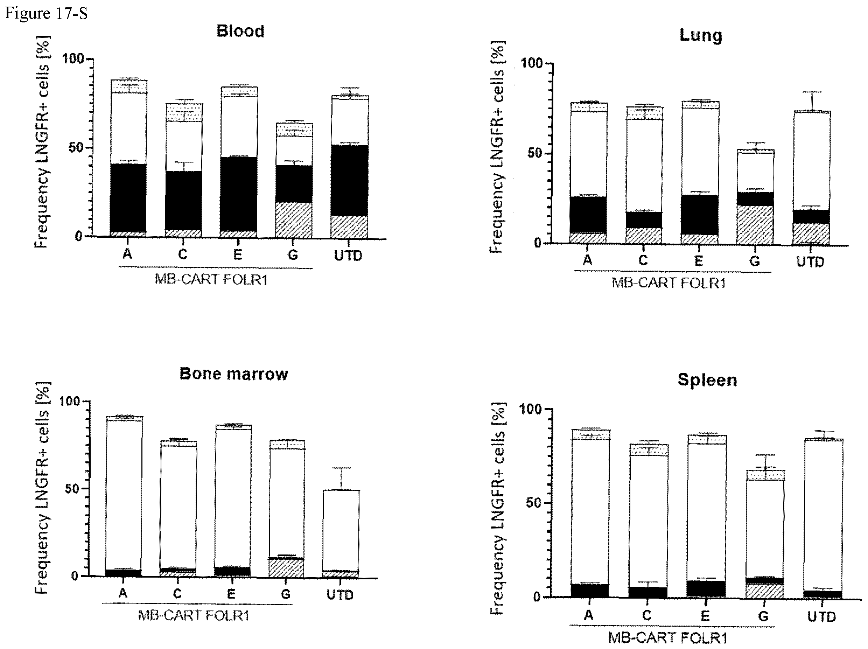

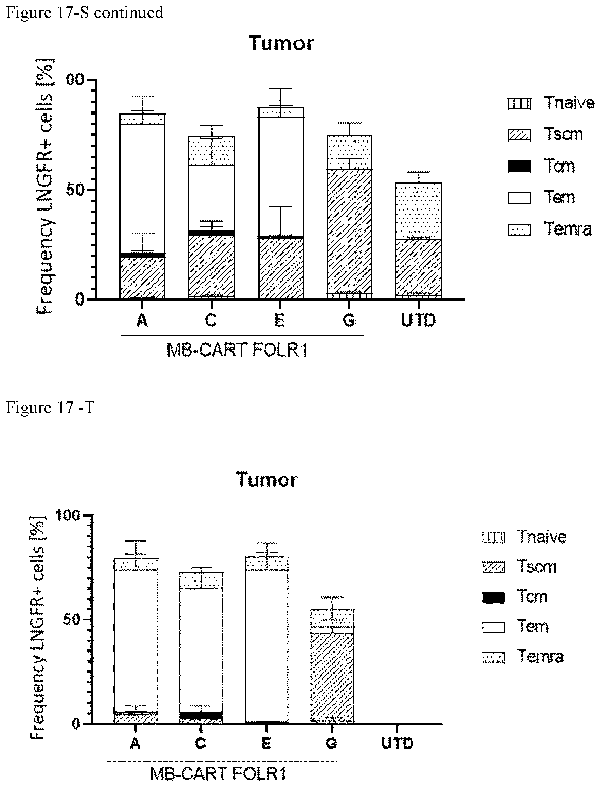

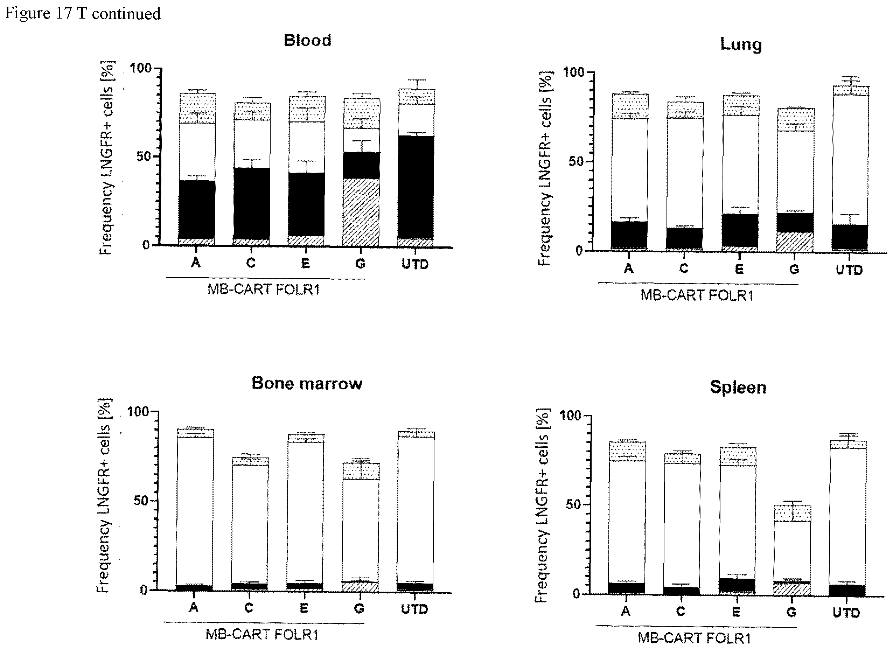

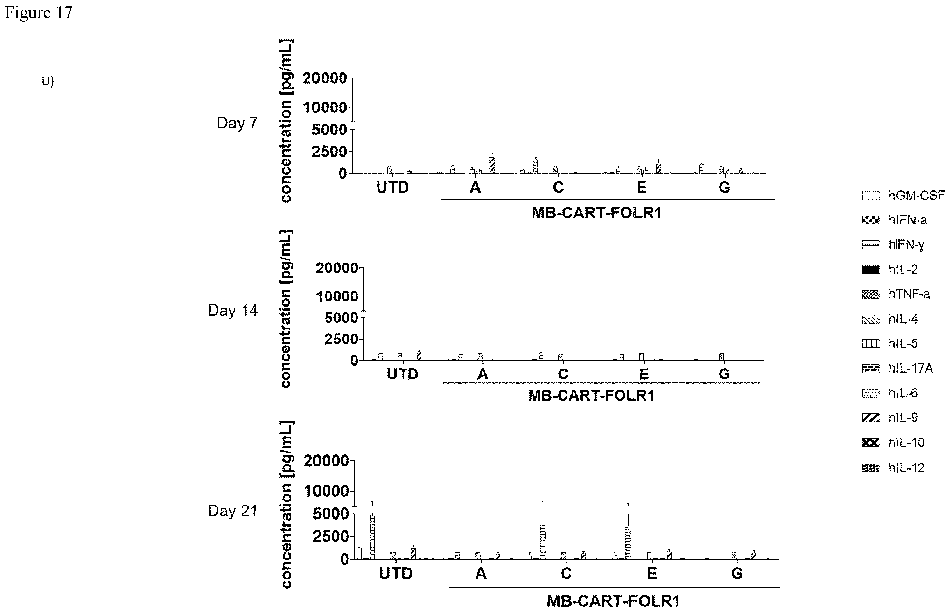

Figure 17: Anti-FOLR1 CAR T candidates efficiently eradicate OV-90 wt tumors in NSG mice. NSG mice were injected with OV-90 cells subcutaneously and after 21 days intravenously injected with anti-FOLR1 CAR T cells (1×10⁷ total T cells; MB-CART-FOLR1 A, C, E, G) or untransduced T cells (UTD) produced in CliniMACS Prodigy^{®} to assess anti-tumor function.
   A) Scheme of study design including time points of analysis.
   B) White blood cell composition of leukapheresis (Ori), isolated pan T cells (Pos) and different MB-CART FOLR1 candidates was analyzed by flow cytometry over the course of CAR T cell generation. Cell populations were defined as: T cells: CD3+, CD56-, CD16-; NKT cells: CD3+, CD56+, CD16+; Monocytes: CD3-, CD14+; B cells: CD3-, CD19+; Neutrophils: CD3-, CD14-, CD19-, CD56+, CD16+; Eosinophils: CD3-, CD14-, CD19-, CD56-, CD16- .
   C) Viability of CAR T cells was monitored over time by flow cytometry during the CliniMACS Prodigy^{®} TCT process and analyzed by flow cytometry (7AAD staining).
   D) Human CD4 and CD8 T cell composition of final CAR T cells at the end of the CliniMACS Prodigy^{®} TCT process was analyzed by flow cytometry.
   E) Transduction efficiency of CAR T cells was monitored over time by flow cytometry during the CliniMACS Prodigy ^{®} TCT process and analyzed by flow cytometry (anti-LNGFR staining)
   F) Vector copy number of the final CAR T cells was determined by qPCR at the end of the CliniMACS Prodigy^{®} TCT process.
   G) Functionality and specificity of CAR T cell products were assessed by *in vitro* co-culture experiments with OV-90 wt (upper panels) and OV-90 FOLR1 KO cells (lower panels), respectively, for 48 hours at different effector cell : target cell ratios of 0.5:1 (left panels) or 2:1 (right panels). Data points of each group are normalized against the baseline (defined as 100% confluency at the first timepoint (T=0 hours)) and each datapoint represents the respective mean +/- SEM (n=3).
   H) Body weight of mice was monitored over 21 days post CAR T cell injection. Each line corresponds to an individual mouse.
   I) Representative bioluminescence images of individual OV-90 xenograft tumor bearing NSG mice post CAR T cell injection. Color scale for all images, min = 1.08×10⁸, max = 1.27×10¹⁰.
   J) Quantification of bioluminescence as total flux in photons per seconds (p/s) of individual mice in the different experimental groups after CAR T cell injection over 21 days. Each line represents an individual mouse.
   K) Quantification of bioluminescence as total flux in photons per seconds (p/s) of the different experimental groups after CAR T cell injection over 21 days. Data points represent mean ± SEM.
   L) Mouse and human leucocytes composition in peripheral blood of CAR T cell treated mice was analyzed. Murine blood samples were analyzed by flow cytometry for murine or human CD45 expression, respectively, at day 7, 14, and 21 post T cell injection. Each data point represents an individual mouse and horizontal lines represent the mean of the respective group.
   M) Human T cell composition (CD4, CD8) in peripheral blood of CAR T cell treated mice was assessed. Human T cells were analyzed by flow cytometry for human CD4 and CD8 expression, respectively, in blood samples collected at day 7, 14 and 21 post T cell injection. Each data point represents an individual mouse and horizontal lines represent the mean of the respective group.
   N) Human leucocytes in peripheral blood of CAR T cell treated mice were analyzed by flow cytometry over time. Human CD45 expression in blood samples collected at day 7, 14, and 21 post T cell injection was measured. Data is shown as mean ± SEM.
   O) CAR expression was analyzed over time in human T cells from peripheral blood samples of mice treated with the indicated CAR candidates. CAR T cells were analyzed by flow cytometry for LNGFR (co-expressed with the CAR) expression, in blood samples collected at day 7, 14, and 21 post T cell injection. Data is shown as mean ± SEM.
   P) Mouse and human leucocytes composition in mouse bone marrow, spleen, lung, and tumor was analyzed. On day 21 post T cell injection mice were sacrificed and the respective organs collected and dissociated. Single cell suspensions of the organs were analyzed ex vivo by flow cytometry for murine or human CD45 expression, respectively. Each data point represents an individual mouse and horizontal lines represent the mean of the respective group.
   Q) Human T cell composition (CD4, CD8) in mouse bone marrow, spleen, lung, and tumor was assessed. On day 21 post T cell injection mice were sacrificed and the respective organs collected and dissociated. Single cell suspensions of the organs were analyzed ex vivo by flow cytometry for human CD4 and CD8 expression, respectively. Each data point represents an individual mouse and horizontal lines represent the mean of the respective group.
   R) CAR expression was analyzed in human T cells from murine blood, lung, bone marrow, spleen and tumor. On day 21 post T cell injection mice were sacrificed and the respective organs collected and dissociated. Single cell suspensions of the organs as well as blood samples were analyzed ex vivo by flow cytometry for LNGFR expression (co-expressed with the CAR). Each data point represents an individual mouse and horizontal lines represent the mean of the respective group together with error bars of ± SEM.
   S) Human CD4 T cells from mouse blood, bone marrow, spleen, lung, and tumor were phenotyped. On day 21 post T cell injection mice were sacrificed and the respective organs collected and dissociated. Single cell suspensions of the organs were analyzed ex vivo. T cells were assessed for phenotype marker expression by flow cytometry. T cell subtypes were defined as following: Tscm: CD45RO-, CD197+, CD62L+, CD95+; Tnaive: CD8+ or CD4+, CD45RO-, CD197+, CD62L+, CD95-; Temra: CD45RO-, CD197-, CD62L-, CD95+; Tem: CD45RO+, CD62L-, CD95+; and Tcm: CD45RO+, CD62L+, CD95+. Each bar represents mean +/- SEM of the respective group.
   T) Human CD8 T cells from mouse blood, bone marrow, spleen, lung, and tumor were phenotyped. On day 21 post T cell injection mice were sacrificed and the respective organs collected and dissociated. Single cell suspensions of the organs were analyzed ex vivo. T cells were assessed for phenotype marker expression by flow cytometry. T cell subtypes were defined as following: Tscm: CD45RO-, CD197+, CD62L+, CD95+; Tnaive: CD8+ or CD4+, CD45RO-, CD197+, CD62L+, CD95-; Temra: CD45RO-, CD197-, CD62L-, CD95+; Tem: CD45RO+, CD62L-, CD95+; and Tcm: CD45RO+, CD62L+, CD95+. Each bar represents mean +/- SEM of the respective group.
   U) Secretion of human cytokines was analyzed in peripheral blood samples over time. Plasma was isolated from blood samples collected at day 7, 14, and 21 post T cell injection and cytokine levels were subsequently determined with the human MACSplex Cytokine 12 Kit. Data is shown as mean ± SEM.

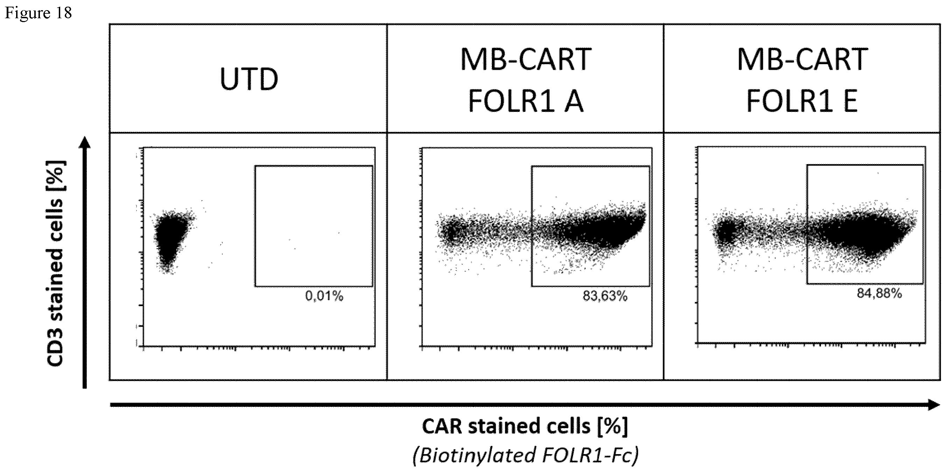

Figure 18 FOLR1-specific CAR candidates are expressed in primary human T cells from alternative lentiviral vector. Flow cytometric analysis of anti-FOLR1 CAR expression via biotinylated FOLR1-Fc fusion protein staining on CAR T cells produced with the CliniMACS Prodigy^{®} TCT process, using anti-biotin-PE secondary staining to determine anti-FOLR1 CAR expression. Flow cytometric data analyzed were selected among the CD3 positive population of viable cells.

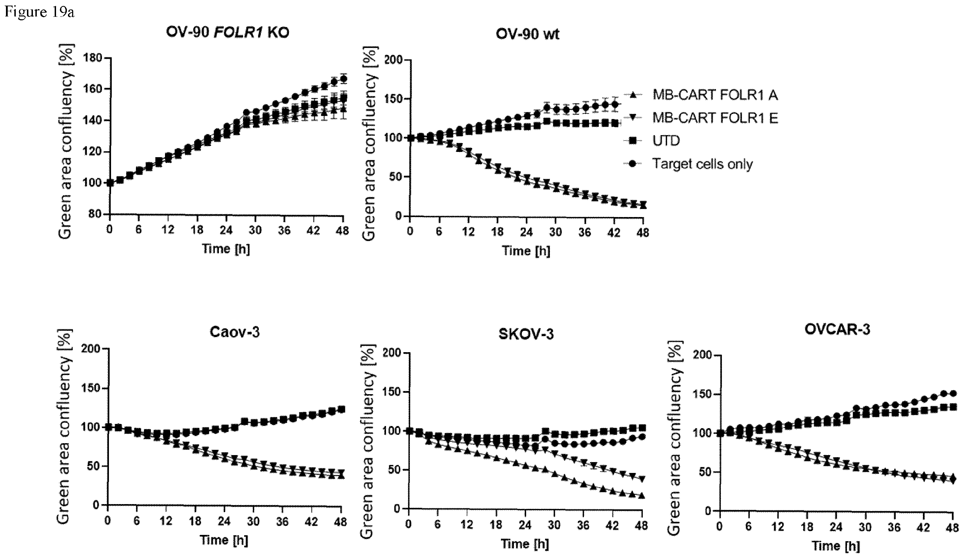

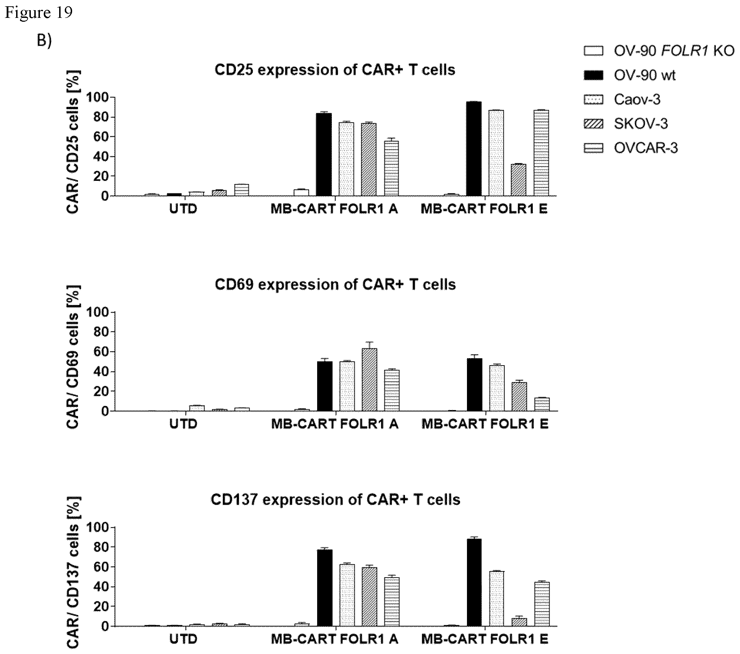

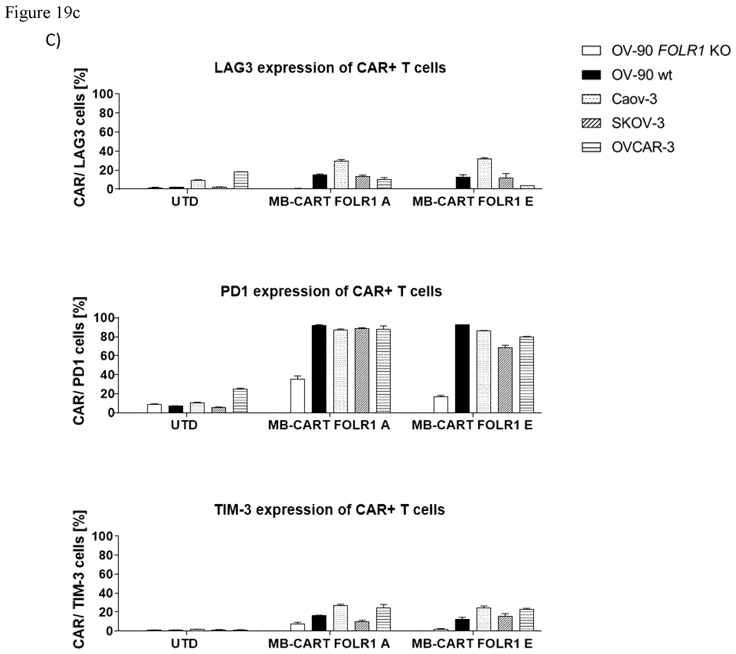

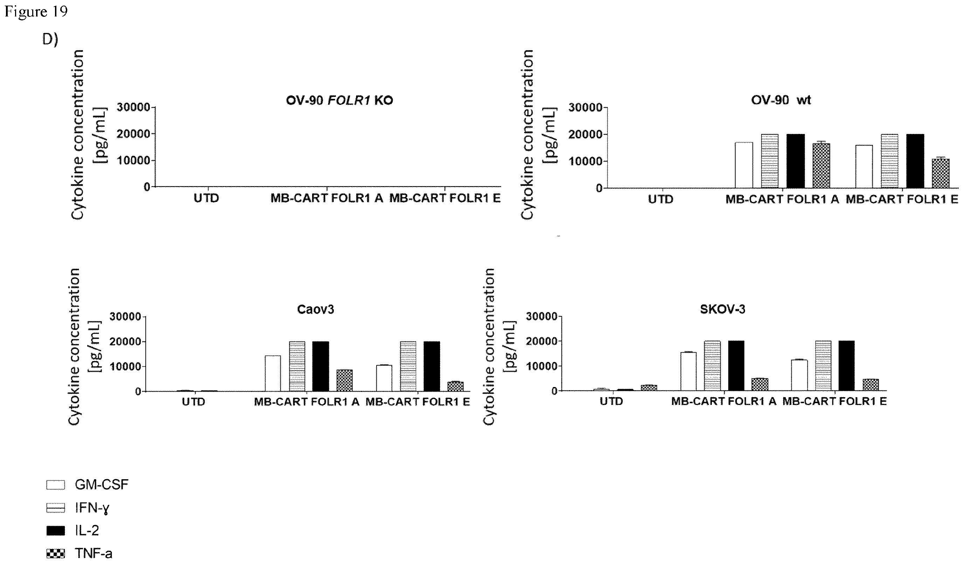

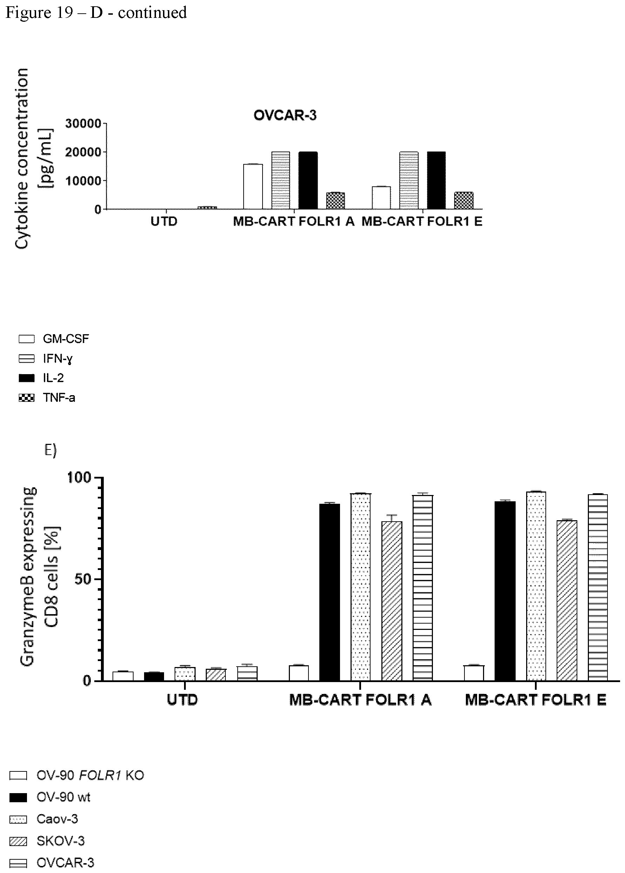

Figure 19 FOLR1-specific CAR T cells are functional *in vitro* in an antigen-dependent manner. MB-CART FOLR1 candidates A and E or untransduced T cells (UTD) produced with the CliniMACS Prodigy^{®} TCT process were co-cultured for 48 hours with ovarian cancer cell lines at an effector cell : target cell ratio of 2:1.
   A) Co-culture experiments of GFP-expressing target cell lines OV-90 FOLR1 KO, OV-90 wt, Caov-3, SKOV-3, and OVCAR-3 cells with MB-CART FOLR1 candidates A and E. Data points of each group are normalized to baseline (defined as 100% confluency at the first timepoint (T=0 hours)) and each datapoint represents mean +/- SEM (n=3).
      Anti-FOLR1 CAR T cells express various levels of activation and exhaustion markers. After 48 hours of co-culture with the different target cells, CAR T cells were assessed by flow cytometry for expression of
   B) activation markers CD25, CD69, and CD137 and
   C) exhaustion markers TIM-3, PD1, and LAG3.
      Each bar represents the mean +/- SEM (n=3).
   D) Anti-FOLR1 CAR T cells antigen-dependently secrete cytokines in co-culture with OV-90 FOLR1 KO, OV-90 wt, Caov-3, SKOV-3 and OVCAR-3 cells. Supernatants were collected after 24 hours of co-culture and cytokine concentration was subsequently determined with the human MACSplex Cytokine 12 Kit. Each bar represents the mean +/- SEM (n=3).
   E) Anti-FOLR1 CAR T cells antigen-dependently express Granzyme B in co-culture with OV-90 FOLR1 KO, OV-90 wt, Caov-3, SKOV-3 and OVCAR-3 cells. T cells were collected after 24h of co-culture, and protein secretion was blocked by incubation with Brefeldin A (1 µg/mL) overnight. Cells were subsequently fixed, stained for CD8 surface marker, permeabilized, and finally intracellular Granzyme B was detected via flow cytometry. Each bar represents the mean +/- SEM (n=3).

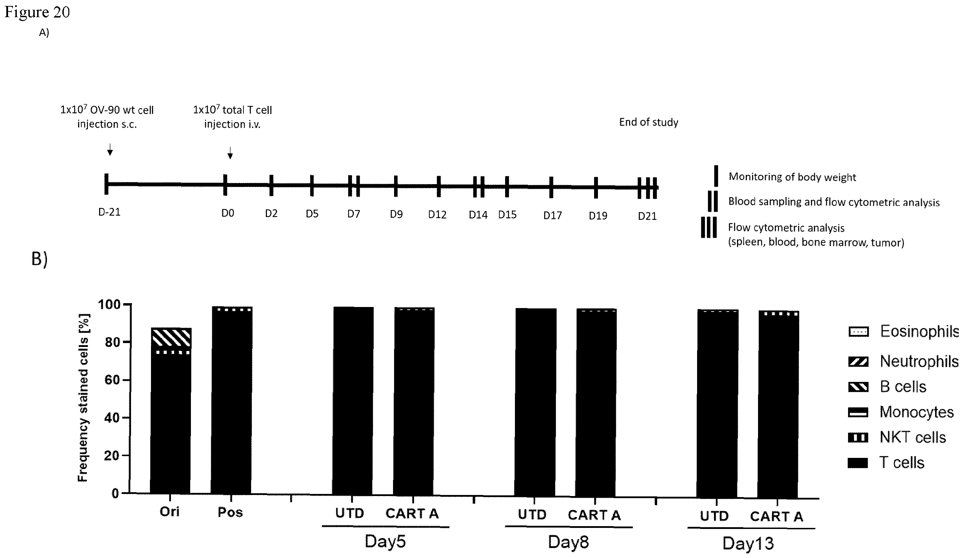

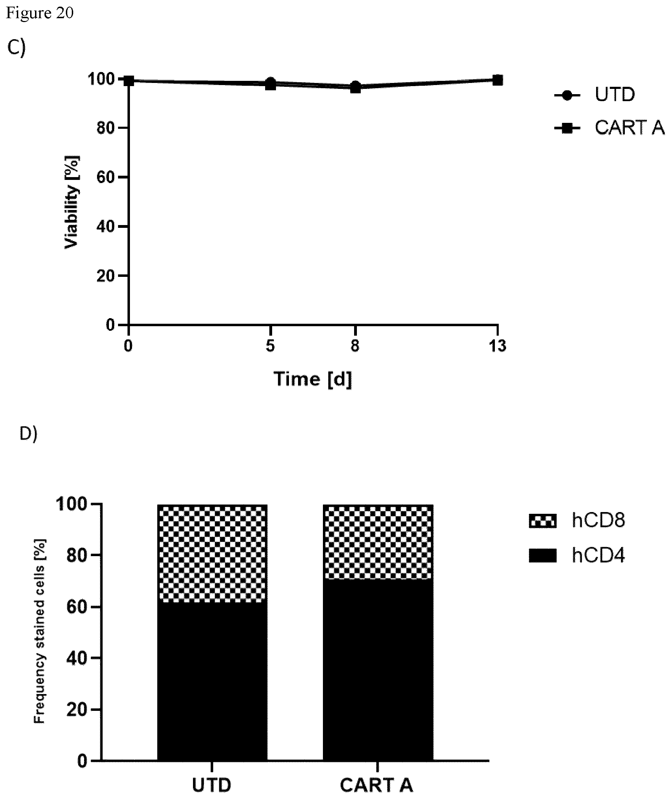

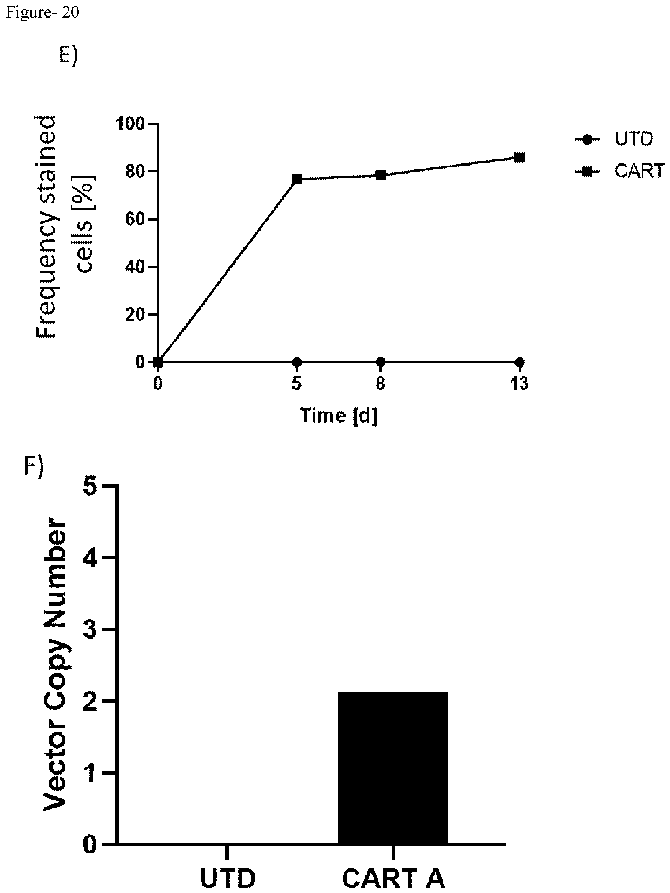

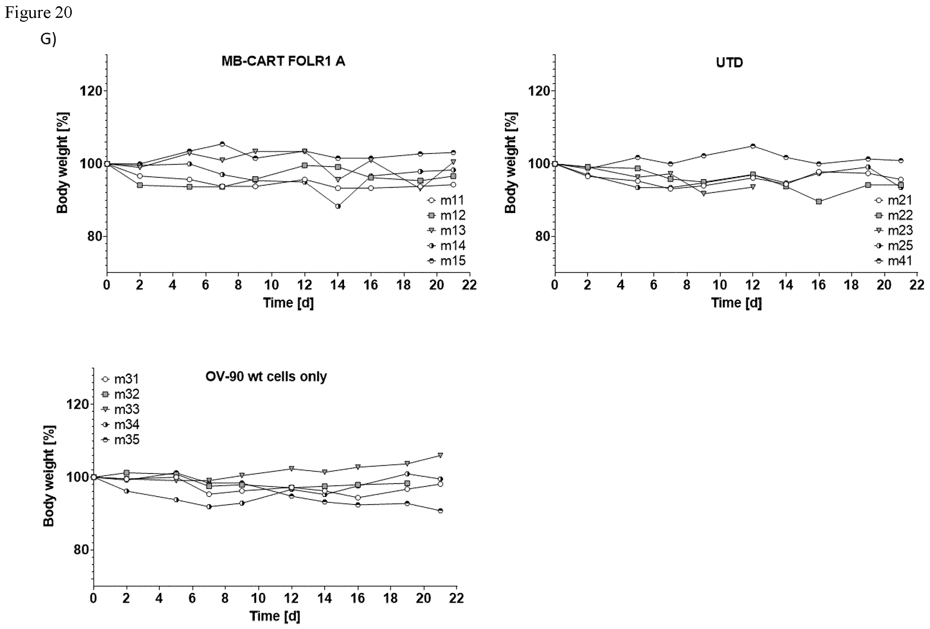

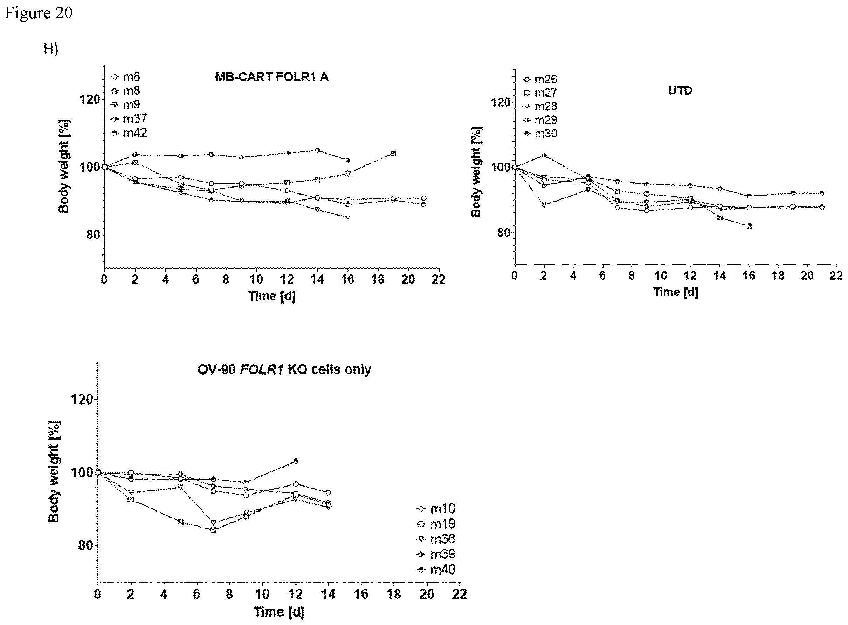

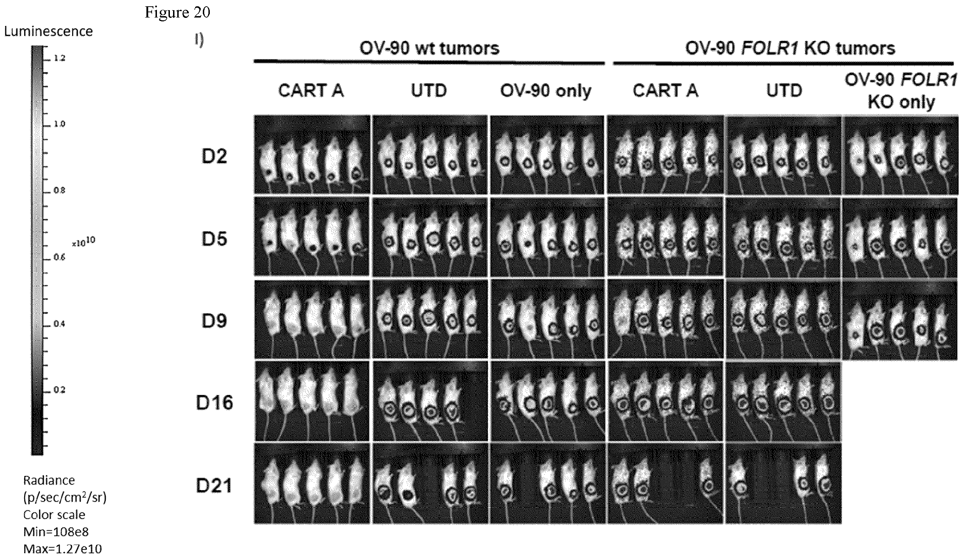

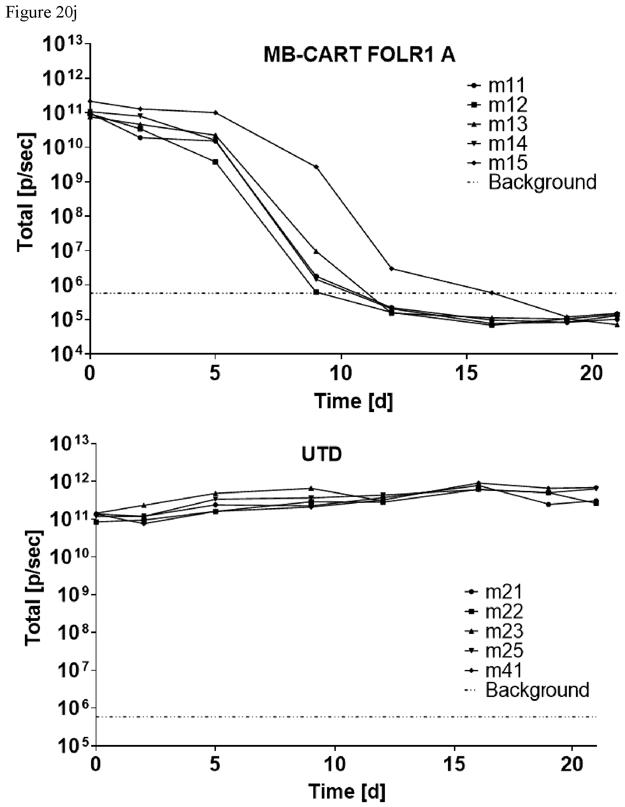

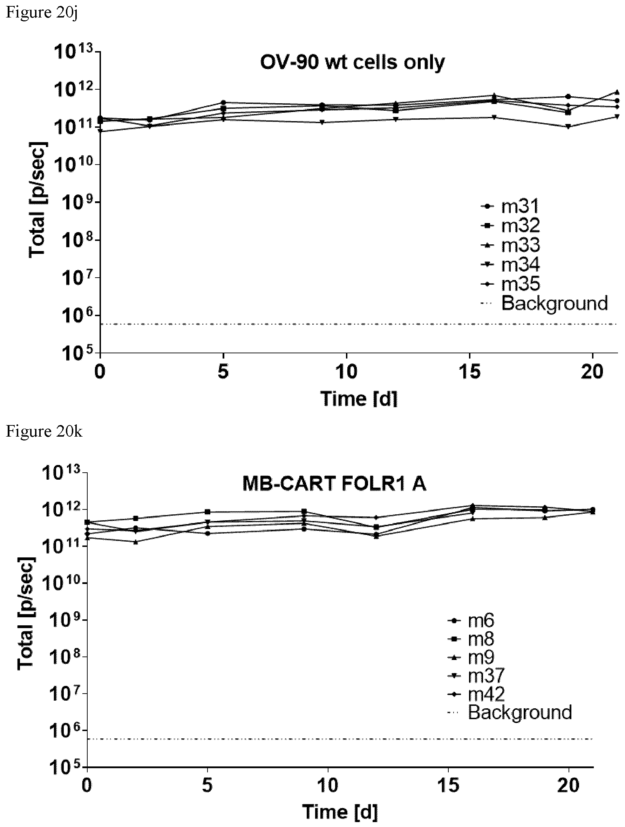

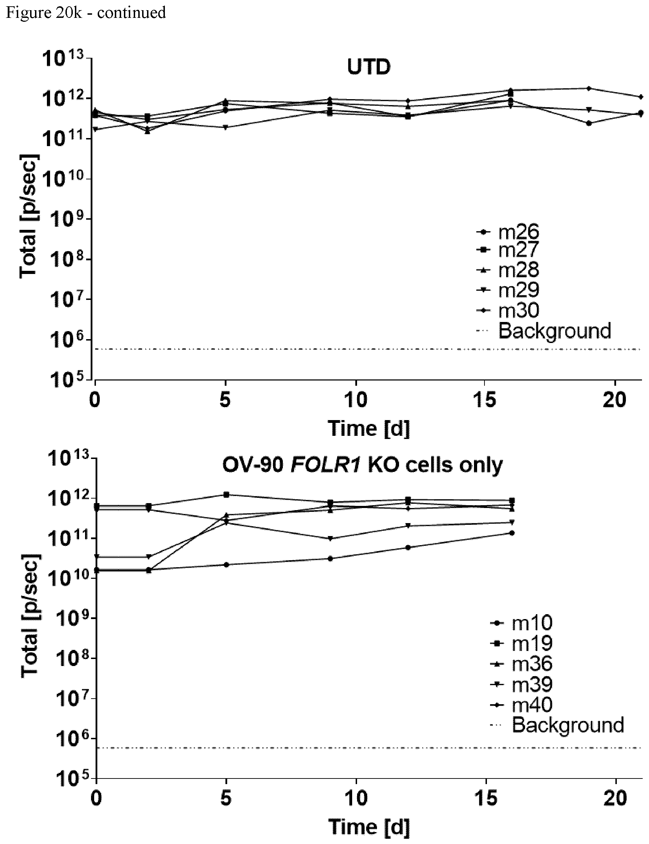

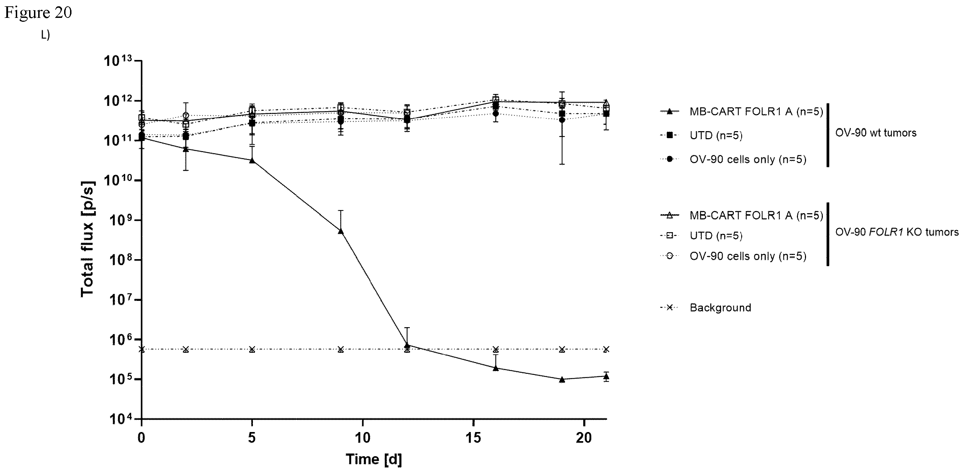

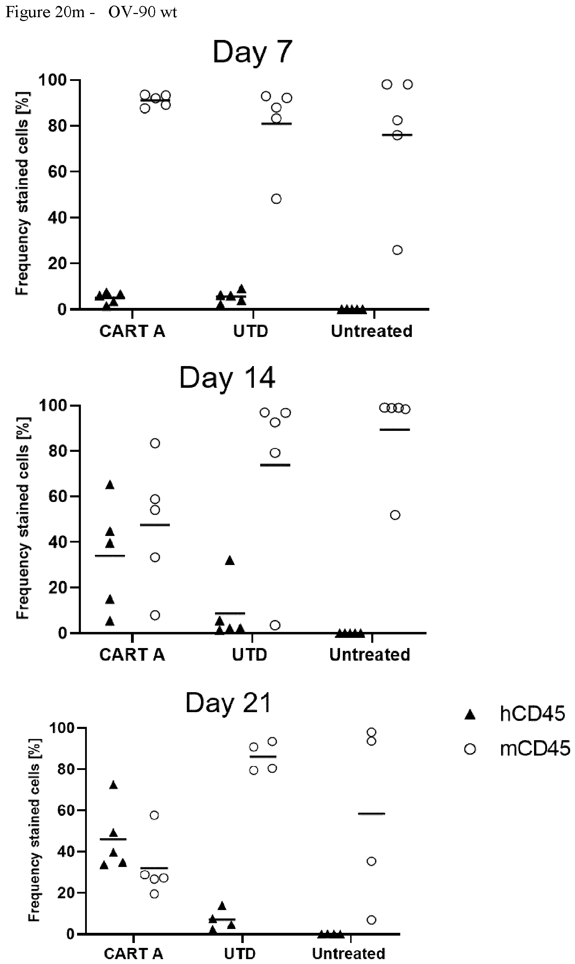

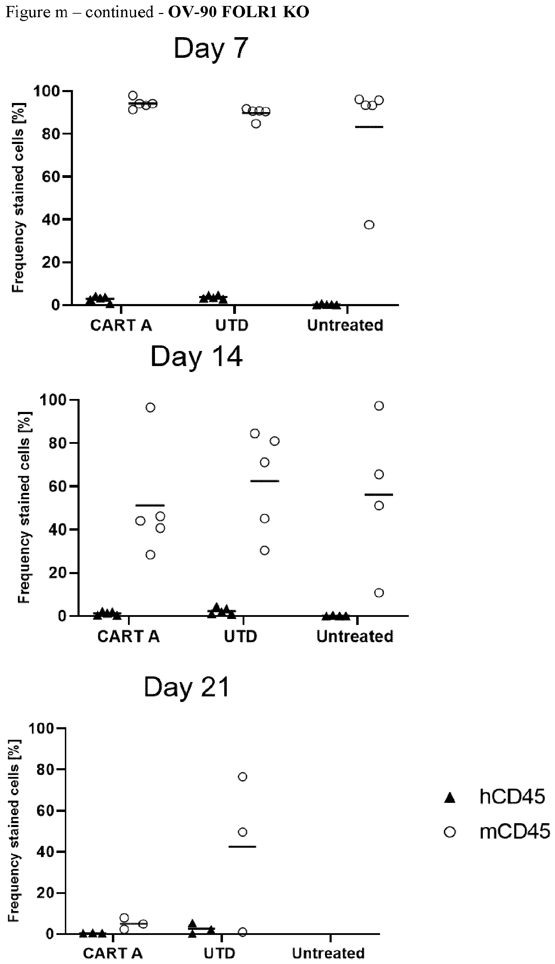

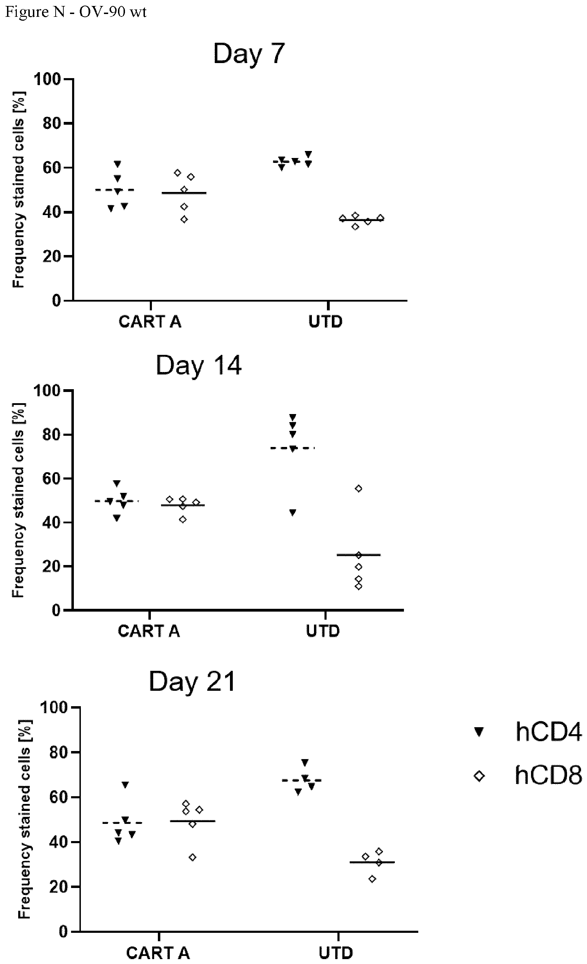

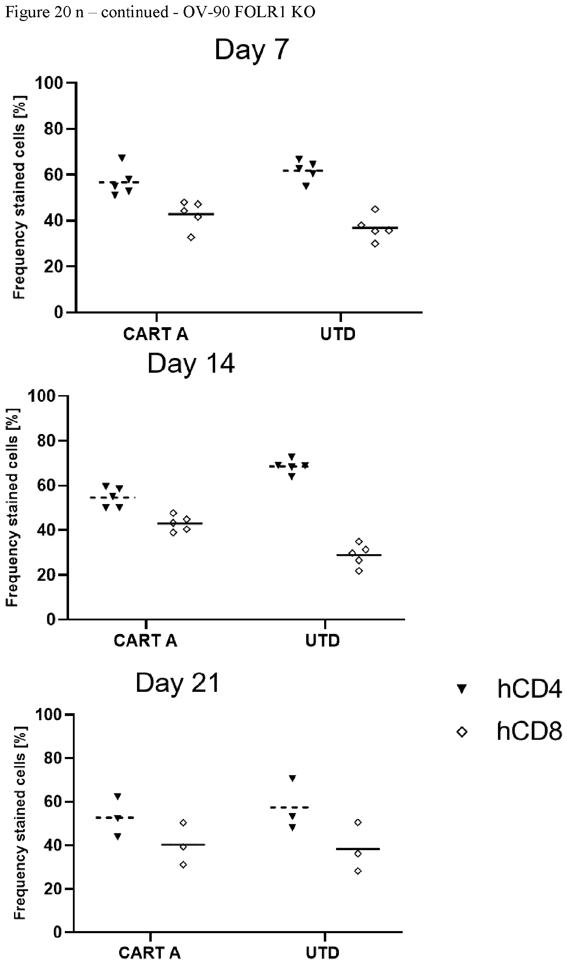

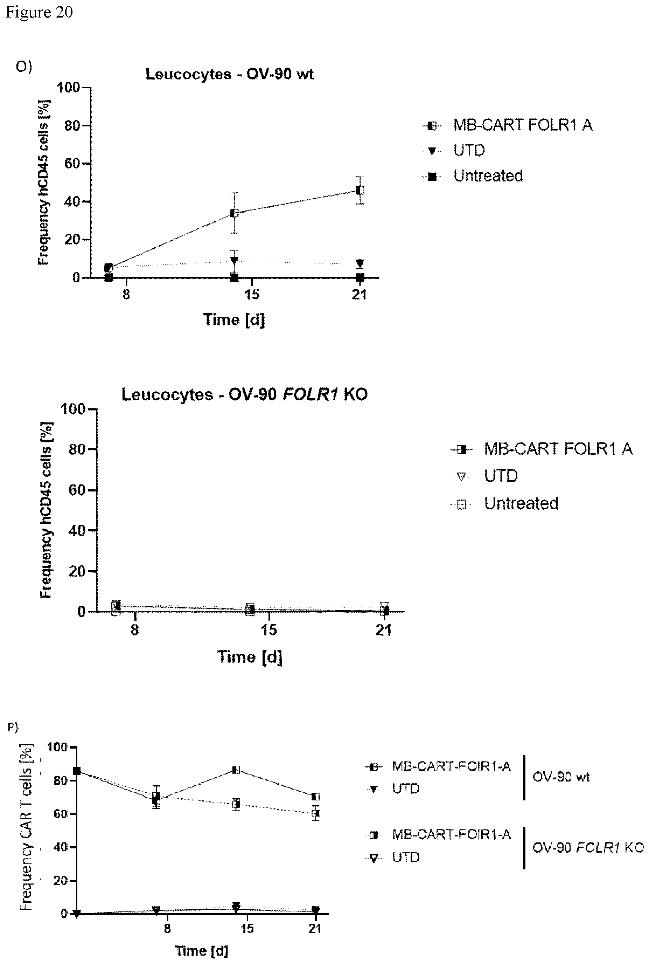

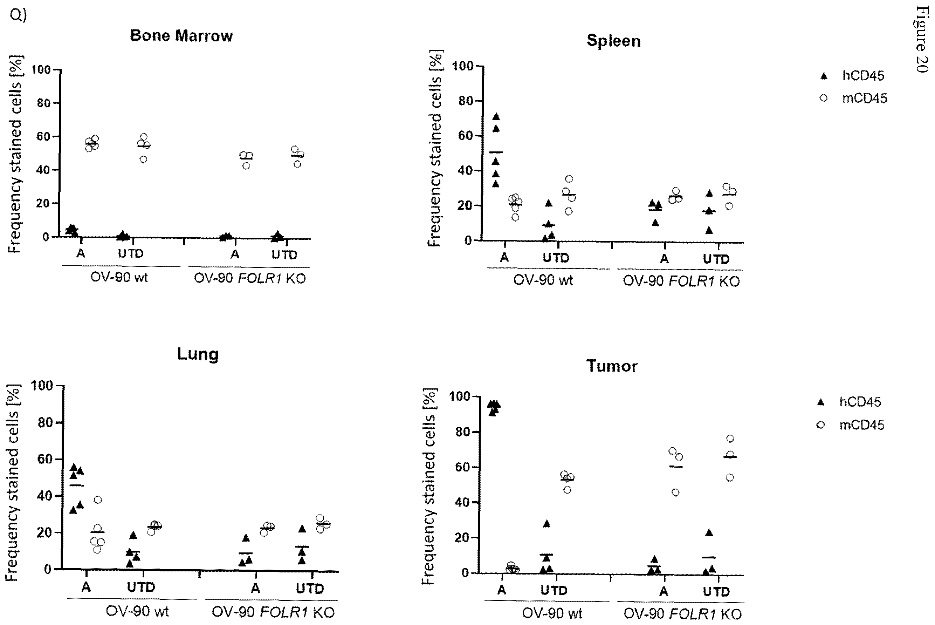

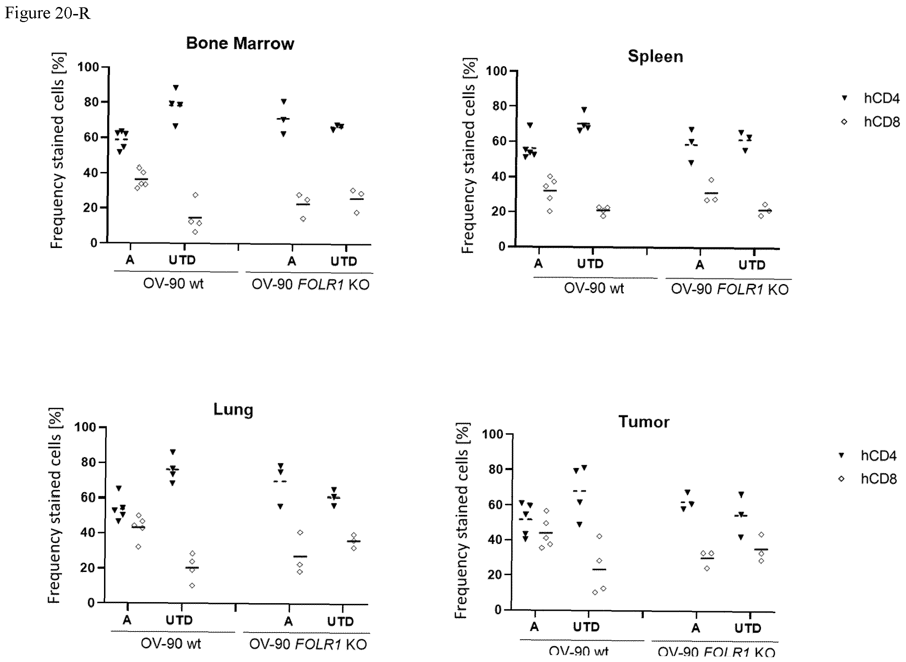

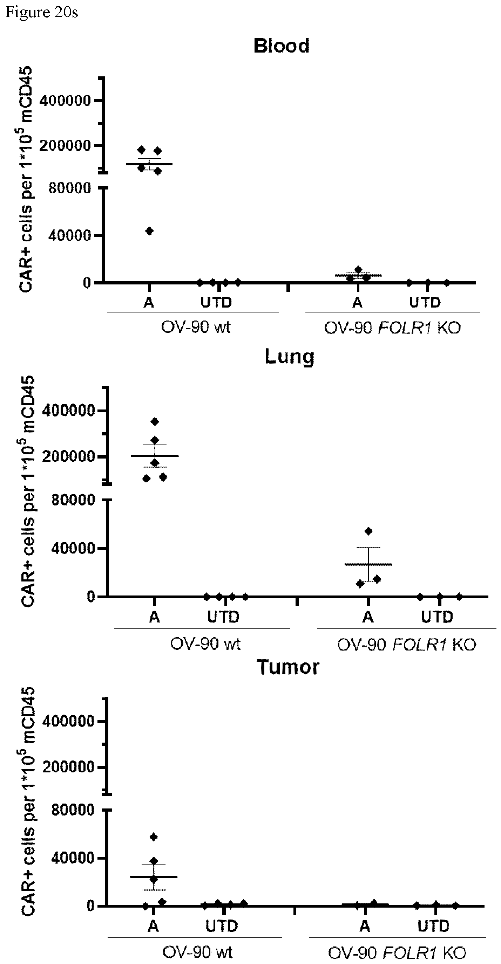

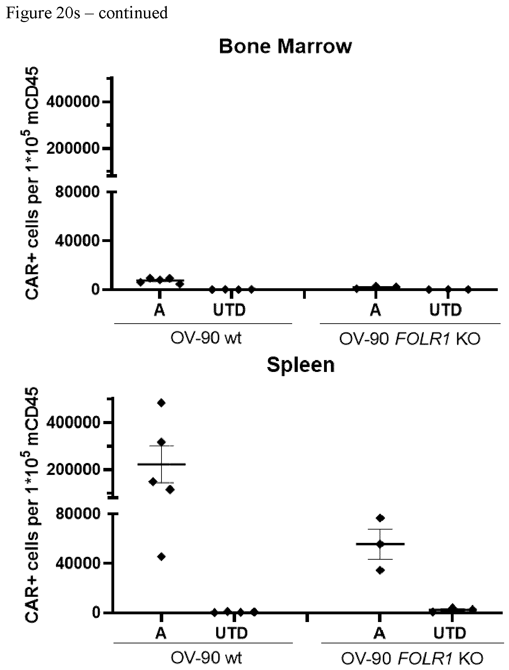

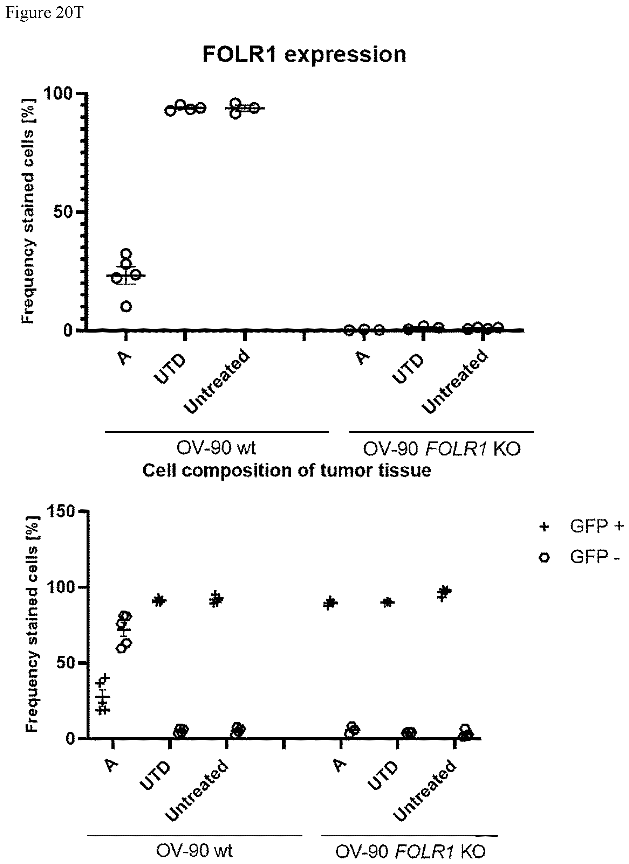

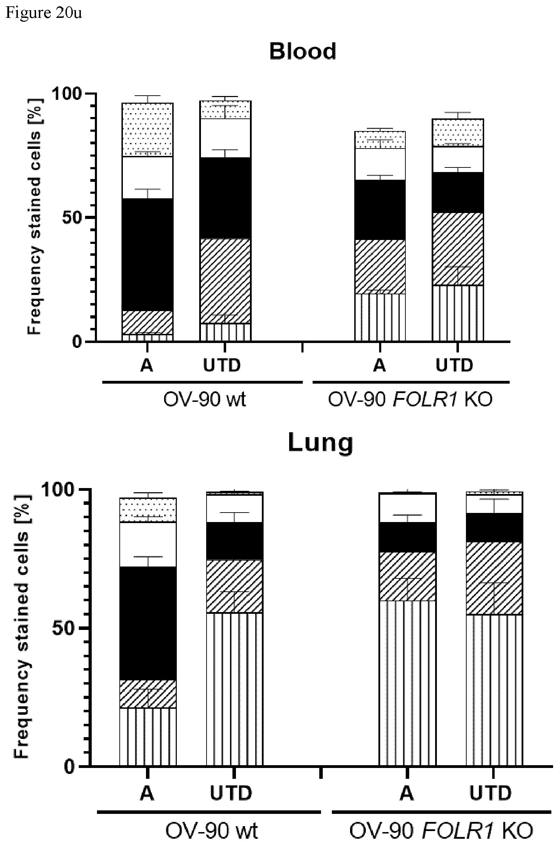

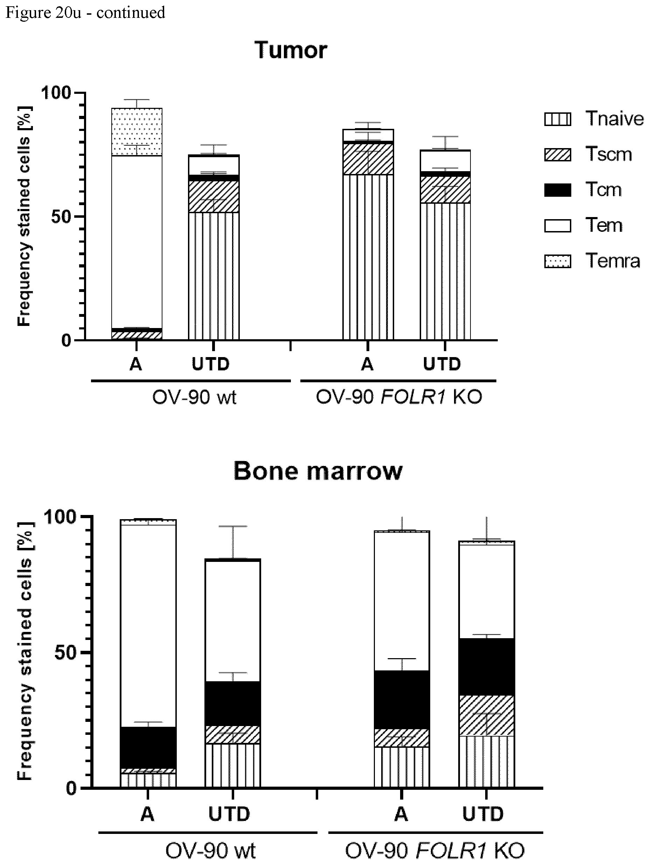

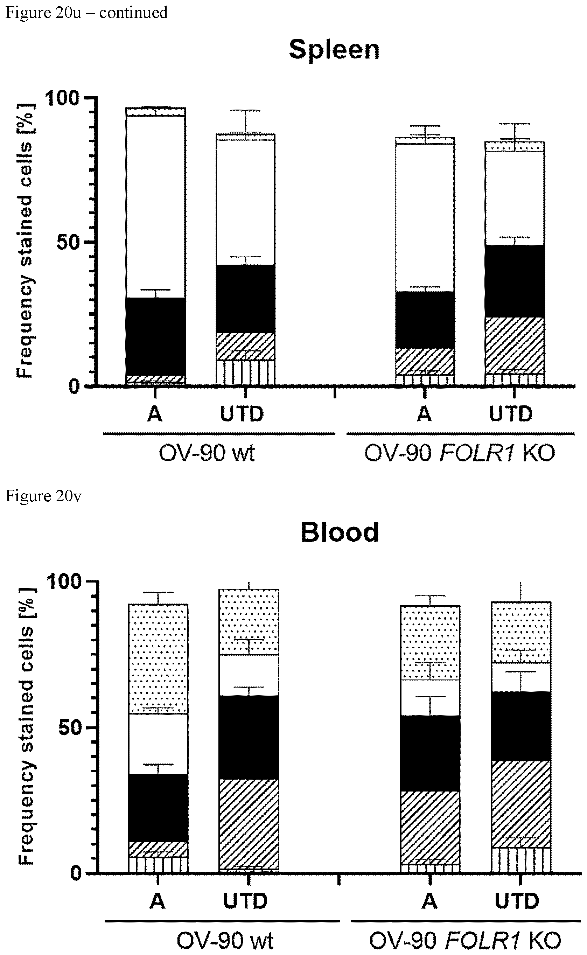

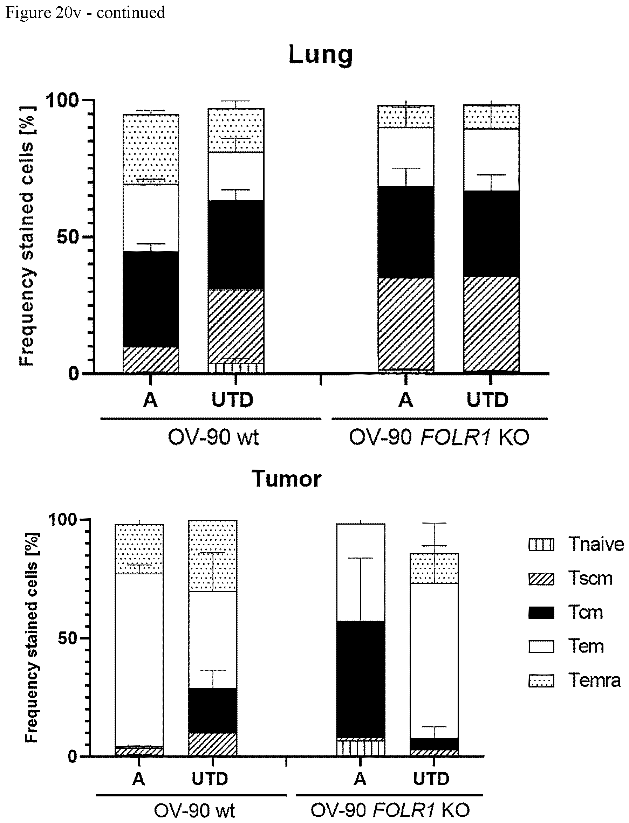

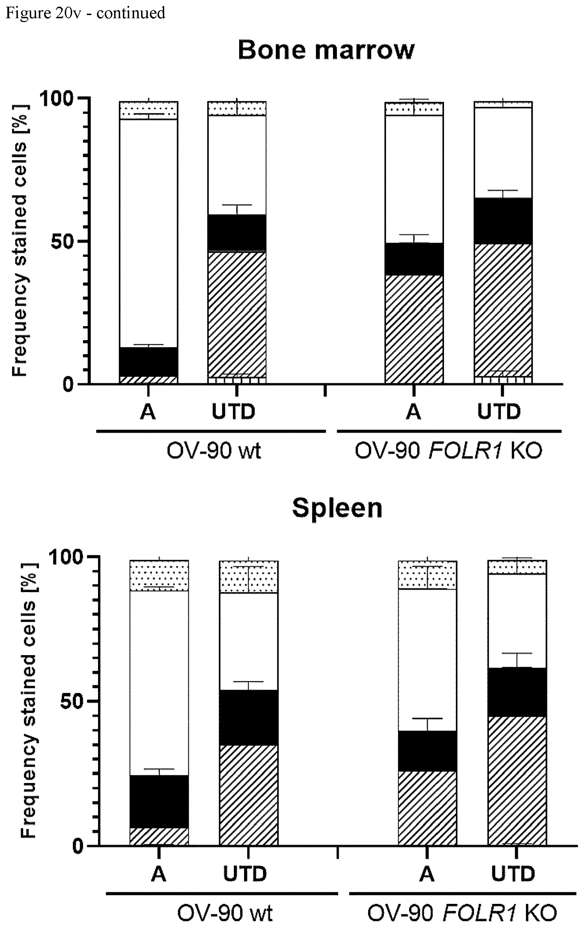

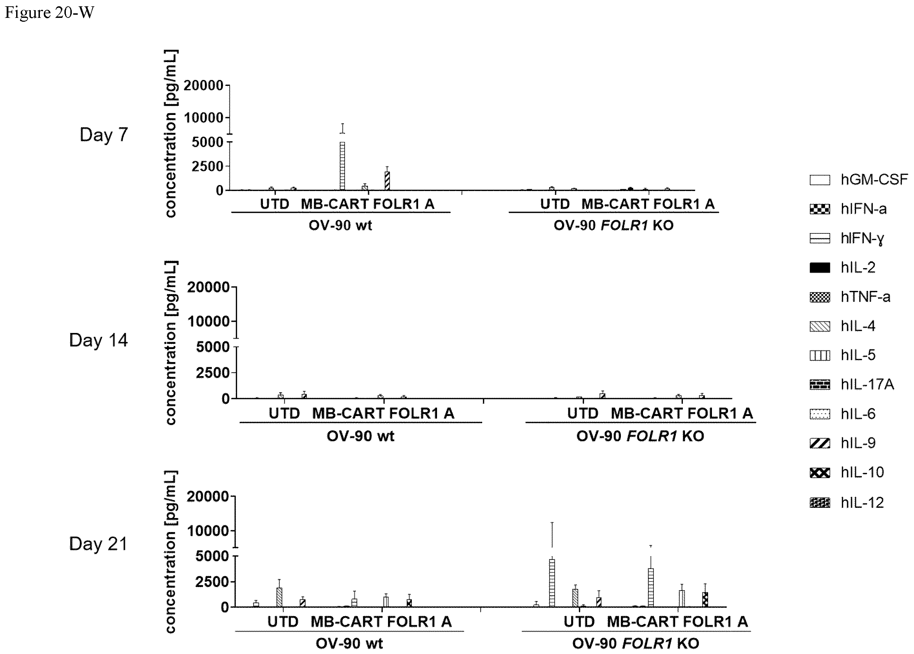

Figure 20: MB-FOLR1 CART A T cells specifically eradicate only OV-90 FOLR1 expressing tumors and not OV-90 FOLR1 KO tumors in NSG mice.
   NSG mice were injected with OV-90 wt or OV-90 FOLR1 KO cells subcutaneously and after 21 days intravenously injected with anti-FOLR1 CAR T cells (1×10⁷ total T cells; MB-CART-FOLR1 A) or untransduced T cells (UTD) produced in CliniMACS Prodigy^{®} to assess anti-tumor function.
   A) Scheme of study design including time points of analysis.
   B) White blood cell composition of leukapheresis (Ori), isolated pan T cells (Pos) and MB-CART FOLR1 candidate A (CART A) was analyzed by flow cytometry over the course of CAR T cell generation. Cell populations were defined as: T cells: CD3+, CD56-, CD16-; NKT cells: CD3+, CD56+, CD16+; Monocytes: CD3-, CD14+; B cells: CD3-, CD19+; Neutrophils: CD3-, CD14-, CD19-, CD56+, CD16+; Eosinophils: CD3-, CD14-, CD19-, CD56-, CD16- .
   C) Viability of CAR T cells was monitored over time by flow cytometry during the CliniMACS Prodigy^{®} TCT process and analyzed by flow cytometry (7AAD staining).
   D) Human CD4 and CD8 T cell composition of final CAR T cells at the end of the CliniMACS Prodigy^{®} TCT process was analyzed by flow cytometry.
   E) Transduction efficiency of CAR T cells was monitored over time by flow cytometry during the CliniMACS Prodigy^{®} TCT process and analyzed by flow cytometry (FOLR1-Fc fusion protein staining).
   F) Vector copy number of the final CAR T cells was determined by qPCR at the end of the CliniMACS Prodigy^{®} TCT process.
   G) Body weight of mice (OV-90 wt cells injected) was monitored over 21 days post CAR T cell injection. Each line corresponds to an individual mouse.
   H) Body weight of mice (OV-90 FOLR1 KO cells injected) was monitored over 21 days post CAR T cell injection. Each line corresponds to an individual mouse.
   I) Representative bioluminescence images of individual OV-90 xenograft tumor bearing NSG mice post CAR T cell injection. Color scale for all images, min = 1.08×10⁸, max = 1.27×10¹⁰.
   J) Quantification of bioluminescence as total flux in photons per seconds (p/s) of individual mice (OV-90 wt cells injected) after CAR T cell injection over 21 days. Each line represents an individual mouse.
   K) Quantification of bioluminescence as total flux in photons per seconds (p/s) of individual mice (OV-90 FOLR1 KO cells injected) after CAR T cell injection over 21 days. Each line represents an individual mouse.
   L) Quantification of bioluminescence as total flux in photons per seconds (p/s) of the different experimental groups after CAR T cell injection over 21 days. Data points represent mean ± SEM.
   M) Mouse and human leucocytes composition in peripheral blood of CAR T cell treated mice was analyzed. Murine blood samples were analyzed by flow cytometry for murine or human CD45 expression, respectively, at day 7, 14, and 21 post T cell injection. No data displayed at day 21 for the untreated group with OV-90 FOLR1 KO tumors since the mice reached endpoint criteria and were thus taken out at day 14. Each data point represents an individual mouse and horizontal lines represent the mean of the respective group.
   N) Human T cell composition (CD4, CD8) in peripheral blood of CAR T cell treated mice was assessed. Human T cells were analyzed by flow cytometry for human CD4 and CD8 expression, respectively, in blood samples collected at day 7, 14 and 21 post T cell injection. Each data point represents an individual mouse and horizontal lines represent the mean of the respective group.
   O) Human leucocytes in peripheral blood of CAR T cell treated mice were analyzed by flow cytometry over time. Human CD45 expression in blood samples collected at day 7, 14, and 21 post T cell injection was measured. Data is shown as mean ± SEM.
   P) CAR expression was analyzed over time in human T cells from peripheral blood samples of mice treated with the indicated CAR candidates. CAR T cells were analyzed by flow cytometry with FOLR1-Fc fusion protein staining, in blood samples collected at day 7, 14, and 21 post T cell injection. Data is shown as mean ± SEM.
   Q) Mouse and human leucocytes composition in mouse bone marrow, spleen, lung, and tumor was analyzed. On day 21 post T cell injection mice were sacrificed and the respective organs collected and dissociated. Single cell suspensions of the organs were analyzed ex vivo by flow cytometry for murine or human CD45 expression, respectively. Each data point represents an individual mouse and horizontal lines represent the mean of the respective group.
   R) Human T cell composition (CD4, CD8) in mouse bone marrow, spleen, lung, and tumor was assessed. On day 21 post T cell injection mice were sacrificed and the respective organs collected and dissociated. Single cell suspensions of the organs were analyzed ex vivo by flow cytometry for human CD4 and CD8 expression, respectively. Each data point represents an individual mouse and horizontal lines represent the mean of the respective group.
   S) Quantification of CAR expressing cells relatively to mouse CD45 cells in murine blood, lung, bone marrow, spleen and tumor. On day 21 post T cell injection mice were sacrificed and the respective organs collected and dissociated. Single cell suspensions of the organs as well as blood samples were analyzed ex vivo by flow cytometry for FOLR1-Fc fusion protein staining. Each data point represents an individual mouse and horizontal lines represent the mean of the respective group together with error bars of ± SEM.
   T) FOLR1 expression and cell composition in remaining tumor tissues at study endpoints. Composition of the xenograft tissue represented as GFP positive tumor cells and other cells (human or murine immune cells) proportions is depicted in the right panel.
      Ex vivo analysis were performed at the study endpoint, 21 days after CAR T cell injection for all groups except for the untreated group with OV-90 FOLR1 KO tumors. For these, the mice reached endpoint criteria earlier and were thus taken out at day 14.
   U) Human CD4 T cells from mouse blood, bone marrow, spleen, lung, and tumor were phenotyped. On day 21 post T cell injection mice were sacrificed and the respective organs collected and dissociated. Single cell suspensions of the organs were analyzed ex vivo. T cells were assessed for phenotype marker expression by flow cytometry. T cell subtypes were defined as following: Tscm: CD45RO-, CD197+, CD62L+, CD95+; Tnaive: CD8+ or CD4+, CD45RO-, CD197+, CD62L+, CD95-; Temra: CD45RO-, CD197-, CD62L-, CD95+; Tem: CD45RO+, CD62L-, CD95+; and Tcm: CD45RO+, CD62L+, CD95+. Each bar represents mean +/- SEM of the respective group.
   V) Human CD8 T cells from mouse blood, bone marrow, spleen, lung, and tumor were phenotyped. On day 21 post T cell injection mice were sacrificed and the respective organs collected and dissociated. Single cell suspensions of the organs were analyzed ex vivo. T cells were assessed for phenotype marker expression by flow cytometry. T cell subtypes were defined as following: Tscm: CD45RO-, CD197+, CD62L+, CD95+; Tnaive: CD8+ or CD4+, CD45RO-, CD197+, CD62L+, CD95-; Temra: CD45RO-, CD197-, CD62L-, CD95+; Tem: CD45RO+, CD62L-, CD95+; and Tcm: CD45RO+, CD62L+, CD95+. Each bar represents mean +/- SEM of the respective group.
   W) Secretion of human cytokines was analyzed in peripheral blood samples over time. Plasma was isolated from blood samples collected at day 7, 14, and 21 post T cell injection and cytokine levels were subsequently determined with the human MACSplex Cytokine 12 Kit. Data is shown as mean ± SEM of values.

### Detailed description of the invention

It was found that FolR1 expressing cancerous cells which are directly targeted by an engineered immune cell expressing a CAR specific for the antigen FolR1 are affected in a manner that these cells fail to grow and/or are prompted to die. In addition to that it was surprisingly found that a specific sequence encoding an antigen binding domain and orientation of the heavy chain variable region of an antibody (VH) and a light chain variable region of an antibody (VL) is more effective in comparison to other variants of anti-FolR1-CARs (Example 2).

In a first aspect, the present invention provides a CAR comprising an antigen binding domain specific for folate receptor 1 (FolR1), a spacer, a transmembrane domain and an intracellular signaling domain. The intracellular signaling domain may comprise a costimulatory signaling domain and/or a primary stimulatory domain.

Said intracellular signaling domain may comprise at least one primary signaling domain comprising an immunoreceptor tyrosine-based activation motif (ITAM) and/or at least one co-stimulatory signaling domain. Said primary cytoplasmic signaling domain of said CAR may be CD3zeta.

The antigen binding domain may comprise e.g. single domain antibody, full length heavy chain, Fab fragments, single chain Fv (scFv) fragments, VHH fragments, divalent single chain antibodies or diabodies, each of which may be specific for the target antigen FolR1.

Said antigen binding domain of said CAR may comprise a heavy chain variable region of an antibody (VH) comprising the amino acid sequence Seq ID No:2 and a light chain variable region of an antibody (VL) comprising the amino acid sequence Seq ID No:4. In one embodiment of the invention the antigen binding domain may comprise from the N- to the C-terminus the VH comprising Seq ID No:2 and VL comprising Seq ID No:4.

Said antigen binding domain of said CAR may be a scFv comprising a heavy chain variable region of an antibody (VH) comprising the amino acid sequence Seq ID No:2 and a light chain variable region of an antibody (VL) comprising the amino acid sequence Seq ID No:4. In one embodiment of the invention the antigen binding domain may be a scFv comprising from the N- to the C-terminus the VH comprising Seq ID No:2 and VL comprising Seq ID No:4.

In one embodiment of the invention, the antigen binding domain of said CAR may comprise the amino acid sequence of Seq ID No: 103.

Said CAR may comprise a hinge domain as spacer, wherein the hinge domain may comprise e.g. a sequence of the hinge of CD8alpha (Seq ID No:34) or IGG4 (Seq ID No:36). In a preferred embodiment the CAR may comprise the hinge domain of CD8alpha, the hinge domain of CD8alpha may comprise SEQ ID NO:34.

In another embodiment of the invention said CAR may comprise an antigen binding domain such as a scFv comprising a heavy chain variable region of an antibody (VH) comprising the amino acid sequence Seq ID No:2 and a light chain variable region of an antibody (VL) comprising the amino acid sequence Seq ID No:4, and said hinge of CD8alpha.

In a preferred embodiment of the invention said CAR may comprise said antigen binding domain such as a scFv comprising from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha.

In another preferred embodiment of the invention said CAR may comprise said antigen binding domain (such as a scFv) comprising from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha comprising SEQ ID NO:34.

In one embodiment of the invention said CAR may comprise said antigen binding domain (such as a scFv) comprising SEQ ID NO:103, and said hinge of CD8alpha.

In another embodiment of the invention said CAR may comprise said antigen binding domain (such as a scFv) comprising SEQ ID NO: 103, and said hinge of CD8alpha comprising SEQ ID NO:34.

Said CAR may comprise a transmembrane domain comprising a sequence of the transmembrane domains from CD8alpha (Seq ID No:38) or CD28 (Seq ID No: 107).

In one embodiment of the invention said CAR may comprise said antigen binding domain (such as a scFv) comprising a heavy chain variable region of an antibody (VH) comprising the amino acid sequence Seq ID No:2 and a light chain variable region of an antibody (VL) comprising the amino acid sequence Seq ID No:4, said hinge of CD8alpha, and said transmembrane domain of CD8alpha.

In another embodiment of the invention said CAR may comprise said antigen binding domain (such as a scFv) comprising a heavy chain variable region of an antibody (VH) comprising the amino acid sequence Seq ID No:2 and a light chain variable region of an antibody (VL) comprising the amino acid sequence Seq ID No:4, and said hinge of CD8alpha comprising SEQ ID NO:34, and said transmembrane domain of CD8alpha comprising Seq ID No:38.

In a preferred embodiment of the invention said CAR may comprise said antigen binding domain (such as a scFv) comprising from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha comprising, and said transmembrane domain of CD8alpha.

In another preferred embodiment of the invention said CAR may comprise said antigen binding domain (such as a scFv) comprising from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha comprising SEQ ID NO:34, and said transmembrane domain of CD8alpha comprising Seq ID No:38.

In one embodiment of the invention said CAR may comprise said antigen binding domain (such as a scFv) comprising SEQ ID NO:103, said hinge of CD8alpha, and said transmembrane domain of CD8alpha.

In preferred embodiment of the invention said CAR may comprise said antigen binding domain (such as a scFv) comprising SEQ ID NO:103, and said hinge of CD8alpha comprising SEQ ID NO:34, and said transmembrane domain of CD8alpha comprising Seq ID No:38.

Said CAR, wherein the intracellular signaling domain of said CAR may comprise e.g. a sequence of the intracellular signaling domains of CD3zeta (Seq ID No:42) and/or one or more of CD28 (SEQ ID NO:109), CD137 (41BB; SEQ ID NO:40) and OX40 (SEQ ID NO:111). In a preferred embodiment the CAR comprises a transmembrane domain of CD8alpha, an intracellular primary (stimulatory) signaling domain of CD3 zeta and an intracellular costimulatory signaling domain of CD137 (41BB).

In one embodiment of the invention said CAR may comprise said antigen binding domain (such as a scFv) comprising a heavy chain variable region of an antibody (VH) comprising the amino acid sequence Seq ID No:2 and a light chain variable region of an antibody (VL) comprising the amino acid sequence Seq ID No:4, said hinge of CD8alpha, said transmembrane domain of CD8alpha, and an intracellular signaling domain comprising the costimulatory domain of CD137 and stimulatory domain of CD3zeta.

In a preferred embodiment of the invention said CAR may comprise said antigen binding domain (such as a scFv) comprising from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha, and said transmembrane domain of CD8alpha, and an intracellular signaling domain comprising the costimulatory domain of CD137 and stimulatory domain of CD3zeta.

In another preferred embodiment of the invention said CAR may comprise said antigen binding domain (such as a scFv) comprising from the N- to the C-terminus a heavy chain variable region of an antibody (VH) comprising the amino acid sequence Seq ID No:2 and a light chain variable region of an antibody (VL) comprising the amino acid sequence Seq ID No:4, said hinge of CD8alpha comprising SEQ ID NO:34, said transmembrane domain of CD8alpha comprising Seq ID No:38, and an intracellular signaling domain comprising the costimulatory domain of CD137 and stimulatory domain of CD3zeta.

In one embodiment of the invention said CAR may comprise said antigen binding domain (such as a scFv) comprising SEQ ID NO:103, and said hinge of CD8alpha, said transmembrane domain of CD8alpha, and an intracellular signaling domain comprising the costimulatory domain of CD137 and stimulatory domain of CD3zeta.

In a preferred embodiment of the invention said CAR may comprise said antigen binding domain (such as a scFv) comprising SEQ ID NO: 103, and said hinge of CD8alpha comprising SEQ ID NO:34, and said transmembrane domain of CD8alpha comprising Seq ID No:38, and an intracellular signaling domain comprising the costimulatory domain of CD137 and stimulatory domain of CD3zeta.

Said CAR may comprise the amino acid sequence of Seq ID No: 105.

In another aspect, the invention provides isolated nucleic acid sequences which encode the FolR1-CARs of the present invention as disclosed herein.

In one embodiment of the present invention a nucleic acid may encode a CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha.

In another embodiment of the present invention a nucleic acid may encode a CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha comprising SEQ ID NO:34.

In a preferred embodiment of the invention a nucleic acid may encode a CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, said hinge of CD8alpha, said transmembrane domain of CD8alpha, and an intracellular signaling domain comprising the costimulatory domain of CD137 and stimulatory domain of CD3zeta.

In one embodiment of the invention a nucleic acid may encode a CAR comprising said antigen binding domain (such as a scFv) comprising SEQ ID NO: 103, and said hinge of CD8alpha.

In a preferred embodiment of the invention a nucleic acid may encode a CAR comprising said antigen binding domain (such as a scFv) comprising SEQ ID NO:103, and said hinge of CD8alpha comprising SEQ ID NO:34.

In yet another preferred embodiment of the invention a nucleic acid may encode a CAR comprising said antigen binding domain (such as a scFv) comprising SEQ ID NO:103, said hinge of CD8alpha, said transmembrane domain of CD8alpha, and an intracellular signaling domain comprising the costimulatory domain of CD137 and stimulatory domain of CD3zeta.

The nucleic acid sequence encoding the CAR as disclosed herein can be contained in a vector, such as a viral vector. The vector may be a DNA vector, an RNA vector, a plasmid vector, a cosmid vector, a herpes virus vector, a measles virus vector, a lentivirus vector, adenoviral vector, or a retrovirus vector, or a combination thereof.

In some embodiments of the invention, the vector further comprises a promoter wherein the promoter is an inducible promoter, a tissue specific promoter, a constitutive promoter, a suicide promoter or any combination thereof.

In another embodiment of the invention, the vector encoding the CAR can be further modified to include one or more operative elements to control the expression of CAR T cells, or to eliminate CAR-T cells by virtue of a suicide switch. The suicide switch can include, for example, an apoptosis inducing signaling cascade or a drug that induces cell death. In a preferred embodiment, the vector expressing the CAR can be further modified to express an enzyme such thymidine kinase (TK) or cytosine deaminase (CD).

In another aspect the invention provides an engineered cell expressing said CAR specific for FolR1 as disclosed herein.

In one embodiment of the present invention an engineered cell may express a CAR comprising an antigen binding domain (such as a scFv) specific for folate receptor 1 (FolR1), a spacer, a transmembrane domain and an intracellular signaling domain, wherein said antigen binding domain may comprise a heavy chain variable region of an antibody (VH) comprising the amino acid sequence Seq ID No:2 and a light chain variable region of an antibody (VL) comprising the amino acid sequence Seq ID No:4.

In another embodiment of the present invention an engineered cell may express a CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha.

In a preferred embodiment of the present invention an engineered cell may express a CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha comprising SEQ ID NO:34.

In another preferred embodiment of the present invention an engineered cell may express a CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, said hinge of CD8alpha, said transmembrane domain of CD8alpha, and an intracellular signaling domain comprising the costimulatory domain of CD137 and stimulatory domain of CD3zeta.

In one embodiment of the invention an engineered cell may express a CAR comprising an antigen binding domain (such as a scFv) specific for folate receptor 1 (FolR1), a spacer, a transmembrane domain and an intracellular signaling domain, wherein said antigen binding domain may comprise SEQ ID NO: 103, and said hinge of CD8alpha.

In another embodiment of the invention an engineered cell may express a CAR comprising an antigen binding domain (such as a scFv) specific for folate receptor 1 (FolR1), a spacer, a transmembrane domain and an intracellular signaling domain, wherein said antigen binding domain may comprise SEQ ID NO: 103, and said hinge of CD8alpha comprising SEQ ID NO:34.

In a preferred embodiment of the invention an engineered cell may express a CAR comprising an antigen binding domain (such as a scFv) specific for folate receptor 1 (FolR1), a spacer, a transmembrane domain and an intracellular signaling domain, wherein said antigen binding domain may comprise SEQ ID NO:103, and said hinge of CD8alpha, said transmembrane domain of CD8alpha, and an intracellular signaling domain comprising the costimulatory domain of CD137 and stimulatory domain of CD3zeta.

In one embodiment of the invention said cell may be an immune cell. In a preferred embodiment of the invention said cell may be a T cell, tumor infiltrating lymphocytes (TILs) or NK cell. In a more preferred embodiment of the invention said cell is a T cell.

In one aspect the invention provides an engineered cell expressing said FolR1 CAR for use in immunotherapy. The immunotherapy may be for treatment of cancer in a subject suffering from cancer, wherein the cancerous cells of said cancer express FolR1. The immunotherapy may be for treatment of cancer in a subject suffering from cancer, wherein at least a subpopulation of the cancerous cells of said cancer express FolR1.

In one embodiment of the present invention an engineered cell expressing a CAR specific for FolR1 may be for use in immunotherapy, the CAR comprising an antigen binding domain (such as a scFv) specific for folate receptor 1 (FolR1), a spacer, a transmembrane domain and an intracellular signaling domain, wherein said antigen binding domain may a heavy chain variable region of an antibody (VH) comprising the amino acid sequence Seq ID No:2 and a light chain variable region of an antibody (VL) comprising the amino acid sequence Seq ID No:4, and said hinge of CD8alpha.

In another embodiment of the invention an engineered cell expressing a CAR specific for FolR1 may be for use in immunotherapy, the CAR comprising an antigen binding domain (such as a scFv) specific for folate receptor 1 (FolR1), a spacer, a transmembrane domain and an intracellular signaling domain, wherein said antigen binding domain may comprise from the N-to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha.

In a preferred embodiment of the present invention an engineered cell expressing a CAR specific for FolR1 may be for use in immunotherapy, the CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha comprising SEQ ID NO:34.

In one embodiment of the present invention an engineered cell expressing a CAR specific for FolR1 may be for use in immunotherapy, the CAR comprising an antigen binding domain (such as a scFv) specific for folate receptor 1 (FolR1), a spacer, a transmembrane domain and an intracellular signaling domain, wherein said antigen binding domain may comprise from the N-to the C-terminus a heavy chain variable region of an antibody (VH) comprising the amino acid sequence Seq ID No:2 and a light chain variable region of an antibody (VL) comprising the amino acid sequence Seq ID No:4, said hinge of CD8alpha, said transmembrane domain of CD8alpha, and an intracellular signaling domain comprising the costimulatory domain of CD137 and stimulatory domain of CD3zeta.

In another embodiment of the present invention an engineered cell expressing a CAR specific for FolR1 may be for use in immunotherapy, the CAR comprising an antigen binding domain (such as a scFv) specific for folate receptor 1 (FolR1), a spacer, a transmembrane domain and an intracellular signaling domain, wherein said antigen binding domain may comprise SEQ ID NO:103, and said hinge of CD8alpha.

In a preferred embodiment of the present invention an engineered cell expressing a CAR specific for FolR1 may be for use in immunotherapy, the CAR comprising an antigen binding domain (such as a scFv) specific for folate receptor 1 (FolR1), a spacer, a transmembrane domain and an intracellular signaling domain, wherein said antigen binding domain may comprise SEQ ID NO: 103, and said hinge of CD8alpha comprising SEQ ID NO:34.

In a more preferred embodiment of the present invention an engineered cell expressing a CAR specific for FolR1 may be for use in immunotherapy, the CAR comprising an antigen binding domain (such as a scFv) specific for folate receptor 1 (FolR1), a spacer, a transmembrane domain and an intracellular signaling domain, wherein said antigen binding domain may comprise SEQ ID NO:103, and said hinge of CD8alpha, said transmembrane domain of CD8alpha, and an intracellular signaling domain comprising the costimulatory domain of CD137 and stimulatory domain of CD3zeta.

Said subpopulation of cancerous cells expressing FolR1 may comprise at least 1 cell which expresses FolR1 out of all cancerous cells in the subject suffering from said cancer. Preferentially said subpopulation of cancerous cells expressing FolR1 may comprise at least 1%, at least 5%, at least 10%, at least 15%, at least 20%, at least 25%, at least 30%, at least 35%, at least 40%, at least 45% or at least 50% of all cancerous cells of a subject suffering from said cancer.

In one aspect the invention provides a population of cells comprising cells expressing FolR1-CAR as disclosed herein.

In one embodiment of the present invention a population of cells may comprise cells expressing FolR1-CAR, the CAR comprising an antigen binding domain (such as a scFv) specific for folate receptor 1 (FolR1), a spacer, a transmembrane domain and an intracellular signaling domain, wherein said antigen binding domain may a heavy chain variable region of an antibody (VH) comprising the amino acid sequence Seq ID No:2 and a light chain variable region of an antibody (VL) comprising the amino acid sequence Seq ID No:4, and said hinge of CD8alpha.

In another embodiment of the present invention a population of cells may comprise cells expressing FolR1-CAR, the CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha.

In preferred embodiment of the present invention a population of cells may comprise cells expressing FolR1-CAR, the CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha comprising SEQ ID NO:34.

In a more preferred embodiment of the present invention a population of cells may comprise cells expressing FolR1-CAR, the CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, said hinge of CD8alpha, said transmembrane domain of CD8alpha, and an intracellular signaling domain comprising the costimulatory domain of CD137 and stimulatory domain of CD3zeta.

In another embodiment of the invention a population of cells may comprise cells expressing FolR1-CAR, the CAR may comprise said antigen binding domain (such as a scFv) comprising SEQ ID NO:103, and said hinge of CD8alpha.

In a preferred embodiment of the invention a population of cells may comprise cells expressing FolR1-CAR, the CAR may comprise said antigen binding domain (such as a scFv) comprising SEQ ID NO:103, and said hinge of CD8alpha comprising SEQ ID NO:34.

In more preferred embodiment of the invention a population of cells may comprise cells expressing FolR1-CAR, the CAR may comprise said antigen binding domain (such as a scFv) comprising SEQ ID NO: 103, said hinge of CD8alpha, hinge of CD8alpha, said transmembrane domain of CD8alpha, and an intracellular signaling domain comprising the costimulatory domain of CD137 and stimulatory domain of CD3zeta.

Said population or isolated population of engineered cells are expanded to therapeutically effective amount of cells before use in said immunotherapy. Said cancer may be selected from the group consisting of triple-negative breast cancer, gastric cancer, lung cancer, renal cancer, pancreatic cancer or ovarian cancer. Said cancer may be ovarian cancer. Said cells may be immune cells or immune cell subsets, preferentially T cell, tumor infiltrating lymphocytes (TILs) or NK cell, more preferentially T-cells.

In one aspect the invention provides a method for treating cancer comprising administering to a subject in need thereof engineered cells expressing FolR1-CAR as disclosed herein. The treatment of cancer may be in a subject suffering from cancer, wherein at least a subpopulation of the cancerous cells of said cancer express FolR1.

In one embodiment of the present invention a method for treating cancer in a subject may comprise administering to a subject in need thereof engineered cells expressing a CAR specific for FolR1, the CAR comprising an antigen binding domain (such as a scFv) specific for folate receptor 1 (FolR1), a spacer, a transmembrane domain and an intracellular signaling domain, wherein said antigen binding domain may the amino acid sequence Seq ID No:2 and a light chain variable region of an antibody (VL) comprising the amino acid sequence Seq ID No:4, and said hinge of CD8alpha.

In another embodiment of the present invention a method for treating cancer in a subject may comprise administering to a subject in need thereof engineered cells expressing a CAR specific for FolR1, the CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha.

In preferred embodiment of the present invention a method for treating cancer in a subject may comprise administering to a subject in need thereof engineered cells expressing a CAR specific for FolR1, the CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha comprising SEQ ID NO:34.

In a more preferred embodiment of the present invention a method for treating cancer in a subject may comprise administering to a subject in need thereof engineered cells expressing a CAR specific for FolR1, the CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, said hinge of CD8alpha, said transmembrane domain of CD8alpha, and an intracellular signaling domain comprising the costimulatory domain of CD137 and stimulatory domain of CD3zeta.

In one embodiment of the invention a method for treating cancer in a subject may comprise administering to a subject in need thereof engineered cells expressing a CAR specific for FolR1, the CAR comprising an antigen binding domain (such as a scFv) specific for folate receptor 1 (FolR1), a spacer, a transmembrane domain and an intracellular signaling domain, wherein said antigen binding domain comprising SEQ ID NO: 103, and said hinge of CD8alpha.

In a preferred embodiment of the present invention a method for treating cancer in a subject may comprise administering to a subject in need thereof engineered cells expressing a CAR specific for FolR1, the CAR comprising an antigen binding domain (such as a scFv) specific for folate receptor 1 (FolR1), a spacer, a transmembrane domain and an intracellular signaling domain, wherein said antigen binding domain comprising SEQ ID NO:103, and said hinge of CD8alpha comprising SEQ ID NO:34.

In a more preferred embodiment of the present invention a method for treating cancer in a subject may comprise administering to a subject in need thereof engineered cells expressing a CAR specific for FolR1, the CAR comprising an antigen binding domain (such as a scFv) specific for folate receptor 1 (FolR1), a spacer, a transmembrane domain and an intracellular signaling domain, wherein said antigen binding domain comprising SEQ ID NO: 103, said hinge of CD8alpha, said transmembrane domain of CD8alpha, and an intracellular signaling domain comprising the costimulatory domain of CD137 and stimulatory domain of CD3zeta.

Said cancer may be selected from the group consisting of triple-negative breast cancer, gastric cancer, lung cancer, renal cancer, pancreatic cancer and ovarian cancer. Said cells may be immune cells or immune cell subsets, preferentially T cells or T cell subsets or NK cells or NK cells subsets.

In one aspect, the invention provides a pharmaceutical composition comprising genetically modified cells expressing a CAR specific for the antigen FoLR1 as disclosed herein and optional a pharmaceutically acceptable carrier.

In one embodiment of the present invention a pharmaceutical composition may comprise genetically modified cells expressing a CAR specific for FolR1, the CAR comprising an antigen binding domain (such as a scFv) specific for folate receptor 1 (FolR1), a spacer, a transmembrane domain and an intracellular signaling domain, wherein said antigen binding domain may comprise the amino acid sequence Seq ID No:2 and a light chain variable region of an antibody (VL) comprising the amino acid sequence Seq ID No:4, and said hinge of CD8alpha and optional a pharmaceutically acceptable carrier.

In another embodiment of the present invention a pharmaceutical composition may comprise genetically modified cells expressing a CAR specific for FolR1, the CAR comprising an antigen binding domain (such as a scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha and optional a pharmaceutically acceptable carrier.

In a preferred embodiment of the present invention a pharmaceutical composition may comprise genetically modified cells expressing a CAR specific for FolR1, the CAR comprising an antigen binding domain (such as scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, and said hinge of CD8alpha comprising SEQ ID NO:34 and optional a pharmaceutically acceptable carrier.

In a more preferred embodiment of the present invention a pharmaceutical composition may comprise genetically modified cells expressing a CAR specific for FolR1, the CAR comprising an antigen binding domain (such as scFv) specific for folate receptor 1 (FolR1), a spacer, a transmembrane domain and an intracellular signaling domain, wherein said antigen binding domain may comprise from the N- to the C-terminus a heavy chain variable region of an antibody (VH) comprising the amino acid sequence Seq ID No:2 and a light chain variable region of an antibody (VL) comprising the amino acid sequence Seq ID No:4, said hinge of CD8alpha, said transmembrane domain of CD8alpha, and an intracellular signaling domain comprising the costimulatory domain of CD137 and stimulatory domain of CD3zetaand optional a pharmaceutically acceptable carrier.

In one embodiment of the present invention a pharmaceutical composition may comprise genetically modified cells expressing a CAR specific for FolR1, the CAR comprising an antigen binding domain (such as scFv) specific for folate receptor 1 (FolR1), a spacer, a transmembrane domain and an intracellular signaling domain, wherein said antigen binding domain may comprise SEQ ID NO:103, and said hinge of CD8alpha, and optional a pharmaceutically acceptable carrier.

In a preferred embodiment of the present invention a pharmaceutical composition may comprise genetically modified cells expressing a CAR specific for FolR1, the CAR comprising an antigen binding domain (such as scFv) specific for folate receptor 1 (FolR1), a spacer, a transmembrane domain and an intracellular signaling domain, wherein said antigen binding domain may comprise SEQ ID NO: 103, and said hinge of CD8alpha comprising SEQ ID NO:34, and optional a pharmaceutically acceptable carrier.

In another preferred embodiment of the present invention a pharmaceutical composition may comprise genetically modified cells expressing a CAR specific for FolR1, the CAR comprising an antigen binding domain (such as a scFv) specific for folate receptor 1 (FolR1), a spacer, a transmembrane domain and an intracellular signaling domain, wherein said antigen binding domain may comprise SEQ ID NO: 103, said hinge of CD8alpha, said hinge of CD8alpha, said transmembrane domain of CD8alpha, and an intracellular signaling domain comprising the costimulatory domain of CD137 and stimulatory domain of CD3zeta and optional a pharmaceutically acceptable carrier.

Pharmaceutical acceptable carriers, diluents or excipients may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

Said pharmaceutical composition may be used for the treatment of cancer in a subject suffering from cancer, wherein said cancerous cells of said cancer express FolR1. Said cancer may be selected from the group triple-negative breast cancer, gastric cancer, lung cancer, renal cancer, pancreatic cancer and ovarian cancer. In a preferred embodiment, said cancer is ovarian cancer. Said cells may be immune cells or immune cell subsets, preferentially T cells or T cell subsets or NK cells or NK cells subsets.

In another embodiment the invention provides a pharmaceutical composition comprising genetically modified cells expressing FolR1-CAR as disclosed herein and a pharmaceutically acceptable carrier and a chemotherapeutical agent for combined treatment of said cancer.

All definitions, characteristics and embodiments defined herein with regard to the first aspect of the invention as disclosed herein also apply mutatis mutandis in the context of the other aspects of the invention as disclosed herein.

### Definitions

Unless defined otherwise, technical and scientific terms used herein have the same meaning as commonly understood by one of ordinary skill in the art to which this invention belongs.

As used herein the term "comprising" or "comprises" is used in reference to compositions, methods, and respective component(s) thereof, that are essential to the method or composition, yet open to the inclusion of unspecified elements, whether essential or not.

The term folate receptor 1, FolR1, FOLR, FOLR1, and FR1 can be used interchangeably. It is understood that this also includes other state of the art synonyms such as folate receptor alpha FRalpha or FRα. Folate-binding protein, FBP or Neurodegeneration due to cerebral folate transport deficiency, NCFTD.

In general, a CAR may comprise an extracellular domain (extracellular part) comprising the antigen binding domain, a transmembrane domain and a cytoplasmic signaling domain (intracellular signaling domain). The antigen binding domain is linked to the transmembrane domain by a spacer (hinge domain). The extracellular domain may also comprise a signal peptide.

A "signal peptide" refers to a peptide sequence that directs the transport and localization of the protein within a cell, e.g. to a certain cell organelle (such as the endoplasmic reticulum) and/or the cell surface.

Generally, an "antigen binding domain" refers to the region of the CAR that specifically binds to an antigen, e.g. to a tumor associated antigen (TAA) or tumor specific antigen (TSA). The CARs of the invention may comprise one or more antigen binding domains (e.g. a tandem CAR). Generally, the targeting regions of the CAR are localized extracellularly. The antigen binding domain may comprise an antibody, single domain antibody or an antigen binding fragment thereof. The antigen binding domain may comprise, for example Fab fragments, single chain Fv (scFv) fragments, VHH fragments, divalent single chain antibodies or diabodies. Any molecule that binds specifically to a given antigen such as affibodies or ligand binding domains from naturally occurring receptors may be used as an antigen binding domain. Often the antigen binding domain is a scFv. Normally, in a scFv the variable regions of an immunoglobulin heavy chain and light chain are fused by a flexible linker to form a scFv. Such a linker may be for example the "(G₄/S)₃-linker" or a "whitlow linker".

In some instances, it is beneficial for the antigen binding domain to be derived from the same species in which the CAR will be used in. For example, when it is planned to use it therapeutically in humans, it may be beneficial for the antigen binding domain of the CAR to comprise a human or humanized antibody or antigen binding fragment thereof. Human or humanized antibodies or antigen binding fragments thereof can be made by a variety of methods well known in the art.

"Spacer" or "hinge" as used herein refers to the hydrophilic region which is between the antigen binding domain of the CAR and the transmembrane domain. The CARs may comprise an extracellular spacer domain but is it also possible to leave out such a spacer. The CAR of the present invention comprises such a spacer. The spacer may include e.g. Fc fragments of antibodies or fragments thereof, hinge regions of antibodies or fragments thereof, CH2 or CH3 regions of antibodies, accessory proteins, artificial spacer sequences or combinations thereof. A prominent example of a spacer is the CD8alpha hinge or IgG4 hinge.

The transmembrane domain of the CAR may be from any desired natural or synthetic source for such domain. When the source is natural the domain may be from any membrane-bound or transmembrane protein. The transmembrane domain may be for example from CD8alpha or CD28. When the key signaling and antigen recognition modules (domains) are on two (or even more) polypeptides then the CAR may have two (or more) transmembrane domains. The splitting key signaling and antigen recognition modules enable for e.g. a small molecule-dependent, titratable and reversible control over CAR cell expression (e.g. WO2014127261A1) due to small molecule-dependent heterodimerizing domains in each polypeptide of the CAR.

The cytoplasmic signaling domain (the intracellular signaling domain or the activating endodomain) of the CAR is responsible for activation of at least one of the normal effector functions of the immune cell in which the CAR is expressed, if the respective CAR is an activating CAR (normally, a CAR as described herein refers to an activating CAR). "Effector function" means a specialized function of a cell, e.g. in a T cell an effector function may be cytolytic activity or helper activity including the secretion of cytokines. The intracellular signaling domain refers to the part of a protein which transduces the effector function signal and directs the cell expressing the CAR to perform a specialized function. The intracellular signaling domain may include any complete, mutated or truncated part of the intracellular signaling domain of a given protein sufficient to transduce a signal which initiates or blocks immune cell effector functions.

Prominent examples of intracellular signaling domains for use in the CARs include the cytoplasmic signaling sequences of the T cell receptor (TCR) and co-receptors that initiate signal transduction following antigen receptor engagement.

Generally, T cell activation can be mediated by two distinct classes of cytoplasmic signaling sequences, firstly those that initiate antigen-dependent primary activation through the TCR (primary cytoplasmic signaling sequences, primary cytoplasmic signaling domain) and secondly those that act in an antigen-independent manner to provide a secondary or co-stimulatory signal (secondary cytoplasmic signaling sequences, co-stimulatory signaling domain). Therefore, an intracellular signaling domain of a CAR may comprise one or more primary cytoplasmic signaling domains and/or one or more secondary cytoplasmic signaling domains.

Primary cytoplasmic signaling domains that act in a stimulatory manner may contain ITAMs (immunoreceptor tyrosine-based activation motifs). Examples of ITAM containing primary cytoplasmic signaling domains often used in CARs are that those from TCRzeta (CD3zeta), FcRgamma, FcRbeta, CD3gamma, CD3delta, CD3epsilon, CD5, CD22, CD79a, CD79b, and CD66d. Most prominent is sequence from CD3zeta.

The cytoplasmic domain of the CAR may be designed to comprise the CD3zeta signaling domain by itself or combined with any other desired cytoplasmic domain(s). The cytoplasmic domain of the CAR can comprise a CD3zeta chain portion and a co-stimulatory signaling region (domain). The co-stimulatory signaling region refers to a part of the CAR comprising the intracellular domain of a co-stimulatory molecule. A co-stimulatory molecule is a cell surface molecule other than an antigen receptor or their ligands that is required for an efficient response of lymphocytes to an antigen. Examples for a co-stimulatory molecule are CD27, CD28, 4-1BB (CD137), OX40, CD30, CD40, PD-1, ICOS, lymphocyte function-associated antigen- 1 (LFA-1), CD2, CD7, LIGHT, NKG2C, B7-H3.

The cytoplasmic signaling sequences within the cytoplasmic signaling part of the CAR may be linked to each other with or without a linker in a random or specified order. A short oligo- or polypeptide linker, which is preferably between 2 and 10 amino acids in length, may form the linkage. A prominent linker is the glycine-serine doublet.

As an example, the cytoplasmic domain may comprise the signaling domain of CD3zeta and the signaling domain of CD28. In another example the cytoplasmic domain may comprise the signaling domain of CD3zeta and the signaling domain of CD137. In a further example, the cytoplasmic domain may comprise the signaling domain of CD3zeta, the signaling domain of CD28, and the signaling domain of CD137.

As aforementioned either the extracellular part or the transmembrane domain or the cytoplasmic domain of a CAR may also comprise a heterodimerizing domain for the aim of splitting key signaling and antigen recognition modules of the CAR.

In some embodiments, the endodomain may contain a primary cytoplasmic signaling domains or a co-stimulatory region, but not both. In these embodiments, an immune effector cell containing the CAR is only activated if another CAR containing the missing domain also binds its respective antigen.

The CAR may be a "SUPRA" (split, universal, and programmable) CAR, where a "zipCAR" domain may link an intra-cellular costimulatory domain and an extracellular leucine zipper (WO2017/091546). This zipper may be targeted with a complementary zipper fused e.g. to an scFv region to render the SUPRA CAR T cell tumor specific. This approach would be particularly useful for generating universal CAR T cells for various tumors; adapter molecules could be designed for tumor specificity and would provide options for altering specificity post-adoptive transfer, key for situations of selection pressure and antigen escape.

The CAR may be further modified to include on the level of the nucleic acid encoding the CAR one or more operative elements to eliminate CAR expressing immune cells by virtue of a e.g. suicide switch. The suicide switch can include, for example, an apoptosis inducing signaling cascade or a drug that induces cell death. In one embodiment, the nucleic acid expressing and encoding the CAR can be further modified to express an enzyme such thymidine kinase (TK) or cytosine deaminase (CD). The CAR may also be part of a gene expression system that allows controlled expression of the CAR in the immune cell. Such a gene expression system may be an inducible gene expression system and wherein when an induction agent is administered to a cell being transduced with said inducible gene expression system, the gene expression system is induced and said CAR is expressed on the surface of said transduced cell.

The CARs of the present invention may be designed to comprise any portion or part of the above-mentioned domains as described herein in any order and/or combination resulting in a functional CAR, i.e. a CAR that mediated an immune effector response of the immune effector cell that expresses the CAR as disclosed herein.

The term "engineered" as used herein can refer to one or more human-designed alterations of a nucleic acid, e.g., the nucleic acid within an organism's genome. An "engineered cell" or "genetically modified cell" can refer to a cell with an added, deleted and/or altered gene. Especially, the terms refer to the fact that cells, preferentially T cells can be manipulated by recombinant methods well known in the art to express stably or transiently peptides or proteins which are not expressed in these cells in the natural state. For example, T cells, preferentially human T cells are engineered to express an artificial construct such as a chimeric antigen receptor on their cell surface.

The engineered cell expressing a CAR may be further modified by genetic engineering using methods well known in the art e.g. Meganucleases, TALEN, CrisprCas, zink finger nucleases, shRNA and /or miRNA. Said cells may be modified to reduce or lack expression of a specific gene, which is normally expressed in the cell e.g. T cell receptor (TCR), MHC, co-inhibitory molecules like PD-1, CTLA-4, BTLA, TIGIT, Tim-3, CD244, LAIR, Lag-3, CD160, HVEM .

Said cells may be modified to express additional transgenes such as therapeutic controls, cytokines and/or fragments, cytokine receptors and/or fragments, cytokine receptor fusion proteins, costimulatory receptors or armoring molecules.

The term "antibody" as used herein is used in the broadest sense to cover the various forms of antibody structures including but not being limited to monoclonal and polyclonal antibodies (including full length antibodies), single domain antibodies, multispecific antibodies (e.g. bispecific antibodies), antibody fragments, i.e. antigen binding fragments of an antibody, immunoadhesins and antibody-immunoadhesin chimeras, that specifically recognize (i.e. bind) an antigen. "Antigen binding fragments" comprise a portion of a full-length antibody, preferably the variable domain thereof, or at least the antigen binding site thereof ("an antigen binding fragment of an antibody"). Examples of antigen binding fragments include Fab (fragment antigen binding), scFv (single chain fragment variable), VHH fragments, diabodies, dsFv, Fab', diabodies, single-chain antibody molecules, and multispecific antibodies formed from antibody fragments.

The terms "having specificity for", "specifically binds" or "specific for" with respect to an antigen-binding domain of an antibody, of a fragment thereof or of a CAR refer to an antigen-binding domain which recognizes and binds to a specific antigen, but does not substantially recognize or bind other molecules in a sample. An antigen-binding domain that binds specifically to an antigen from one species may bind also to that antigen from another species. This cross-species reactivity is not contrary to the definition of that antigen-binding domain as specific. An antigen-binding domain that specifically binds to an antigen may bind also to different allelic forms of the antigen (allelic variants, splice variants, isoforms etc.). This cross reactivity is not contrary to the definition of that antigen-binding domain as specific.

The terms "immune cell" or "immune effector cell" may be used interchangeably and refer to a cell that may be part of the immune system and executes a particular effector function such as T cells, alpha-beta T cells, NK cells, NKT cells, B cells, innate lymphoid cells (ILC), cytokine induced killer (CIK) cells, lymphokine activated killer (LAK) cells, gamma-delta T cells, monocytes or macrophages. Preferentially these immune cells are human immune cells. Preferred immune cells are cells with cytotoxic effector function such as T cells, alpha-beta T cells, NK cells, NKT cells, ILC, CIK cells, LAK cells, macrophages or gamma-delta T cells. Most preferred immune effector cells are T cells and NK cells. "Effector function" means a specialized function of a cell, e.g. in a T cell an effector function may be cytolytic activity or helper activity including the secretion of cytokines.

As used herein, the term "antigen" is intended to include substances that bind to or evoke the production of one or more antibodies and may comprise, but is not limited to, proteins, peptides, polypeptides, oligopeptides, lipids, carbohydrates such as dextran, haptens and combinations thereof, for example a glycosylated protein or a glycolipid. The term "antigen" as used herein refers to a molecular entity that may be expressed on the surface of a target cell and that can be recognized by means of the adaptive immune system including but not restricted to antibodies or TCRs, or engineered molecules including but not restricted to endogenous or transgenic TCRs, CARs, scFvs or multimers thereof, Fab-fragments or multimers thereof, antibodies or multimers thereof, single chain antibodies or multimers thereof, or any other molecule that can execute binding to a structure with high affinity.

The term "epitope" means the part of an antigen, e.g. a soluble antigen, that may be recognized and specifically bound by antibodies or antigen bindings fragments thereof (antigen binding domains).

The tumor associated antigen (TAA) as used herein refers to an antigenic substance produced by tumor cells. Tumor associated antigens are useful tumor or cancer markers in identifying tumor/cancer cells with diagnostic tests and are potential candidates for use in cancer therapy.

Preferentially, the TAA may be expressed on the cell surface of the tumor/cancer cell, so that it may be recognized by the antigen binding receptor as disclosed herein.

The term "target cell" as used herein refers to cell which expresses an antigen on its cell surface that should be recognized (bound) by the antigen binding domain of the CAR as disclosed herein or by the antigen binding domain of the tag of the tagged polypeptide as disclosed herein.

Said target cell may be e.g. a cancerous cell or a cell associated with an autoimmune disease or a cell associated with an infectious disease.

Immunotherapy is a medical term defined as the "treatment of disease by inducing, enhancing, or suppressing an immune response". Immunotherapies designed to elicit or amplify an immune response are classified as activation immunotherapies, while immunotherapies that reduce or suppress are classified as suppression immunotherapies. Cancer immunotherapy as an activating immunotherapy attempts to stimulate the immune system to reject and destroy tumors. Adoptive cell transfer uses cell-based, preferentially T cell-based or NK cell-based cytotoxic responses to attack cancer cells. T cells that have a natural or genetically engineered reactivity to a patient's cancer are generated *in-vitro* and then transferred back into the cancer patient. Then the immunotherapy is referred to as "CAR immunotherapy" or in case of use of T cells only as "CAR T cell therapy" or "CAR T cell immunotherapy".

The term "treatment" as used herein means to reduce the frequency or severity of at least one sign or symptom of a disease.

The terms "therapeutically effective amount" or "therapeutically effective population" mean an amount of a cell population which provides a therapeutic benefit in a subject.

As used herein, the term "subject" refers to an animal. Preferentially, the subject is a mammal such as mouse, rat, cow, pig, goat, chicken dog, monkey or human. More preferentially, the individual is a human. The subject may be a subject suffering from a disease such as cancer.

The term "expression" as used herein is defined as the transcription and/or translation of a particular nucleotide sequence driven by its promoter in a cell.

The term "isolated" is used herein to indicate that the polypeptide, nucleic acid or host cell exist in a physical milieu distinct from that in which it occurs in nature. For example, the isolated polypeptide may be substantially isolated (for example enriched or purified) with respect to the complex cellular milieu in which it naturally occurs, such as in a crude extract.

The term "cancer" is known medically as a malignant neoplasm. Cancer is a broad group of diseases involving unregulated cell growth and includes all kinds of leukemia. In cancer, cells (cancerous cells) divide and grow uncontrollably, forming malignant tumors, and invading nearby parts of the body. The cancer may also spread to more distant parts of the body through the lymphatic system or bloodstream. There are over 200 different known cancers that affect humans.

### Examples

The following examples are intended for a more detailed explanation of the invention but without restricting the invention to these examples.

Example 1: Identification of conjugates comprising a fluorescent moiety and an antigen recognizing moiety which in combination allow to discriminate between FOLR1-expressing cancer cells and FOLR1-non-expressing cells

To identify target molecules for CAR T cell therapy we employed the following steps. High-grade serous ovarian carcinoma (OvCa) resections were dissociated and single cell suspensions were analyzed by flow cytometry (Fig.1A). Employing an unbiased surface marker screening library revealed the expression of FOLR1 on the majority of high-grade ovarian carcinoma patients (Fig.1B). Subsequently, this distinct target was validated by cyclic immunofluorescence microscopy. Fresh-frozen high-grade serous ovarian carcinoma resections were sliced and fixed with acetone. A screening was performed using a novel high content imaging platform enabling fully automated cyclic fluorescence imaging of individual biological samples. Sequential staining of OvCa specimen with several antigen recognizing moiety conjugated to a fluorescent moiety revealed the expression of a number of antigens. The co-expression of FOLR1 with EPCAM, which is a well-known marker for human epithelial tissues and carcinomas, was validated in all patients analyzed (Fig.2). Data analysis and further co-stainings confirmed that FOLR1 and EPCAM expression correlate on more than 90% of epithelial cells with elevated levels of FOLR1 (Fig.3).

On-target off-tumor toxicities of CAR T cell therapy, i.e. CAR T cells bind to and lyse non-tumor cells which express the CAR target under physiological conditions, have been described for several CAR T cell products¹ Hence FOLR1 expression was analyzed in healthy tissues (Fig.4A, B, C). The expression on healthy tissue was found to be at least partially discriminative, opening the possibility to get a selective labelling of cells to discriminate FOLR1-expressing OvCa from healthy cells (Fig.5). Healthy tissues express no or low levels of FOLR1. This finding confirmed recent studies².

Thus, the targeting of FOLR1 opens the possibility for CAR T cell treatment of OvCa. FOLR1 is expressed on the majority of OvCa cells but restricted or not or to a lesser extent on healthy tissues. Therefore, using CAR T cells which are activated by the detection of FOLR1, but not activated in the absence of the marker, should result in an efficient killing of FOLR1-expressing OvCa cells while leaving healthy cells unaffected.

Example 2: Design and generation of FOLR1-specific CAR T cells to discriminate between FOLR1-expressing cancer cells and FOLR1-non-expressing cells.

In order to identify the best CAR architecture we designed several CAR constructs derived from two existing monoclonal antibodies MORAb-0033 , Seq ID No: 1-4, Seq ID No:9-20) and M9346A4 , Seq ID No:5-8, Seq ID No:21-32), respectively. In total eight different CAR constructs were generated with varying Vh-Vl orientation and different hinges (human CD8alpha hinge Seq ID No:33-34, or human IgG4 hinge Seq ID No:35-36). All constructs are composed of human CD8alpha transmembrane domain (Seq ID No:37-38), human 41BB costimulatory domain (Seq ID No:39-40), and human CD3zeta domain (Seq ID No:41-42). Vh (Seq ID No:45, 46, 49, 50) and VI (Seq ID No:47, 48, 51, 52) were linked via a (G4S)3 linker (Seq ID No:43-44):

### Construct

**Table1: Overview of MB-CART FOLR1 candidate designs**

| MB CART FOLR1 | Hinge | scFv orientation |
|---|---|---|
| A (Seq ID No:53 ,54) | CD8alpha (Seq ID No:33-34) | Vh-Vl |
| B (Seq ID No:55, 56) | CD8alpha (Seq ID No:33-34) | Vl-Vh |
| C (Seq ID No:57, 58) | IgG4 (Seq ID No:35-36) | Vh-Vl |
| D (Seq ID No:59, 60) | IgG4 (Seq ID No:35-36) | Vl-Vh |
| E (Seq ID No 61, 62) | CD8alpha (Seq ID No:33-34) | Vh-Vl |
| F (Seq ID No:63, 64) | CD8alpha (Seq ID No:33-34) | Vl-Vh |
| G (Seq ID No:65, 66) | IgG4 (Seq ID No:35-36) | Vh-Vl |
| H (Seq ID No 67, 68) | IgG4 (Seq ID No:35-36) | Vl-Vh |

These constructs (Table1) were cloned in lentiviral transfer plasmids and lentiviral particles were produced via transfection of HEK293T cells with the respective plasmid mix. Subsequently, lentiviral particles were harvested from cell culture supernatant.

To determine the functional titer of the different lentiviral particles, SUP-T1 or activated primary human T cells were transduced with varying amounts of lentiviral vector and transduction efficiency was determined by detection of the co-expressed LNGFR marker or by detection of the CAR itself using biotinylated FOLR1-Fc protein )) to the detect the FOLR1-specific CAR via flow cytometry on two independent donors (Fig.6A, B, C, D).

All constructs were expressed in the respective cell types independent of the detection method applied, i.e. flow cytometry with direct and indirect CAR labelling, for two independent donors (Fig.6E, F). The expression levels between direct and indirect CAR detection were comparable for each donor (Fig.6G).

Example3: Use of FOLR1-specific CAR T cells which allow to discriminate between FOLR1-expressing cancer cells and FOLR1-non-expressing cells

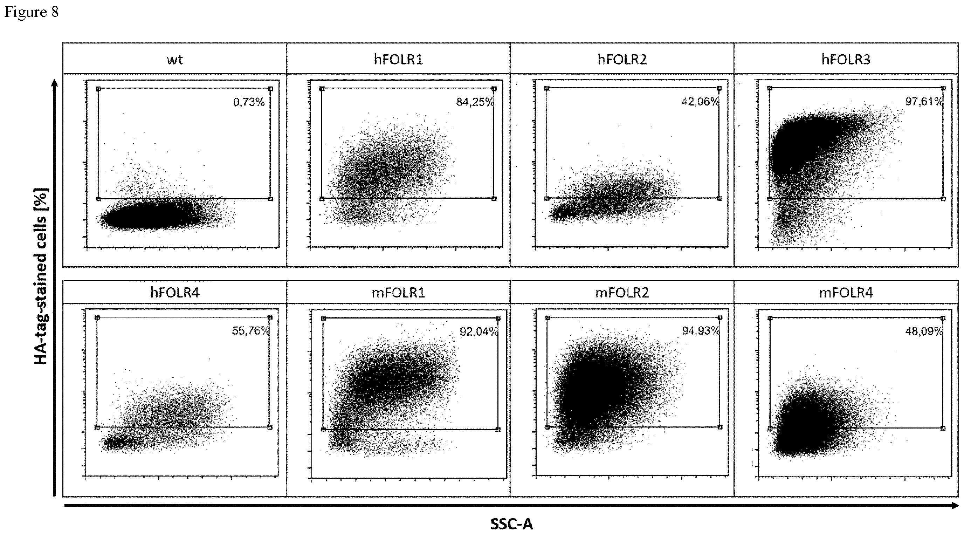

In order to identify the most efficient and specific CAR construct several *in vitro* assays were performed. To address whether differences in the CAR design, i.e. scFv orientation or hinge variant, affect CAR specificity, the eight constructs were co-cultured with cells expressing different human and murine FOLR variants. Since FOLR1 is part of the FOLR family which have a high degree of identity (Fig. 7), we sought to assess the specific reactivity of the different CAR constructs, i.e. CAR T cell-mediated killing of the respective target cell lines expressing human or murine FOLR variants. These target cell lines were generated by lentiviral transduction of Jurkat cells with human FOLR1 (Seq ID No:75, 76), human FOLR2 (Seq ID No:77, 78), human FOLR3 (Seq ID No:79, 80), human FOLR4 (Seq ID No:81, 82), murine FOLR1 (Seq ID No:83, 84), murine FOLR2 (Seq ID No:85, 86), or murine FOLR4 (Seq ID No:87, 88), respectively, and expression of the variants was confirmed by flow cytometry using an anti-3xHA-tag antibody (Fig. 8).

CAR T cells for the co-culture experiments were generated as follows. PBMCs were derived from whole blood or buffy coats via density gradient centrifugation and T cells were negatively isolated using MACS^{®} technology. Thereafter, purified human T cells were cultured in TexMACS^{™} medium and activated with T cell TransAct^{™} reagent. Afterwards T cells were transduced with the CAR-coding lentiviral particles and cultured for 12 days in supplemented TexMACS^{™} medium.

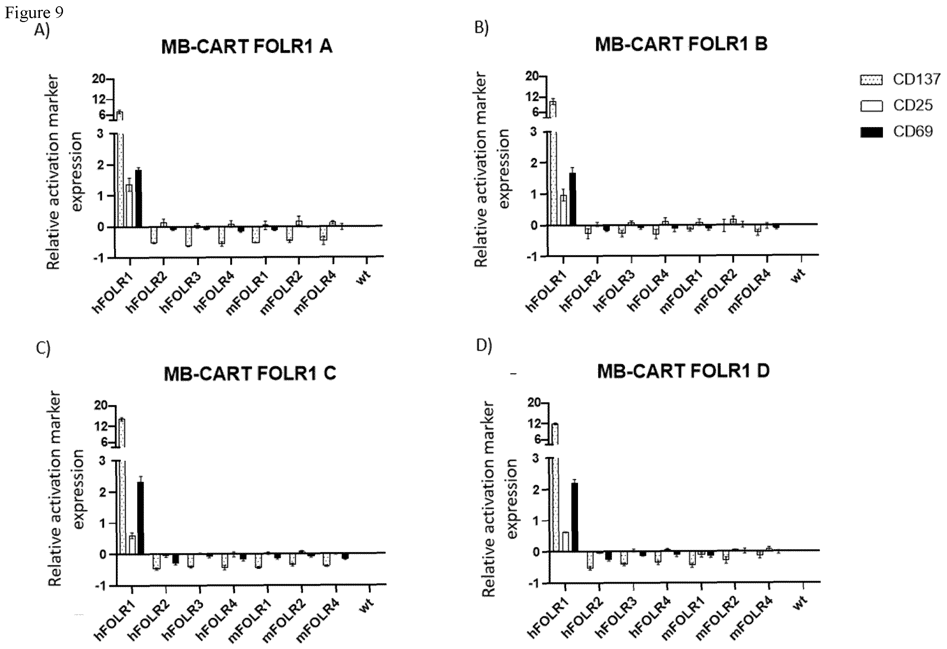

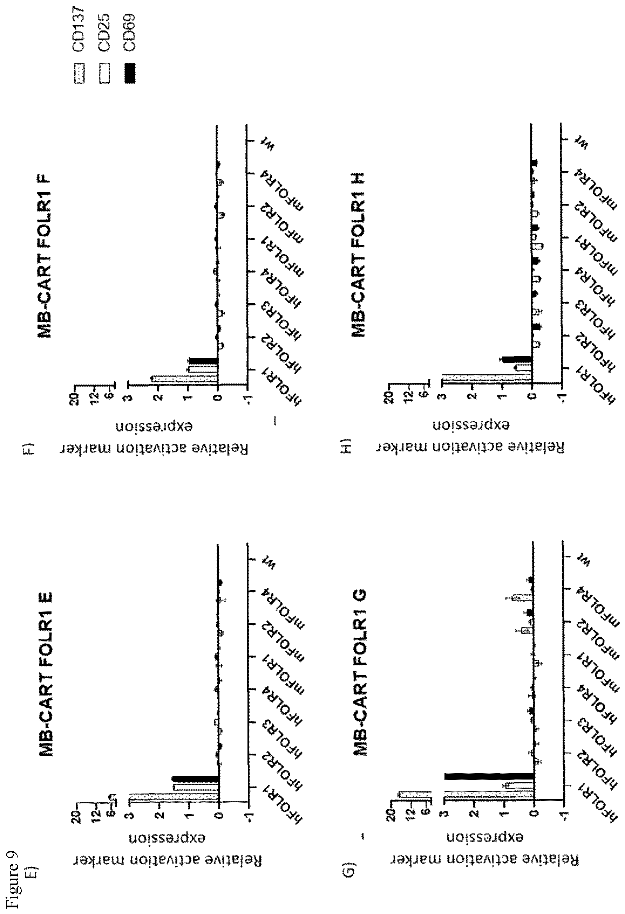

Co-culture experiments were performed in TexMACS^{™} medium at an effector to target ratio of 2:1 for 48h. At the end of the experiment CAR T cells were analyzed by flow cytometry for the expression of activation markers, CD137, CD25, CD69, as indicator for CAR T cell functionality (Fig.9). None of the tested CAR constructs (Fig.9A-I) showed reactivity to any FOLR variant other than human FOLR1.

In a second step the different CAR candidates were co-cultured with a human ovarian cancer cell line OV-90 expressing GFP and luciferase. OV-90 cells were either FOLR1-proficient or FOLR1-deficient, respectively. Specific-FOLR1 knockout in OV-90 cells was performed via electroporation of ribonucleo-particles of CAS9 and guideRNAs for FOLR1 (Seq ID No:89). FOLR1 knockout was confirmed by sequencing (Seq ID No:90, 91) of genomic DNA (Fig. 10A), by qPCR (primer S Seq ID No:92-101) (Fig.10B), and immunofluorescence (Fig. 10C). FOLR1 expression on OV-90 wt and OV-90 FOLR1 KO cells as well as further ovarian cancer cell lines, i.e. OVCAR-3, SKOV-3, and Caov-3, was additionally analyzed by flow cytometry (Fig.11A). FOLR1 expression varied over the different cell lines analyzed and FOLR1 expression was not detectable in OV-90 FOLR1 KO cells (Fig.11B, C, D). Subsequently, we confirmed FOLR1 expression on the cell lines using different anti-FOLR1 clones (Fig.11E, F, G, H, I, J).

Additionally, different co-culture experiments of FOLR1-directed CAR T cells were performed with ovarian cancer cell lines expressing GFP in TexMACS^{™} medium at an effector to target ratio of 2:1 for 48h. GFP expression of ovarian cancer cell lines was measured over time with the IncuCyte system. CAR T cell mediated killing of target cells is measured by the decrease of GFP signal over time.

First, OV-90 cells (with or without FOLR1 expression) were used as target cells for the co-culture experiments and GFP expression of OV-90 cells was measured over time (OV-90 wt cells: Fig.12A, B; OV-90 FOLR1 KO cells: Fig. 12C, D). Efficient target cell lysis causes a decrease in GFP signal, whereas in efficient or now target cell lysis increases GFP signal over time. Here MB-CART-FOLR1 candidates B, D, F, and H (candidates with Vl-Vh orientation of scFv) tended to be less efficient than MB-CART-FOLR1 candidates A, C, E, and G (candidates with Vh-Vl orientation of scFv). Additionally, MB-CART-FOLR1 candidates F and H tend to control OV-90 FOLR1 KO cells antigen-independently. At the end of the experiment CAR T cells were analyzed by flow cytometry to assess activation marker expression (Fig.12E, F, G), exhaustion marker expression (Fig.12H, I), and phenotype of T cells (OV-90 wt cells: Fig.12J; OV-90 FOLR1 KO cells: Fig. 12K). Activation markers CD25, CD69, and CD137 were induced in an antigen-dependent manner (Fig.12E-G). Moreover, MB-CART-FOLR1 candidates A, B, F, and H express lower levels of exhaustion markers TIM-3, PD1, and LAG3 (Fig.12H, I). After co-culture of CAR T cells with OV-90 cells *in vitro,* more Tₑₘ and fewer less differentiated subtypes like T_{cm} or T_{scm} were present in antigen-dependent manner (OV-90 wt cells: Fig.12J; OV-90 FOLR1 KO cells: Fig. 12K). The co-culture medium was analyzed after 24h for the presence of human cytokines using the MACSplex^{™} assay. The tested CAR constructs showed differential behavior. MB-CART-FOLR 1 constructs secreted cytokines GM-CSF, IFN-gamma, IL-2, and TNF-alpha antigen-dependently. (Fig.12L, Fig.12M) Finally, FOLR1-directed CAR T cells expressed significantly more Granzyme B when the antigen was present (Fig. 12N).

Further co-culture experiments of FOLR1-specific CAR T cells with alternative human ovarian cancer cell lines (Caov-3, OVCAR-3) expressing different levels of FOLR1 (Fig. 11) were also efficiently killed *in vitro* . MB-CARTFOLR1 candidates efficiently lysed Caov-3 cells expressing high levels of FOLR1 (Fig.13A). However, MB-CARTFOLR1 candidates A-D responded better to ovarian cancer cells with lower expression of FOLR1 (OVCAR-3, Fig.13B), i.e. cancer cells were lysed by CAR T cells even at lower levels of FOLR1 expression. This finding was further confirmed by an increase in activation markers on the respective FOLR1-specific CAR T cells (Fig.13C).

Finally, in a co-culture experiment of FOLR1-specific CAR T cells with OV-90 wt cells isolated from xenografts, CAR T cells efficiently killed *in vitro,* too (Fig. 14).This is indicating that *in vivo* passaging of OV-90 cells does not significantly impact efficient *in vitro* killing capacity of CAR candidates. Here, the MB-CART-FOLR1 candidates A-D efficiently killed xenograft-derived OV-90 tumor cells (Fig.14A). MB-CART-FOLR1 candidates E-H killed xenograft-derived OV-90 tumor cells, too. However, MB-CART-FOLR1 F was less efficient than the other candidates (Fig.14B).

Additionally, in a more challenging *in vitro* assay, serial killing experiments, were performed, i.e. CAR T cells were re-challenged by the addition of fresh target cells after 96h (20,000 target cells) and 192h (50,000 target cells) of co-culture, respectively. This was done using either OV-90 FOLR1 proficient (Fig. 15A) or OV-90 FOLR1 deficient cells (Fig.15B). Here, we assessed functionality of MB-CART-FOLR1 candidates A and E. MB-CART-FOLR1 A killed more efficient and proliferated more than the MB-CART-FOLR1 E. Interestingly, candidate MB-CART-FOLR1 E tended to inhibit growth of the OV-90 FOLR1 deficient cells (Fig.15B). Although, MB-CART-FOLR1 candidates A and E did not proliferate when co-cultured with OV-90 FOLR1 KO cells (Fig. 15C), MB-CART-FOLR1 candidate E, in contrast to MB-CART-FOLR1 candidate A, did not proliferate in co-culture with FOLR1-expressing OV-90 cells (Fig.15C).

In order to assess functionality of the anti-FOLRl CAR T cells *in vivo,* CAR T cells were generated in an automated cell processing system, the CliniMACS Prodigy^{®}. In a first approach four CAR constructs (MB-CART-FOLR1 candidates A, C, E, and G which performed best *in vitro*, i.e. Vh-Vl oriented candidates, were injected at two doses (1×10⁶ and 1×10⁷CAR T cells) intravenously in NSG mice, respectively, to analyze tolerability of these CAR T cell products over a period of 21 days including weekly blood withdrawals (Fig. 16A). Before injection of the different CAR constructs the respective cell products were analyzed for cellular composition (Fig. 16B), viability (Fig. 16C), frequency of human CD4 and CD8 T cells (Fig. 16D) and transduction efficiency (Fig. 16E). All CAR T cell products were composed of more than 97% T cells (mainly CD4 T cells) with high viabilities of 95%. Transduction efficiency was approximately 60%.

All CAR candidates were well tolerated at the 1×10⁶ CAR T cell dose (Fig.16F). However, at the higher dose of 1×10⁷ CAR T cells, one of the tested constructs MB-CART-FOLR1-C) induced a temporary weight loss (Fig.16G). This could indicate an antigen-independent CAR T cell activation. In line with this hypothesis, a temporary increase in CAR- and human CD45- -expressing cells was detected at day 15 (Fig.16H, I). Particularly, human CD8 T cells proliferated (Fig. 16J). After 21 days bone marrow and spleen were collected from the different groups and analyzed by flow cytometry for the presence of human leucocytes. Human leucocytes could be detected in bone marrow and spleen at high but not low dose of CAR T cell injection (Fig.16K) Likewise, human CD4 and CD8 T cells were more frequently detected in bone marrow and spleen at high CAR T cell dose (Fig.16L). Consequently, CAR T cells were also more frequently detected in bone marrow and spleen at high CAR T cell dose (Fig.16M)

Analysis of human cytokine secretion in murine blood samples was measured over 21 days once a week. Experimental groups treated with low doses of CAR T cells did not differ significantly from the UTD control (Fig.16N).

Within the second experimental group which was treated with the higher dose of CAR T cells human IFNgamma was elevated at day 8 in the group injected with MB-CART-FOLR1-C (Fig. 160), which is the group with temporary weight loss and T cell expansion. After 21 days groups receiving MB-CART FOLR1 C and E had elevated levels of human IFNgamma in the plasma compared with the UTD control group. Murine cytokines were only secreted at very low levels during the 21 days in both groups (Fig. 16P). However, the group injected with MB-CART-FOLR1-C, which is the group with temporary weight loss and T cell expansion, had an increase in murine GM-CSF at day 8.

In a second approach NSG mice were injected with OV-90 cells subcutaneously. After tumor establishment, the same four CAR constructs (MB-CART-FOLR1 A, C, E, and G) were injected at a single dose (1×10⁷ total T cells) intravenously to assess anti-tumor efficacy of these CAR T cell products over a period of 21 days including weekly blood withdrawals (Fig.17A). Before injection of the different CAR constructs, the respective cell products were analyzed for cellular composition (Fig. 17B), viability (Fig. 17C), frequency of human CD4 and CD8 T cells (Fig. 17D), transduction efficiency (Fig. 17E), and vector copy number (Fig. 17F). All CAR T cell products were composed of more than 96% T cells with high viabilities of 97%. Transduction efficiency was approximately 55% with vector copy numbers below three. The different CAR T cell products were also tested- in parallel to the animal study- *in vitro* co-culture experiments with FOLR1-proficient and -deficient OV-90 cells, respectively, to confirm CAR T cell functionality of the injected CAR T cell products (Fig.17G). Here candidate MB-CART FOLR1 G lysed OV-90 FOLR1 KO cells in an antigen-independent way at an effector to target ratio of 2:1.

Moreover, one of the tested constructs (MB-CART-FOLR1-C) induced a temporary weight loss (Fig.17H) comparable to the tolerability study group dosed with 1×10⁷ CAR T cells (Fig.16H).

All CAR construct efficiently reduced tumor burden (Fig. 171, J, K). Especially, candidate MB-CART-FOLR1 A showed superior anti-tumor efficacy accompanied by expansion of human CD45- and CAR-expressing cells (Fig.17L, N, O) with changes in the human CD4 and CD8 ratio in the T cell population at day 21 Fig.17M).

At the end of the study human and murine leukocytes (Fig. 17P), human CD4 and CD8 T cells (Fig.17Q), as well as CAR T cells (Fig.17R) were detectable in spleen, bone marrow, lung, and the xenograft tumors. Interestingly, MB-CART-FOLR1 A showed increased presence of human CD45 and CD8 T cells in the tumor tissues. The T cells in the different organs were mainly of the TEM phenotype (CD4 T cells: Fig.17S; CD8 T cells: Fig. 17T), whereas the TCM phenotype was more frequently represented in peripheral blood (CD4 T cells: Fig.17S; CD8 T cells: Fig.17T). Analysis of cytokine secretion during the 21 days post CAR T cell infusion revealed moderated and stable secretion of IFNgamma for the MB-CART FOLR1 A group(Fig.17U).Thereby, we concluded that candidate MB-CART FOLR1 A displayed rapid tumor eradication, pronounced proliferation, CAR expression, and short-term persistence. Moreover candidate MB-CART-FOLR1 A showed pronounced homing to bone marrow and spleen, and tumor infiltration.

Example 4: Use of alternative lentiviral vector based FOLR1-specific CAR T cells which allow to discriminate between FOLR1-expressing cancer cells and FOLR1-non-expressing cells

In a complementary approach, CAR T cells were generated with an alternative lentiviral vector lacking the LNGFR reporter. Candidates MB-CART-FOLR1 A (Seq ID No:53, 54) and MB-CART-FOLR1 E (Seq ID No:61, 62) were assessed with this modified vector due to their *in vitro* performance. First, CAR T cells were generated in an automated cell processing system, the CliniMACS Prodigy^{®}, from primary human T cells via transduction with lentiviral vector and transduction efficiency, i.e. CAR expression, was determined by detection of the CAR using biotinylated FOLR1-Fc protein (Fig. 18) and anti-biotin secondary staining to detect FOLR1-specific CARs via flow cytometry. Both candidates MB-CART-FOLR1 A and MB-CART-FOLR1 E were expressed at high and comparable levels.

In a second step, these optimized and automated manufactured CAR T cells were co-cultured with various ovarian cancer cell lines at an effector to target ratio of 2:1 for 48h (Fig. 19). Killing kinetics were evaluated similarly as in example 3. Both candidates MB-CART-FOLR1 A and MB-CART-FOLR1 E efficiently lysed ovarian cancer cells OV-90 wt, OVCAR3, and Caov-3 while OV-90 FOLR1 KO grew out independently of the presence of CAR T cells in the coculture (Fig.19A). However, candidate MB-CART-FOLR1 A was more efficient in lysing SKOV-3 cells compared to candidate MB-CART-FOLR1 E (Fig.19A). At the end of the experiment CAR T cells were analyzed by flow cytometry to assess activation marker expression (Fig.19B) and exhaustion marker expression (Fig.19C). Activation and exhaustion markers were upregulated in an antigen-dependent manner and to similar expression levels for both FOLR1 CAR candidates. However, MB-CART FOLR1 E displayed lower activation markers and PD1 levels in co-culture with SKOV-3 compared to candidate A (Fig.19B, C), correlating with the reduced cytolytic kinetics observed with this cell line (Fig. 19A).

The co-culture medium was analyzed after 24h for the presence of human cytokines using the MACSplex^{™} assay. Both MB-CART FOLR 1 constructs secreted cytokines GM-CSF, IFN-gamma, IL-2, and TNF-alpha antigen-dependently, with a tendency for MB-CART FOLR1 E to secrete less GM-CSF and TNF-alpha (Fig.19D).

Finally, both FOLR1-directed CAR T cell candidates expressed similar levels of Granzyme B in an antigen-dependent way (Fig.19E).

Taken together, both candidates MB-CART FOLR 1 A and E in the alternative lentiviral vector are functional and specific *in vitro.* Since MB-CART FOLR 1 A performed better in the previous *in vitro* and *in vivo* experiments (see example 3) as well as in this series of *in vitro* assays based on the alternative lentiviral vector and the CAR manufacturing via the automated cell processing system, CliniMACS Prodigy^{®}, only MB-CART FOLR1 candidate A was further characterized.

In order to assess functionality of the anti-FOLR1 CAR T cells *in vivo,* NSG mice were subcutaneously injected with OV-90 wt or OV-90 FOLR1 KO cells, respectively. After tumor establishment, the CAR construct MB-CART-FOLR1 A was intravenously injected at a single dose (1×10⁷ total T cells) to analyze efficacy of this CAR T cell product over a period of 21 days including weekly blood withdrawals (Fig.20A). Before injection of the different CAR constructs, the respective cell product was analyzed for cellular composition (Fig.20B), viability (Fig.20C), human CD4 and CD8 T cell composition (Fig.20D), transduction efficacy (Fig.20E), and vector copy number (Fig.20F). CAR T cell product was composed of more than 96% T cells with high viability of 99%. Transduction efficiency was approximately 80% with a vector copy number of 2.1.

Over the course of the study, mice maintained a stable body weight across all conditions (OV-90 wt: Fig21G, OV-90 FOLR1 KO: Fig21H). MB-CART FOLR1 A efficiently reduced OV-90 wt tumor burden (Fig.20I, J, L) while no tumor elimination was observed with OV-90 FOLR1 KO tumors (Fig.20I, K, L).

In an antigen-dependent manner, candidate MB-CART-FOLR1 A showed anti-tumor efficacy (Fig.20I, L) accompanied by expansion of human CD45- and CAR-expressing cells (Fig.20M, O, P) as well as an increase of human CD8 T cells (Fig.20N).

At the end of the study bone marrow, spleen, lung, and remaining tumor tissue were collected and dissociated to single cell suspensions. Subsequently, human and murine immune cells (Fig.20Q), human CD4 and CD8 T cells (Fig.20R), and CAR T cells (Fig.20S), were detected in spleen, bone marrow and the xenograft tumors via flow cytometry.

Human CD45 cells were enriched in an antigen-dependent manner in spleen, lung, and tumor (Fig.20Q). Human CD8 cells were also antigen-dependently enriched in bone marrow, lung, and tumor tissue (Fig.20R), whereas human CD4 cells did not enriched differentially in the tissues analyzed.

Analysis of the remaining tumor tissue revealed that the frequency of FOLR1-expressing cells is strongly reduced in OV-90 wt bearing mice after treatment with MB-CART FOLR1 A (Fig.20T left panel). Additionally, cellular composition of the tumor tissue changes after MB-CART FOLR1 treatment (Fig.20T right panel). Tumors mainly consist of GFP-expressing OV-90 cancer cells. However, treatment with MB-CART FOLR1 A changes the cellular composition to a mainly non-cancer cell, i.e. non-GFP-expressing cells, based composition. Reduction in the frequency of FOLR1- and GFP-expressing cells indicates efficient tumor eradication and changes in the tumor tissue composition.

The CD4 CAR T cells were mainly of the TEM phenotype in the bone marrow, tumor and spleen, and the TCM phenotype in blood and lung contrary to the corresponding controls- MB-FOLR1 CART A with OV-90 FOLR1 KO or untransduced T cells with both tumor types, which were mainly of the Tnaive phenotype in tumor and lung, TEM in spleen and bone marrow, and a more distributed phenotype composition in blood (Fig.20U).

The CD8 CAR T cells were mainly of the TEM phenotype in the bone marrow, tumor and spleen, and a more distributed phenotype composition in blood and lung contrary to the corresponding controls- MB-FOLR1 CART A with OV-90 FOLR1 KO or untransduced T cells with both tumor types, which were mainly of a more distributed phenotype composition in blood, lung, tumor, bone marrow and spleen (Fig.20V).

Analysis of cytokine secretion over 21 days post CAR T cell infusion revealed elevated levels of IFN-gamma and IL-9 at day 7 in the MB-CART FOLR1 A treated group bearing OV-90 wt xenografts (Fig.20W).

Taken together, candidate MB-CART-FOLR1 A induced rapid tumor eradication, CAR T cell proliferation, and short-term persistence. Moreover candidate MB-CART FOLR1 A showed pronounced tumor infiltration and cytokine release.

### References

1. Shangjun Sun, He Hao, Ge Yang, Yi Zhang, Yang Fu. Immunotherapy with CAR-Modified T Cells: Toxicities and Overcoming Strategies. Journal of Immunology Research, 2018. doi: 10.1155/2018/2386187; PMCID: PMC5932485; PMID: 29850622.
2. Lana E Kandalaft, Daniel J Powell, Jr, and George Coukos. A phase I clinical trial of adoptive transfer of folate receptor-alpha redirected autologous T cells for recurrent ovarian cancer. Journal of Translational Medicine, 2012. doi: 10.1186/1479-5876-10-157; PMCID: PMC3439340; PMID: 22863016.
3. GRASSO, Luigi, NICOLAIDES, Nicholas, C. SASS, Philip, M. Monoclonal antibodies that specifically bind to folate receptor alpha.Pub. Date: 01.09.2005. Appl. No.: PCT/US2005/004240. Pub. No.: WO2005080431A2.
4. Olga AB, Millis, MA (US); Daniel TAVARES, Natick, MA (US); Lingyun RUI, Weston, MA (US); Gillian PAYNE, Waban, MA (US); Viktor S. GOLDMAKHER, Newton, MA (US). Folate receptor 1 antibodies and immunoconjugates and uses thereof. Pub. Date: 01.09.2011. Appl. No.: PCT/US2011/026079. Pub. No WO2011106528.
5. Thomas Paul Wallace, Gight, Methlick; William Joseph Harris, Carnoustie; Francis Joseph Carr, Balmedie, all of United Kingdom; Wolfgang J. Rettig, Pilar; Garin-Chesa, both of Biberach, Germany; Lloyd J. Old, New York, N.Y. Recombinant human anti-lk26 antibodies. Pub. Date: 01.09.2005. Appl. No.: 08/760,840. Pub. No.: USOO5952484A.
6. Luigi Grasso, Bala Cynwyd, PA (US); Nicholas C. Nicolaides, Boothwyn, PA (US); Philip M. Sass, Audubon, PA (US). Monoclonal antibodies that specifically block biological activity of a tumor antigen. Pub. Date: Oct. 20, 2005. Appl. No.: 11/056,776. Pub. No.: US 2005/0232919 A1.
7. O'Shannessy DJ, Somers EB, Albone E, Cheng X, Park YC, Tomkowicz BE, Hamuro Y, Kohl TO, Forsyth TM, Smale R, Fu YS, Nicolaides NC. Characterization of the human folate receptor alpha via novel antibody-based probes. Oncotarget. 2011 Dec;2(12):1227-43. doi: 10.18632/oncotarget.412. PMID: 22204844; PMCID: PMC3282080.
8. Sato S, Itamochi H. Profile of farletuzumab and its potential in the treatment of solid tumors. Onco Targets Ther. 2016;9:1181-1188https://doi.org/10.2147/OTT.S98242; Thomas A et al Lung Cancer. 2013 April; 80(1): 15-18. doi:10.1016/j.lungcan.2012.12.021
9. Hansen T., Datamonitor Healthcare, Informa Pharma Intelligence, Ovarian cancer disease analysis, Ref Code: DMKC0215129, 2021
10. Leamon CP, Low PS, 1991, PNAS; Kandalaft et al., 2012, J Transl Med
11. Kelemen, 2006, Int. J. Cancer

## Claims

1. A chimeric antigen receptor (CAR) comprising
a. an antigen binding domain specific for folate receptor 1 (FolR1)
b. a spacer
c. a transmembrane domain and
d. an intracellular signaling domain

2. The CAR according to claim 1, wherein the antigen binding domain comprises a heavy chain variable region of an antibody (VH) comprising the amino acid sequence Seq ID No:2 and a light chain variable region of an antibody (VL) comprising the amino acid sequence Seq ID No:4

3. The CAR according to claim 2, wherein the antigen binding domain comprises from the N- to the C-terminus the VH comprising Seq ID No:2 and VL comprising Seq ID No:4.

4. The CAR according to claim 3, wherein the antigen binding domain comprises the amino acid sequence Seq ID No: 103

5. The CAR according to any of the claims 1-4, wherein said spacer comprises the hinge domain of CD8 alpha

6. The CAR according to any of the claims 1-5, wherein said transmembrane domain comprises the transmembrane domain of CD8 alpha and wherein said intracellular signaling domain comprises the costimulatory domain of CD137 and stimulatory domain of CD3zeta.

7. The CAR according to claim 6 wherein said CAR comprises the amino acid sequence Seq ID No: 105.

8. An engineered cell expressing a CAR according to any of the claims 1-7.

9. The engineered cell according to claim 8, wherein said cell is a immune cell .

10. The engineered cell according to any of the claims 8 or 9 for use in immunotherapy.

11. The engineered cell according any of the claims 8 -10 for use in treatment of cancer, wherein the cancerous cells of said cancer express FolR1.

12. A pharmaceutical composition comprising engineered cells expressing a CAR according to any of the claims 8-9.

13. A nucleic acid sequence encoding a CAR according to any one of claims 1-7.
